# EUROPEAN PATENT SPECIFICATION

(11) **EP 3 846 811 B1**
(45) Date of publication and mention of the grant of the patent: **05.11.2025**
(21) Application number: 19783171.2
(22) Date of filing: 06.09.2019
(51) Int. Cl.: A61K 31/445, A61K 31/5377, A61K 31/5415, A61P 23/00, A61P 23/02, A61K 9/00, A61K 9/107, A61K 45/06, A61K 47/24

(54) **COMBINATION OF APREPITANT, BUPIVACAINE, AND MELOXICAM FOR USE IN THE TREATMENT OF POST-SURGICAL PAIN**
KOMBINATION AUS APREPITANT, BUPIVACAIN UND MELOXICAM ZUR VERWENDUNG BEI DER BEHANDLUNG VON POSTOPERATIVEM SCHMERZ
COMBINAISON D'APREPITANT, DE BUPIVACAINE ET DE MELOXICAM POUR L'UTILISATION DANS LE TRAITEMENT DE LA DOULEUR POST-CHIRURGICALE

(30) Priority: 07.09.2018 US 201862728732 P; 11.06.2019 US 201962860190 P
(43) Date of publication of application: 14.07.2021
(73) Proprietor: Heron Therapeutics, Inc., San Diego, CA 92121 (US)
(72) Inventor: QUART, Barry D., San Diego, California 92121 (US); OTTOBONI, Thomas B., Belmont, California 94002 (US)
(74) Representative: Sagittarius IP
(86) International application number: PCT/US2019/050079
(87) International publication number: WO 2020/051543

(56) References cited:
- WO-A1-2004/043479
- WO-A1-2018/048460
- CA-A1- 3 048 429
- US-A1- 2018 000 947
- US-B2- 9 592 227
- NAMI KAKUTA ET AL: "Neurokinin-1 receptor antagonism, aprepitant, effectively diminishes post-operative nausea and vomiting while increasing analgesic tolerance in laparoscopic gynecological procedures", 5 August 2011 (2011-08-05), pages 246 - 251, XP055642863, Retrieved from the Internet <URL:http://medical.med.tokushima-u.ac.jp/jmi/vol58/pdf/v58_n3-4_p246.pdf> [retrieved on 20191115]
- BARRY QUART: "Company update JP Morgan Conference January 2018", INTERNET CITATION, 1 January 2018 (2018-01-01), XP002790559, Retrieved from the Internet <URL:https://herontherapeutics.gcs-web.com/static-files/57e99f1a-b9d6-414a-a694-ebc521f83b05> [retrieved on 1077]
- DUFFY R A: "POTENTIAL THERAPEUTIC TARGETS FOR NEUROKININ-1 RECEPTOR ANTAGONISTS", EXPERT OPINION ON EMERGING DRUGS, INFORMA HEALTHCARE, UK, vol. 9, no. 1, 1 May 2004 (2004-05-01), pages 9 - 21, XP009051073, ISSN: 1472-8214, DOI: 10.1517/14728214.9.1.9
- RAYMOND A. DIONNE ET AL: "The substance P receptor antagonist CP-99,994 reduces acute postoperative pain", CLINICAL PHARMACOLOGY AND THERAPEUTICS, vol. 64, no. 5, 1 November 1998 (1998-11-01), US, pages 562 - 568, XP055642613, ISSN: 0009-9236, DOI: 10.1016/S0009-9236(98)90140-0

## Description

### TECHNICAL FIELD

The disclosure relates generally to compositions for use in methods of treating postsurgical pain. The compositions comprise a neurokinin 1 (NK-1) receptor antagonist , in particular aprepitant, in combination with a pain medicatior, in particular bupivacaine and meloxicam. This disclosure also relates to a pharmaceutical composition comprising aprepitant, bupivacaine and meloxicam, and a delivery vehicle.

### BACKGROUND

Pain is defined by the International Association for the Study of Pain (IASP) as an unpleasant sensory and emotional experience associated with actual or potential tissue damage, or described in terms of such damage (Classification of Chronic Pain, 2nd Ed., Eds. Merkskey & Bogduk, IASP Press, 1994). An effective pain treatment modality is generally considered to be one which provides relief of pain with minimal adverse and/or unwanted side-effects. Treatment of acute pain, such as post-operative pain following surgery, is an area of active investigation. Indeed, the effective treatment of post-operative pain is now considered to be an essential component of the overall care of a surgical patient.

Post-surgical pain, which can be nociceptive in nature, is generally due to inflammation from tissue trauma (e.g., due to the surgical incision, dissection or burns) or direct nerve injury (e.g., nerve transection, stretching, or compression). Pain relief is of primary importance to almost every patient undergoing surgery and to medical personnel treating or caring for a patient undergoing or recovering from a surgical procedure. Pre-operatively, one of the most common questions asked by patients pertains to the amount of pain that they will experience following surgery (Vadivelu, N., Yale J. of Biology and Medicine 83 (2010), p. 11-25). Effective analgesia is vital for ensuring patient comfort, encouraging early mobilization, promoting earlier patient discharge from the medical setting (e.g., hospital, outpatient facility or the like), and for providing enhanced recovery times. Effective treatment of post-operative pain may also reduce the onset/occurrence of chronic pain syndromes such as neuropathic pain and/or the development of depression. Additional advantages of effective post-operative pain management include fewer pulmonary and cardiac complications and a reduced risk of deep vein thrombosis (Ramsay, M., Proc (Bayl Univ Med Centr). 2000 Jul; 13(3):244-247). In contrast, inadequate pain control may result in increased morbidity or mortality (Sharrock NE, et al., Anesth Analg. 1995 Feb; 80(2):242-8).

Unfortunately, although there has been a significant increase in knowledge related to the physiology of pain over the last decade, the resulting implications in clinical practice have failed to follow suit. Even after decades of advances in the understanding of the physiology and psychology of pain, one of the mainstays of pain therapy remains the use of opioids. While effective analgesics, opioids also carry with them many undesirable side effects, such as sedation, respiratory depression, nausea and vomiting, hypotension, bradycardia, risk of addiction, to name a few.

One approach for providing localized, effective, long-acting relief of pain, particularly acute pain such as post-surgical pain, is the utilization of a sustained or extended release system. Numerous factors can impact the design of an effective drug delivery system and certain classes of drugs, such as the local anesthetics, are typically considered to be relatively short lasting such that they are most often used only in relatively minor or moderate procedures. Nami Kakuta ET AL: "Neurokinin-1 receptor antagonism, aprepitant, effectively diminishes post-operative nausea and vomiting while increasing analgesic tolerance in laparoscopic gynecological procedures", 5 August 2011 (2011-08-05), pages 246-251 and CA 3 048 429 disclose a method for treating post-surgical pain comprising administering aprepitant in combination with a pain medication.

WO 2018/048460 discloses compositions comprising bupivacaine and meloxicam for use in the treatment of post-surgical pain. There remains a need for methods for the treatment of post-operative pain that are long-lasting, efficacious, convenient to administer, and that can overcome some of the drawbacks associated with the use of opioids. The present methods satisfy these and other needs.

NK-1 receptor antagonists are a class of compounds known for the treatment of nausea and vomiting associated with cancer chemo-therapy. They have failed to show efficacy in clinical trials of a variety of clinical pain states. This application demonstrates that the combinational use of a NK-1 receptor antagonist and pain medication in a particular manner can reduce post-surgical pain more significantly than the use of the individual active agent alone or other manners of the same combinational use.

### BRIEF SUMMARY

The following aspects and embodiments thereof described and illustrated below are meant to be exemplary and illustrative, not limiting in scope. The invention is set out in the appended set of claims.

The references to the methods of treatment by therapy or surgery of this description are to be interpreted as references to compounds, pharmaceutical compositions and medicaments of the present invention for use in those methods.

In one aspect, a pharmaceutical composition comprising a pain medication, an NK-1 receptor antagonist, and a delivery vehicle is provided.

In one embodiment, a pharmaceutical composition comprising a local anesthetic, an NK-1 receptor antagonist, and a delivery vehicle is provided.

In one embodiment, a pharmaceutical composition comprising an anti-inflammatory agent, an NK-1 receptor antagonist, and a delivery vehicle is provided.

In one embodiment, a pharmaceutical composition comprising a nonsteroidal anti-inflammatory agent (NSAID), an NK-1 receptor antagonist, and a delivery vehicle is provided. In one embodiment, a pharmaceutical composition comprising a nonsteroidal anti-inflammatory agent (NSAID), an NK-1 receptor antagonist, a local anesthetic, and a delivery vehicle is provided.

In one embodiment, a pharmaceutical composition comprising a steroidal anti-inflammatory agent, an NK-1 receptor antagonist, a local anesthetic, and a delivery vehicle is provided.

In one embodiment, a pharmaceutical composition comprising an analgesic agent, an NK-1 receptor antagonist, and a delivery vehicle is provided.

In some embodiments, a pharmaceutical composition comprising a NK-1 receptor antagonist, a local anesthetic, and a steroid is provided. In some embodiments, a pharmaceutical composition comprising a NK-1 receptor antagonist, a local anesthetic, and an analgesic is provided.

In some embodiment, the local anesthetic is a caine type anesthetic. In some embodiment, the local anesthetic is selected from the group consisting of bupivacaine, ropivacaine, levobupivacaine, dibucaine, mepivacaine, procaine, lidocaine, and tetracaine. In some embodiments, the local anesthetic is bupivacaine or ropivacaine.

In some embodiments, the local anesthetic is bupivacaine.

In some embodiments, the local anesthetic is ropivacaine.

In some embodiments the NSAID is selected from the group consisting of diflunisal, indomethacin, tolmetin, sulindac, etodolac, ketorolac, diclofenac, nabumetone, ibuprofen, dexibuprofen, naproxen, fenoprofen, ketoprofen, dexketoprofen, flurbiprofen, oxaprozin, loxoprofen, mefenamic acid, meclofenamic acid, flufenamic acid, tolfenamic acid, meloxicam, piroxicam, tenoxicam, droxicam, lornoxicam, isoxicam, celecoxib, rofecoxib, and valdecoxib.

In some embodiments, the NSAID is meloxicam.

In some embodiments the steroidal anti-inflammatory agent is selected from the group consisting of 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, dexamethasone 21-acetate, dexamethasone 21-phosphate di-Na salt, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide and a combinations thereof.

In some embodiments the analgesic agent is selected from the group consisting of capsaicinoids including capsaicin, dihydrocapsaicin, nordihydrocapsaicin, homocapsaicin, homodihydrocapsaicin, and nonivamide and combinations thereof.

In some embodiments, the NK-1 receptor antagonist is selected from the group consisting of aprepitant, rolapitant, netupitant, lanepitant, vestipitant, orvepitant maleate, casopitant, ezlopitatit, serlopitant, befetupitant and maropitant, or a pharmaceutically acceptable salt thereof.

In some embodiments, the NK-1 receptor antagonist is aprepitant.

In some embodiment, the composition comprises bupivacaine and aprepitant.

In some embodiments, the composition comprises the local anesthetic in an amount varying from about 0.1 wt% to 1wt%, or from about 0.25 wt% to 30 wt%, or from about 0.1 wt% to 10 wt%, or from about 1 wt% to 5 wt%, or from about 0.25 wt% to 3.5 wt%, or from 2 wt% to 5 wt%, or from about 0.5 wt% to 4%, or from about 0.1 wt% to 5wt% by weight of the composition.

In some embodiments, the composition comprises the NK-1 receptor antagonist in an amount varying from about 0.001 wt% to 30 wt%, or from about 0.25 wt% to 10 wt%, or from about 0.01 wt% to 10 wt%, or from about 0.1 wt% to 5 wt%, or from about 0.1 wt% to 3.5 wt%, or from 0.1 wt% to 2 wt%, or from about 0.0.1 wt% to 1%, or from about 0.01 wt% to 2 wt% by weight of the composition.

In some embodiments, the weight ratio of local anesthetic: NK-1 receptor antagonist ranges from about 100:1 to 1:1, 100:1 to 4:1, 100:1 to 10:1, 75:1 to 1:1, 75:1 to 4:1, 75:1 to 10:1, 50:1 to 1:1, 50:1 to 4:1, 50:1 to 10:1, 25:1 to 1:1, 25:1 to 4:1, 25:1 to 10:1, 10:1 to 1:1.

In one embodiment, the composition comprises bupivacaine and aprepitant, wherein the weight ratio of bupivacaine: aprepitant ranges from about from about 100:1 to 1:1, 100:1 to 4:1, 100:1 to 10:1, 75:1 to 1:1, 75:1 to 4:1, 75:1 to 10:1, 50:1 to 1:1, 50:1 to 4:1, 50:1 to 10:1, 25:1 to 1:1, 2.5:1 to 4:1, 25:1 to 10:1, 10:1 to 1:1.

In one embodiment, the composition comprises ropivacaine and aprepitant.

In one embodiment, the composition comprises ropivacaine and aprepitant, wherein the weight ratio of ropivacaine:aprepitant ranges from about 100:1 to 1:1, 100:1 to 4:1, 100:1 to 10:1, 75:1 to 1:1, 75:1 to 4:1, 75:1 to 10:1, 50:1 to 1:1, 50:1 to 4:1, 50:1 to 10:1, 25:1 to 1:1, 25:1 to 4:1, 25:1 to 10:1, 10:1 to 1:1.

In one embodiment, the composition comprises the caine type local anesthetic in an amount from about 0.001 to 10 wt%, 0.25 to 0.75 wt%, 0.25 to 2.5 wt%, 0.25 to 3.5 wt%, 0.25 to 5 wt%, 1 to 5 wt%, 1 to 3.5 wt%, 1 to 3.0 wt%, 2 to 3 wt%, 2.1 to 2.9 wt%, 2.2 to 2.8 wt%, 2.3 to 2.7 wt%, 2.4 to 2.6 wt%, or 2.25 to 2.75 wt% by weight of the composition.

In another embodiment, the composition further comprises an NK-1 receptor antagonist, wherein the NK-1 receptor antagonist is present in an amount ranging from about 0.001 wt% to 30 wt%, or from about 0.25 wt% to 10 wt%, or from about 0.01 wt% to 10 wt%, or from about 0.1 wt% to 5 wt%, or from about 0.1 wt% to 3.5 wt%, or from 0.1 wt% to 2wt%, or from about 0.0.1 wt% to 1%, or from about 0.01 wt% to 2 wt% by weight of the composition .

In a further embodiment, the composition further comprises an NSAID, wherein the the ratio of amide-type local anesthetic:NSAID ranges from about 10:1 to 50:1, 15:1 to 50:1, 20:1 to 50:1, 25:1 to 50:1, 15:1 to 45:1, 20:1 to 45:1, 25:1 to 45:1, 30:1 to 45:1, 20:1 to 30:1, 20:1 to 35:1, 20:1 to 40:1, or 30:1 to 15:1. In another embodiment, the ratio of amide-type local anesthetic:NSAID is about 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1,44:1, 45:1, 46:1, 47:1, 48:1, 49:1, 50:1, 51:1, 52:1, 53:1, 54:1, 55:1, 56:1, 57:1, 58:1, 59:1, or 60:1. In another embodiment, the amide-type local anesthetic is of the caine classification.

In one embodiment, the composition comprises bupivacaine and meloxicam, wherein the ratio of bupivacaine: meloxicam ranges from about 10:1 to 50:1, 15:1 to 50:1, 20:1 to 50:1, 25:1 to 50:1, 15:1 to 45:1, 20:1 to 45:1, 25:1 to 45:1, 30:1 to 45:1, 20:1 to 30:1, 20:1 to 35:1, 20:1 to 40:1, or 30:1 to 35:1.. In another embodiment, the ratio of bupivacaine:meloxicam is about 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1,44:1, 45:1, 46:1, 47:1, 48:1, 49:1, 50:1, 51:1, 32:1, 53:1,54:1, 55:1, 56:1, 57:1, 58:1, 59:1, or 60:1.

In one embodiment, the composition further comprises an NSAID in an amount above about 0.01 wt% of the composition, above about 0.1 wt% of the composition, or above about 0.25 wt%, or between about 0.01-10 wt%, or between about 0.01-7.5 wt%, or between about 0.01-5.0 wt%, or between about 0.01-3.5 wt%.

In another embodiment, the composition further comprises an NSAID, preferably meloxicam, wherein the NSAID is present in an amount ranging from about 0.005 to 0.006 wt%, 0.005 to 0.007 wt%, 0.005 to 0.008 wt%, 0.005 to 0.009 wt%, 0.005 to 1 wt%, 0.005 to 0.75 wt%, 0.005 to 0.5 wt%, 0.005 to 0.25 wt%, 0.005 to 0.125 wt%, 0.01 to 1 wt%, 0.01 to 0.75 wt%, 0.01 to 0.5 wt%, 0.01 to 0.25 wt%, 0.01 to 0.125 wt%, 0.020 to 0.100 wt%, 0.030 to 0.100 wt%, 0.040 to 0.100 wt%, 0.050 to 0.090 wt%, 0.060 to 0.080 wt%, 0.070 to 0.080 wt% by weight of the composition.

In yet another embodiment, the compostion further comprises meloxicam present in an amount which is above 0.001 wt% and less than 1 wt%, less than 0.5 wt%, less than 0.1 wt%, less than 0.08 wt%, less than 0.5 wt%, or less than 0.1 wt% by weight of the composition.

In one embodiment, the composition is an aqueous based solution.

In one embodiment, the composition is injectable.

In another embodiment, the composition is suitable for administration as an intramuscular injection, transdermally, topically, as a subcutaneous injection, as a perineural injection or to a wound.

In one embodiment, the delivery vehicle is a sustained-release delivery vehicle.

In one embodiment, the sustained-release delivery vehicle is a polymeric composition, a liposomal composition, a microsphere composition, a non-polymeric composition or an implantable device.

In one embodiment, the sustained release delivery vehicle is not a microsphere composition.

In one embodiment, the sustained release delivery vehicle is not a liposomal composition.

In one embodiment, the sustained release delivery vehicle is not a non-polymeric composition.

In one embodiment, the sustained release delivery vehicle is not an implantable device.

In one embodiment, the sustained-release delivery vehicle is not a polymeric composition.

In one embodiment, the delivery vehicle is not a sustained-release vehicle. In another embodiment, the delivery vehicle is an immediate-release vehicle. In still another embodiment, at least about 80% of the anesthetic and the NK-1 receptor antagonist are released from the delivery vehicle within a time period of about 15 minutes, 30 minutes, 45 minutes, 60 minutes, 90 minutes or 2 hours. In yet another embodiment, the release of the anesthetic and the NK-1 receptor antagonist is determined in an in vitro dissolution test at 37 °C.

In one embodiment, the composition has a viscosity of less than 150,000 mPa-s when viscosity is measured at 25 °C using a viscometer. In another embodiment, the composition has a viscosity of less than 100,000 mPa-s when viscosity if measured at 25 °C using a viscometer. In one embodiment, the composition has a viscosity of less than 50,000 mPa-s when viscosity is measured at 25 °C using a viscometer. In yet another embodiment, the composition has a viscosity ranging from about 1,000 mPa-s to 150,000 mPa-s when measured at 25 °C using a viscometer. In some embodiments, the viscometer is a cone and plate viscometer.

In yet another embodiment, the sustained-release delivery vehicle is a liposome selected from the group consisting of small unilamellar vesicles (SUV), large unilamellar vesicles (LUV), multilamellar vesicles (MLV) and multivesicular liposomes (MVL).

In one embodiment, the caine type local anesthetic is entrapped in an aqueous space of the liposome or in a lipid layer of the liposome.

In one embodiment, the NK-1 receptor antagonist is entrapped in an aqueous space of the liposome or in a lipid layer of the liposome.

In still another embodiment, the sustained-release delivery vehicle is a microsphere comprised of a bioerodible or biodegradable polymer.

In one embodiment, the amide-type local anesthetic and the NK-1 receptor antagonist are entrapped in the microsphere.

In one embodiment, the implantable device is an osmotic pump with a reservoir comprising the caine type local anesthetic and the NK-1 receptor antagonist.

In one embodiment, the sustained-release delivery vehicle is a non-polymeric formulation comprising sucrose acetate isobutyrate.

In one embodiment, the sustained-release delivery vehicle is a polymeric formulation in the form of a semi-solid polymer formulation comprising a polymer.

In one embodiment, the polymer is a bioerodible or biodegradable polymer.

In yet another embodiment, the polymer formulation forms an implant or depot in situ.

In still another embodiment, the polymer is selected from the group consisting of polylactides, polyglycolides, poly(lactic-co-glycolic acid) copolymers, polycaprolactones, poly-3-hydroxybutyrates, and polyorthoesters.

In a further embodiment, the sustained-release delivery vehicle is a polymeric formulation in the form of a semi-solid polymer formulation comprising a polyorthoester.

In a further embodiment related to any one or more of the foregoing embodiments, the composition is a semi-solid or solid composition.

In one embodiment, the delivery vehicle is a sustained-release delivery vehicle. In one embodiment, the sustained-release vehicle is a polymeric vehicle or formulation.

In another embodiment, the sustained-release polymeric vehicle is a solid or semi-solid vehicle comprising a bioerodible or biodegradable polymer.

In an embodiment, the biodegradable or bioerodible polymeric formulation comprises a polymer selected from the group consisting of polylactide, polyglycolide, a poly(lactic-co-glycolic acid) copolymer, polycaprolactone, poly-3-hydroxybutyrate, or a polyorthoester.

In one embodiment, the polyorthoester is selected from the polyorthoesters represented by Formulas I, II, III and IV set forth herein.

In yet a particular embodiment related to the foregoing, the polyorthoester is represented by Formula I.

In an alternative embodiment, the sustained-release vehicle does not comprise a polylactide, polyglycolide, a poly(lactic-co-glycolic acid) copolymer, polycaprolactone, poly-3-hydroxybutyrate, or a polyorthoester. In another embodiment, the sustained-release vehicle does not comprise a polyorthoester. In still another embodiment, the sustained-release vehicle does not comprise a polyorthoester represented by Formula I, II, III or IV. In still another embodiment, the sustained-release vehicle does not comprise a polyorthoester represented by Formula I.

In yet an additional embodiment, the composition or delivery vehicle further comprises a solvent. The solvent may be either protic or aprotic in nature. In one embodiment, the composition comprises as the delivery vehicle a polyorthoester and a solvent.

In another embodiment, the sustained-release delivery vehicle is selected from the group consisting of microspheres, microparticles, and homogeneous or heterogeneous matrix depots. In one embodiment, the microsphere, microparticle or depot vehicle is biodegradable or bioerodible.

In another embodiment, the sustained-release delivery vehicle is a liposomal formulation or a lipid-based formulation.

In another embodiment, the sustained-release formulation is a polymeric-based solid or semi-solid implant where the local anesthetic and the NK-1 receptor antagonist are dispersed in the polymeric-based implant. In one embodiment, the implant is a solid polymeric-based vehicle in the form of a suture or a staple.

In a particular embodiment, the composition is effective to provide measurable plasma concentrations of the caine type local anesthetic and/or the NK-1 receptor antagonist for a period of at up to about 3 days or up to about 5 days or up to about 7 days or up to about 10 days following administration, or for a period of about 1 day to about 3 days, about 1 day to about 5 days, about 1 day to about 7 days, about 3 days to about 5 days, about 3 days to about 7 days or about 5 days to about 10 days. In one embodiment, the composition is effective to provide measurable plasma concentrations of the caine type local anesthetic and/or the NK-1 receptor antagonist for a period of about 1 day to about 3 days, about 1 day to about 5 days, about 1 day to about 7 days, about 1 day to about 10 days, about 1 day to about 12 days, about 1 day to about 13 days, or about 1 day to about 14 days following administration. In another embodiment, the plasma concentration of the caine type local anesthetic and/or the the NK-1 receptor antagonist is measured by LC/MS/MS (liquid chromatography/tandem mass spectrometry).

In a particular embodiment, the composition is effective to release a significant portion of both the caine type anesthetic and the NK-1 receptor antagonist from the composition, such that about 80% by weight or more of the caine type anesthetic and/or the NK-1 receptor antagonist is released, either *in vitro* or *in vivo,* over a period of up to about 3 days or up to about 5 days or up to about 7 days or up to about 10 days following administration or initiation of an in vitro drug release experiment (e.g. as described in Example 5), or for a period of about 1 day to about 3 days, about 1 day to about 5 days, about 1 day to about 7 days or about 5 days to about 10 days, about 2 days to about 5 days, about 3 days to about 5 days, about 4 days to about 5 days, about 2 days to about 4 days, about 3 days to about 4 days, or about 3 days, about 4 days or about 5 days.

In one embodiment, the composition is a synergistic composition wherein release of the the caine type anesthetic and the NK-1 receptor antagonist in combination provides a synergistic level of pain relief that is greater than a level of pain relief provided by an additive effect of adding the the caine type anesthetic and the NK-1 receptor antagonist independently. In another embodiment, the composition provides a duration of pain relief that is longer than a duration resulting from an additive effect of adding the caine type anesthetic and the NK-1 receptor antagonist independently.

In one embodiment, the composition is a synergistic composition comprising bupivacaine and aprepitant, wherein the weight ratio of bupivacaine: aprepitant ranges from about 1:100 to 100:1, 20:1 to 1:20, 1:5 to 5:1, 1:1 to 15:1, or 1: 1 to 1:15. In yet another embodiment, administration of the synergistic composition comprising bupivacaine and aprepitant to a subject provides a synergistic level of pain relief that is greater than the pain relief provided by the additive effect of administering the bupivacaine and aprepitant independently. In another embodiment, the subject is a post-operative patient. In another embodiment, the subject is a patient during operation. In some embodiments, the subject is a patient to receive an operation.

In one embodiment, the composition is a synergistic composition comprising ropivacaine and aprepitant, wherein the weight ratio of ropivacaine:aprepitant ranges from about 1:100 to 100:1, 20:1 to 1:20, 1:5 to 5:1, 1:1 to 15:1, or 1:1 to 1:15.

In yet another embodiment, administration of the synergistic composition comprising ropivacaine and aprepitant to a subject provides a synergistic level of pain relief that is greater than the pain relief provided by the additive effect of administering the ropivacaine and aprepitant independently. In another embodiment, the subject is a post-operative patient. In another embodiment, the subject is a patient during operation. In some embodiments, the subject is a patient to receive an operation.

In some embodiments, the delivery vehicle comprises a polyorthoester, a solvent comprising a triglyceride viscosity reducing agent, and a polar aprotic solvent in which the polyorthoester is miscible to form a single phase. The triglyceride viscosity reducing agent comprises three fatty acid groups each independently comprising between 1-7 carbon atoms, which is also referred to herein as a 'short chain' triglyceride. In another embodiment, the delivery vehicle further comprises a dicarboxylic acid.

In one embodiment the triglyceride viscosity reducing agent is glycerin triacetate (also called triacetin, 1,2,3-triacetoxypropane, or glycerol triacetate).

In one embodiment, the polar aprotic solvent is an organic solvent having a water solubility of greater than 25% by weight of the solvent in water at room temperature.

In one embodiment, the polar aprotic solvent has a dipole moment greater than about 2 Debye (D).

In one embodiment, the polar aprotic solvent is in a class selected from the group consisting of an amide, an ether, a ketone, and a sulfoxide.

In another embodiment, the polar aprotic solvent is a sulfoxide selected from the group consisting of dimethyl sulfoxide and decylmethylsulfoxide.

In yet another embodiment, the polar aprotic solvent is an amide selected from the group consisting of 2-pyrrolidone, dimethyl formamide, N-methyl-2-pyrrolidone, and dimethyl acetamide.

In one embodiment, the polar aprotic solvent is an ether selected from dimethyl isosorbide and tetrahydrofuran.

In one embodiment, the polar aprotic solvent is a ketone selected from the group consisting of acetone and methyl ethyl ketone.

In one embodiment, the polar aprotic solvent is a lactone selected from the group consisting of ester-caprolactone and butyrolactone.

In one embodiment, the polar aprotic solvent is an ester of an alcohol, propylene carbonate (4-methyl-1,3-diololan-2-one).

In one embodiment, the polar aprotic solvent is 1-dodecylazacycloheptan-2-one.

In one embodiment, the polar aprotic solvent is dimethylsulfoxide (DMSO) or N-methyl pyrrolidone (NMP) or dimethyl acetamide (DMAC).

In one embodiment, the polar aprotic solvent is dimethylsulfoxide (DMSO) or N-methyl pyrrolidone (NMP).

In one embodiment, the polar aprotic solvent is dimethylsulfoxide (DMSO).

In one embodiment, the dicarboxylic acid is maleic acid.

In one embodiment, the polyorthoester is the polyorthoester represented by the structure shown as Formula I, 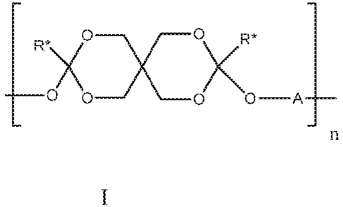 where: R* is a methyl, ethyl, propyl or butyl, n is the number of repeating units and is an integer ranging from 5 to 400, and A in each subunit is R¹ or R³.

In one embodiment, R* is ethyl.

In one embodiment, A corresponds to R¹, where R¹ is 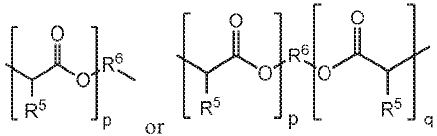 where p and q are each independently integers ranging from about 1 to 20, each R⁵ is independently hydrogen or C₁₋₄ alkyl; and R⁶ is: 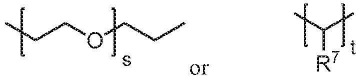 where s is an integer from 0 to 10; t is an integer from 2 to 30; and R⁷ is hydrogen or C₁₋₄ alkyl. In another embodiment, R⁷ is C1, C2, C3, or C4 alkyl. In a particular embodiment, R⁷ is H. In still another embodiment, the R¹ subunits are α-hydroxy acid-containing subunits. In another embodiment, p and q are each independently selected from 1, 2, 3, 4, 5, 6, 7, 8, 9, 10, 11, 12, 13, 14, 15, 16, 17, 18, 19, and 20. In yet another embodiment, R⁵ is independently hydrogen, or C1, C2, C3, or C4 alkyl.

In one embodiment, A corresponds to R³, where R³ is: 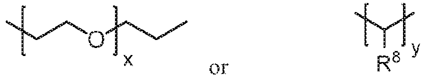 and x is an integer ranging from 1 to 100. In another embodiment, x is selected from 0, 1, 2, 3, 4, and 5; y is an integer in a range from 2 to 30; and R⁸ is hydrogen or C₁₋₄ alkyl. In still another embodiment, R⁸ is a C1, C2, C3 or C4 alkyl. In another embodiment, R⁸ is H.

In one embodiment, the polyorthoester is one in which A is R¹ or R³, where R¹ is 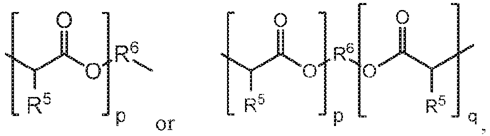 where p and q are each independently selected from 1, 2, 3, 4, 5, 6, 7, 8, 9, 10, 11, 12, 13, 14, 15, 16, 17, 18, 19, and 20 in any repeating unit, where the average number of p or the average number of the sum of p and q (p + q) is between about 1 and 7; x and s are each independently an integer ranging from 0 to 10; and t and y are each independently an integer ranging from 2 to 30. In another embodiment, the sum of p and q is 1, 2, 3, 4, 5, 6 or 7 in any repeating unit of R¹. In yet another embodiment, R⁵ is H.

In one embodiment, A is R¹ or R³, where R¹ is 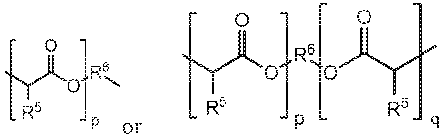 and p and q are each independently integers ranging from about 1 and 20, about 1 and 15, or about 1 and 10 in any repeating unit of R¹, where the average number of p or the average number of the sum of p and q (i.e., p + q) is between about 1 and 7. In another embodiment, *x* and *s* each independently range from 0 to about 7 or from 1 to about 5. In still another embodiment, t and y each independently range from 2 to 10.

In one embodiment, R⁵ is hydrogen or methyl.

In one embodiment, s and x are each independently selected from 1, 2, 3, 4, 5, 6, 7 and 8. In another embodiment, s is 2. In still another embodiment, x is 2.

In one embodiment, the polyorthoester comprises alternating residues of 3,9-diethyl-3,9-2,4,8,10-tetraoxaspiro[5.5]undecane-3,9-diyl and A : 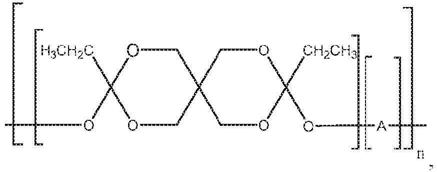 where A is as described above.

In one particular embodiment related to the polyorthoester in the delivery system, the polyorthoester has a molecular weight ranging from about 2,500 daltons to 10,000 daltons.

In one embodiment related to the delivery vehicle, the polyorthoester is represented by the structure shown as Formula I and is in an amount ranging from about 5 to 95 wt%, 65 to 75 wt%, 60 to 70 wt%, 50 to 70 wt%, 20 to 70 wt%, 50 to 65 wt%, or 55 to 65 wt% by weight of the composition. In another embodiment, the polyorthoester represented by the structure shown as Formula I is in an amount of about 40 percent, 45 percent, 50 percent, 55 percent, 60 percent, 65 percent, or 70 percent by weight of the composition.

In one embodiment related to the delivery vehicle, the triglyceride viscosity reducing agent is present in an amount ranging from about 1 wt% to 50 wt%, 1 wt% to 45 wt%, 1 wt% to 40 wt%, 1 wt% to 35 wt5, 1 wt% to 25 wt%, 1 wt% to 20 wt%, 1 wt% to 15 wt%, 1 wt% to 10 wt%, or about 1 wt% to 5 wt% of the composition.

In one embodiment related to the delivery vehicle, the aprotic solvent is present in an amount ranging from about 1 wt% to 50 wt%, 1 wt% to 45 wt%, 1 wt% to 40 wt%, 1 wt% to 35 wt5, 1 wt% to 25 wt%, 1 wt% to 20 wt%, 1 wt% to 15 wt%, 1 wt% to 10 wt%, or about 1 wt% to 5 wt% of the composition.

In one embodiment related to the delivery vehicle, the dicarboxylic acid is present in an amount ranging from about 0.01 to 0.5 wt%, 0.02 to 0.4 wt%, 0.03 to 0.3 wt%, 0.04 to 0.2 wt%, 0.04 to 0.15 wt%, 0.05 to 0.15 wt%, 0.02 to 0.08 wt%, 0.03 to 0.07 wt%, 0.04 to 0.06 wt%, 0.1 to 0.2 wt%, 0.125 to 0.175 wt%, or 0.14 to 0.16 wt% by weight of the composition. In another embodiment related to the delivery vehicle, the dicarboxylic acid is present in an amount ranging from about 0.01 wt% to 0.6 wt%, 0.01 wt%to 0.05 wt%, 0.05 wt%to 0.6 wt%, 0.01 wt% to 0.15 wt%, 0.05 wt% to 0.15 wt%, 0.01 wt%, 0.02 wt%, 0.03 wt%, 0.04 wt%, 0.050 wt%, 0.060 wt%, 0.070 wt%, 0.080 wt%, 0.090 wt%, 0.100 wt%, 0.125 wt%, 0.150 wt%, 0.175 wt%, 0.2 wt%, 0.3 wt%, 0.4 wt%, 0.5 wt%, or 0.6wt% by weight of the composition.

In a specific embodiment, the composition is administered as a nerve block to treat a painful condition in a subject in need thereof.

In a further specific embodiment, the composition is administered as a nerve block as prophylactic treatment of a painful condition, such as administration prior to surgery for the treatment of pain after surgery, in a subject in need thereof.

In another aspect, a method of treating postsurgical pain is provided which comprises administration of therapeutically effective amount of a NK-1 receptor antagonist in combination with a pain medication to a subject in need thereof. The post-surgical pain can be neuropathic and/or nociceptive pain. In some embodiments, the post-surgical pain does not comprise neuropathic pain. In some embodiments, the post-surgical pain is nociceptive pain.

Pain medication refers to an active agent for the treatment of pain. The active agent for the treatment of pain is selected from local anesthetics, opioids, non-steroid anti-inflammatory drug (NSAID), steroids, analgesics, anticonvulsants, serotonin and norepinephrine reuptake inhibitors (SNRIs), acetaminophen, and tricyclic antidepressants. The pain medication can include more than one active agent.

In some embodiments, the NK-1 receptor antagonist and the pain medication are administered concurrently wherein the NK-1 receptor antagonist and the pain medication are comprised in a single pharmaceutical composition or in separate/different pharmaceutical compositions.

In some embodiments, the NK-1 receptor antagonist and the pain medication are administered sequentially wherein the NK-1 receptor antagonist is administered first and the pain medication second or vice versa. Such sequential administration may be close in time or remote in time. In some embodiments, both the NK-1 receptor antagonist and the pain medication are administered before surgery. In some embodiments, both the NK-1 receptor antagonist and the pain medication are administered during surgery. In some embodiments, both the NK-1 receptor antagonist and the pain medication are administered after surgery. In some embodiments, one of the NK-1 receptor antagonist and the pain medication is administered before surgery and the other is aministered after surgery. In some embodiments, the NK-1 receptor antagonist is administered before surgery and the pain medication is administered after surgery. In some embodiments, the pain medication is administered before surgery and the NK-1 receptor antagonist is administered after surgery. In some embodiments, the NK-1 receptor antagonist is administered before surgery and the pain medication is administered during surgery. In some embodiments, the pain medication is administered before surgery and the NK-1 receptor antagonist is administered during surgery.

In some embodiments, the method for treating post-surgical pain comprises administering to a patient in need thereof a therapeutically effective amount of NK-1 receptor antagonist in combination with pain medications selected from local anesthetics, opioids, NSAID, anticonvulsants, SNRIs, acetaminophen, and tricyclic antidepressants. In some embodiments, the method for treating post-surgical pain comprises administering to a patient in need thereof a therapeutically effective amount of a NK-1 receptor antagonist in combination with a local anesthetic. In some embodiments, the method for treating post-surgical pain comprises administering to a patient in need thereof a therapeutically effective amount of NK-1 receptor antagonist in combination with two pain medications selected from local anesthetics, opioids, NSAID, steroids, analgesics, anticonvulsants, SNRIs, acetaminophen, and tricyclic antidepressants. In some embodiments, the method for treating post-surgical pain comprises administering to a patient in need thereof a therapeutically effective amount of a NK-1 receptor antagonist in combination with a local anesthetic and a NSAID. In some embodiments, the method for treating post-surgical pain comprises administering to a patient in need thereof a therapeutically effective amount of a NK-1 receptor antagonist in combination with a local anesthetic and a steroid. In some embodiments, the method for treating post-surgical pain comprises administering to a patient in need thereof a therapeutically effective amount of a NK-1 receptor antagonist in combination with a local anesthetic and an analgesic. In some embodiments, the method for treating post-surgical pain comprises administering to a patient in need thereof a therapeutically effective amount of a NK-1 receptor antagonist in combination with an opioid. In some embodiments, the method for treating post-surgical pain comprises administering to a patient in need thereof a therapeutically effective amount of a NK-1 receptor antagonist in combination with acetaminophen. In some embodiments, the method for treating post-surgical pain comprises administering to a patient in need thereof a therapeutically effective amount of a NK-1 receptor antagonist in combination with acetaminophen and an opioid.

In some embodiments, the NK-1 receptor antagonist is administered systemically, wherein the administration can take place via enteral administration (absorption of the drug through the gastrointestinal tract) or parenteral administration (generally injection, infusion, or implantation). In some embodiments, the NK-1 receptor antagonist is administered locally, such as into a wound site. In some embodiments, the NK-1 receptor antagonist is administered via an injection selected from subcutaneous, intramuscular, intravenous, intraperitoneal, intracardiac, intraarticular and intracavernous injection. In some embodiments, the NK-1 receptor antagonist is administered via intravenous injection. In some embodiments, the NK-1 receptor antagonist is administered via intramuscular injection.

In some embodiments, the pain medication is administered systemically, wherein the administration can take place via enteral administration (absorption of the drug through the gastrointestinal tract) or parenteral administration (generally injection, infusion, or implantation). In some embodiments, the pain medication is administered locally, such as to a wound site. In some embodiments, pain medication is administered via an injection selected from subcutaneous, intramuscular, intravenous, intraperitoneal, intracardiac, intraarticular and intracavernous injection. In some embodiments, the pain medication is administered via intravenous injection. In some embodiments, the pain medication is administered via intramuscular injection

In some embodiments, the NK-1 receptor antagonist is selected from the group consisting of aprepitant, rolapitant, netupitant, lanepitant, vestipitant, orvepitant maleate, casopitant, ezlopitant, serlopitant, fosaprepitant, befetupitant and maropitant, or a pharmaceutically acceptable salt thereof.

In some embodiments, the NK-1 receptor antagonist is aprepitant.

In some embodiment, the local anesthetic is selected from the group consisting of bupivacaine, ropivacaine, levobupivacaine, dibucaine, mepivacaine, procaine, lidocaine, and tetracaine. In some embodiments, the local anesthetic is bupivacaine or ropivacaine. In some embodiments, the local anesthetic is bupivacaine. In some embodiments, the local anesthetic is ropivacaine.

In some embodiments the NSAID is selected from the group consisting of diflunisal, indomethacin, tolmetin, sulindac, etodolac, ketorolac, diclofenac, nabumetone, ibuprofen, dexibuprofen, naproxen, fenoprofen, ketoprofen, dexketoprofen, flurbiprofen, oxaprozin, loxoprofen, mefenamic acid, meclofenamic acid, flufenamic acid, tolfenamic acid, meloxicam, piroxicam, tenoxicam, droxicam, lornoxicam, isoxicam, celecoxib, rofecoxib, and valdecoxib. In a specific embodiment, the NSAID is meloxicam.

In some embodimments the steroid is selected from the group consisting of 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, dexamethasone 21-acetate, dexamethasone 21-phosphate di-Na salt, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide and a combinations thereof.

In some embodiments the analgesic agent is selected from the group consisting of capsaicinoids including capsaicin, dihydrocapsaicin, nordihydrocapsaicin, homocapsaicin, homodihydrocapsaicin, and nonivamide and combinations thereof.

### BRIEF DESCRIPTION OF DRAWINGS

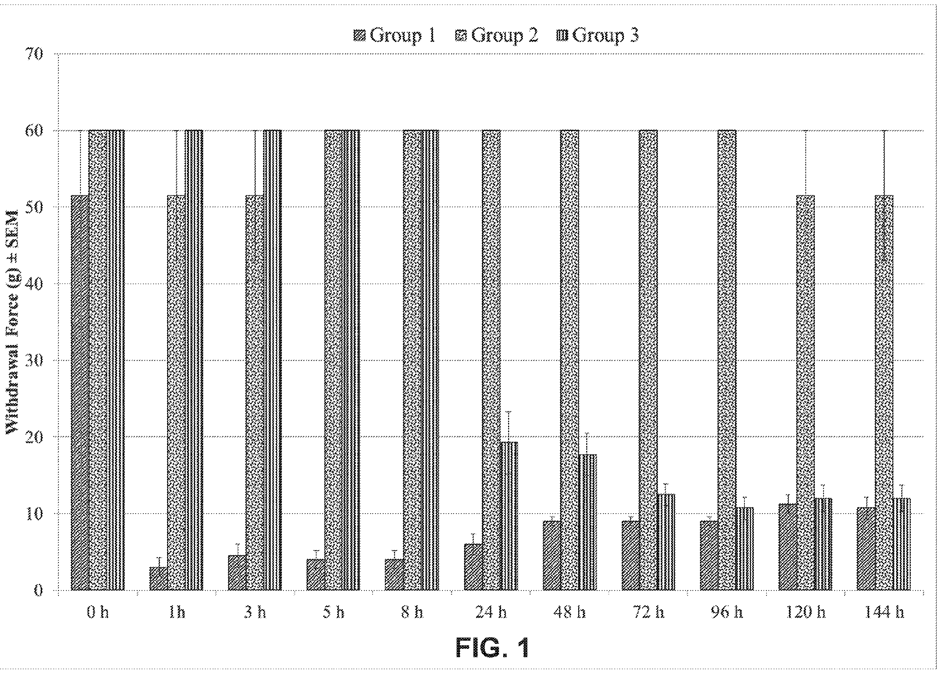

FIG.1 is a bar graph of withdrawal force, in gram force, as a function of time, in hours, after administration *in vivo* to animals of a composition comprised of the test compositions denoted as follows: (i) IV aprepitant dosed once prior to surgery and daily for 5 days after surgery at a dose of 6mg/kg (Group 1, bars with diagonal line fill); (ii) IV aprepitant dosed once prior to surgery and daily for 5 days after surgery at a dose of 6mg/kg and an extended release formulation of bupivacaine and meloxicam dosed 3.4 mL once during surgery by injection into the wound margins (Group 2, bars with dotted fill); (iii) IV aprepitant dosed once 30 minutes before surgery at a dose of 6mg/kg and an extended release formulation of bupivacaine and meloxicam dosed 2.6 mL once during surgery by injection into the wound margins (Group 3, bars with vertical line fill).

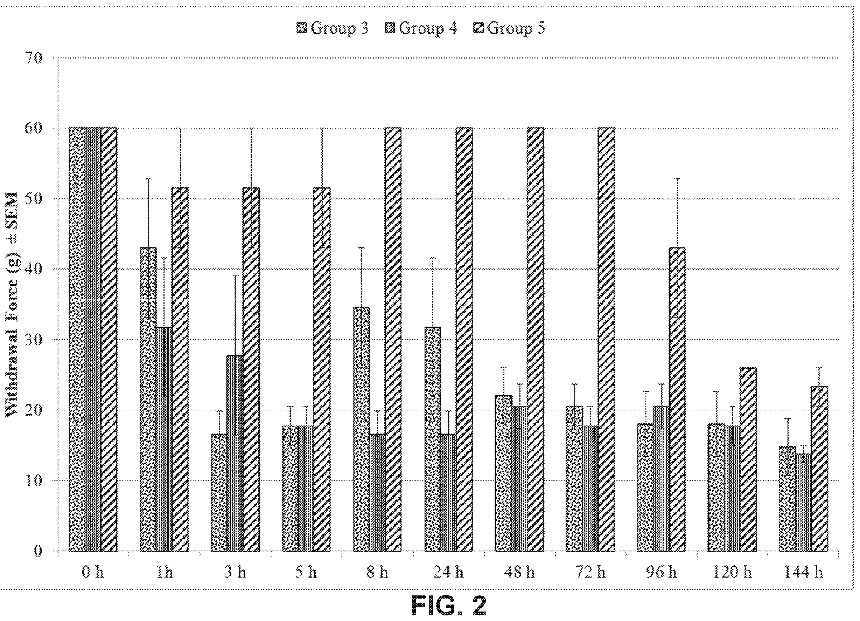

FIG. 2 is a bar graph of withdrawal force, in gram force, as a function of time, in hours after administration *in vivo* to animals of a composition comprised of the test compositions denoted as follows: (i) IV aprepitant dosed once prior to surgery and daily for 5 days after surgery at a dose of 3.0 mg/kg and an extended release formulation of bupivacaine and meloxicam dosed 3.4 mL once during surgery by injection into the wound margins (Group 5, bars with diagonal line fill), (ii) IV aprepitant dosed once prior to surgery and daily for 5 days after surgery at a dose of 6.0 mg/kg and an extended release formulation of bupivacaine dosed 3.4 mL once during surgery by injection into the wound margins (Group 3, bars with dotted fill), and (iii) IV aprepitant dosed once prior to surgery and daily for 5 days after surgery at a dose of 6.0 mg/kg and an extended release formulation of meloxicam dosed 3.4 mL once during surgery by injection into the wound margins (Group 4, bars with with vertical line fill).

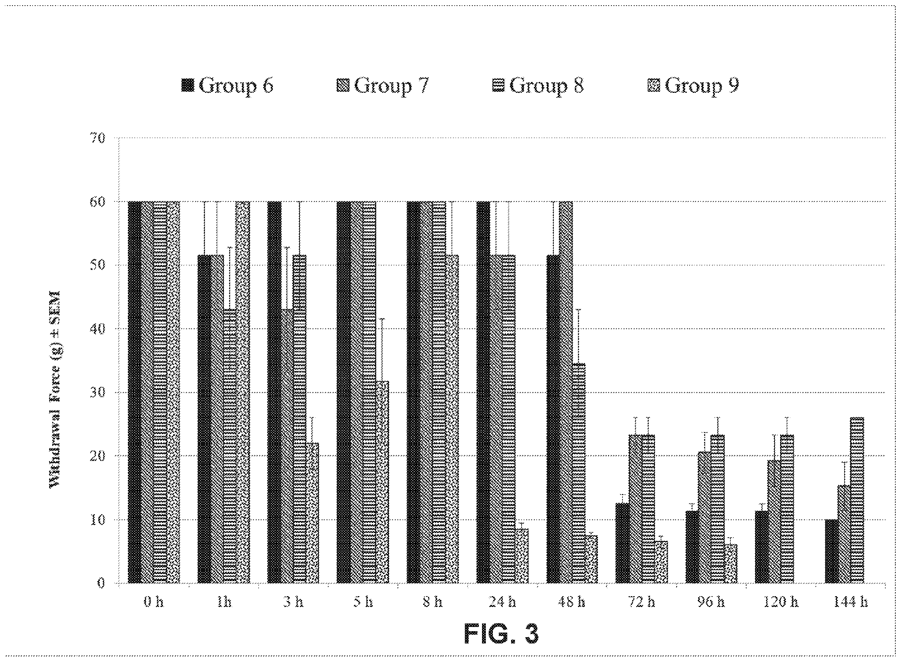

FIG. 3 is a bar graph of withdrawal force, in gram force, as a function of time, in hours after administration *in vivo* to animals of a composition comprised of the test compositions denoted as follows: (i) IV aprepitant dosed once prior to surgery and daily for 5 days after surgery at a dose of 1.5 mg/kg and an extended release formulation of bupivacaine and meloxicam dosed 3.4 mL once during surgery by injection into the wound margins (Group 6, bars with solid fill), (ii) ) IV aprepitant dosed once prior to surgery and once on days 2 and 3 after surgery at a dose of 6.0 mg/kg and an extended release formulation of bupivacaine and meloxicam dosed 3.4 mL once during surgery by injection into the wound margins (Group 7, bars with diagonal line fill), (iii) IV aprepitant dosed once prior to surgery and once on days 3, 4, and 5 after surgery at a dose of 6.0 mg/kg and an extended release formulation of bupivacaine and meloxicam dosed 3.4 mL once during surgery by injection into the wound margins (Group 8, bars with horizontal line fill); and (iv) oral suspension of meloxicam dosed once at 0.5 mg/kg prior to surgery and IV aprepitant dosed once prior to surgery and once daily for 5 days after surgery at a dose of 6.0 mg/kg and an extended release formulation of bupivacaine dosed 3.4 mL once during surgery by injection into the wound margins (Group 9, bars with dotted fill).

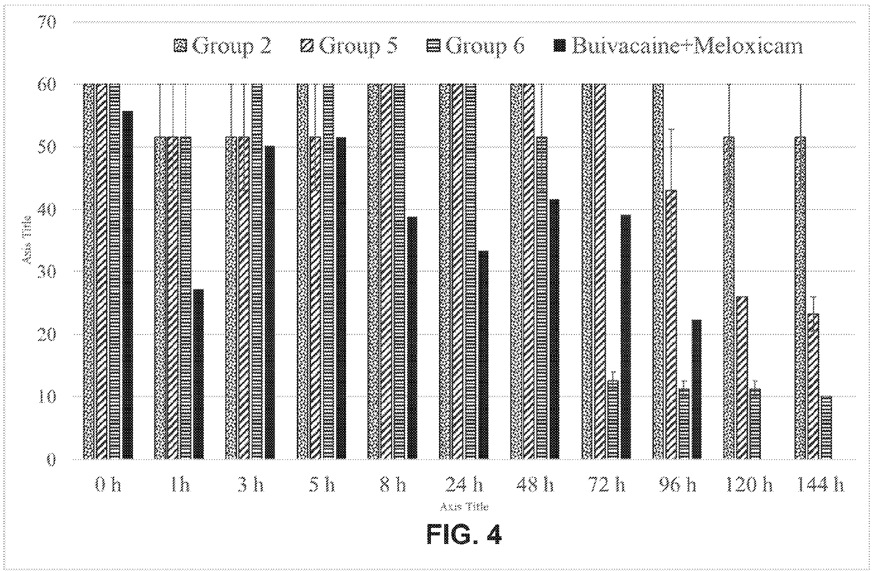

FIG. 4 is a bar graph of withdrawal force, in gram force, as a function of time, in hours after administration *in vivo* to animals of a composition comprised of the test compositions denoted as follows: (i) IV aprepitant dosed once prior to surgery and daily for 5 days after surgery at a dose of 6.0 mg/kg and an extended release formulation of bupivacaine and meloxicam dosed 3.4 mL once during surgery by injection into the wound margins (Group 2, bars with dotted fill), (ii) IV aprepitant dosed once prior to surgery and daily for 5 days after surgery at a dose of 3.0 mg/kg and an extended release formulation of bupivacaine and meloxicam dosed 3.4 mL once during surgery by injection into the wound margins (Group 5, bars with diagonal line fill), (iii) IV aprepitant dosed once prior to surgery and daily for 5 days after surgery at a dose of 1.5 mg/kg and an extended release formulation of bupivacaine and meloxicam dosed 3.4 mL once during surgery by injection into the wound margins (Group 6, bars with horizontal line fill), and (iv) an extended release formulation of bupivacaine and meloxicam dosed 3.4 mL once during surgery by injection into the wound margins (historical data, bars with solid fill).

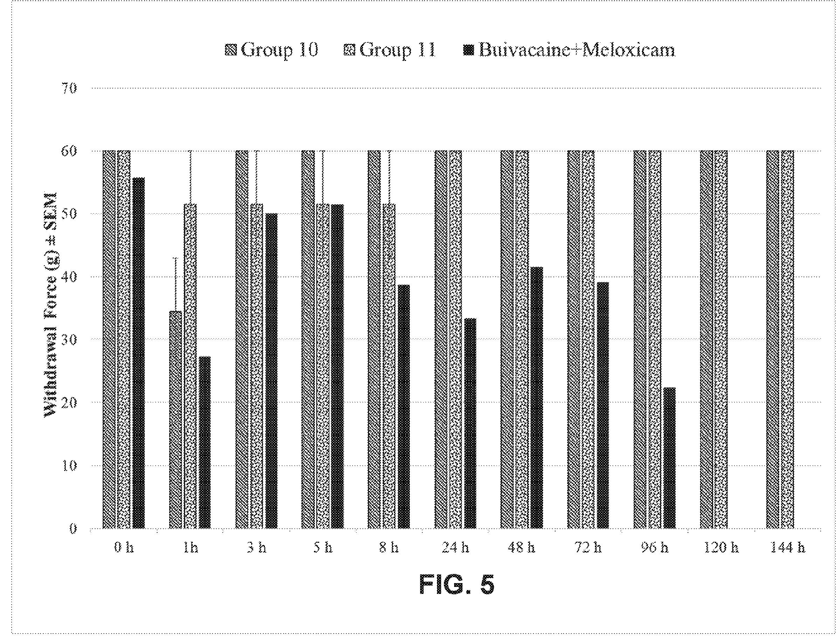

FIG. 5 is a bar graph of withdrawal force, in gram force, as a function of time, in hours and days, after administration *in vivo* to animals of a composition comprised of the test compositions denoted as follows: (i) an extended release formulation of bupivacaine,meloxicam, and aprepitant (high concentration), dosed with 3.4 mL once during surgery by injection into the wound margins (Group 10, bars with diagonal line fill), (ii) an extended release formulation of bupivacaine,meloxicam, and aprepitant,dosed with 3.4 mL once during surgery by injection into the wound margins (Group 11, bars with dotted fill), and (iii) an extended release formulation of bupivacaine and meloxicam dosed with 3.4 mL once during surgery by injection into the wound margins (historical data, bars with solid fill).

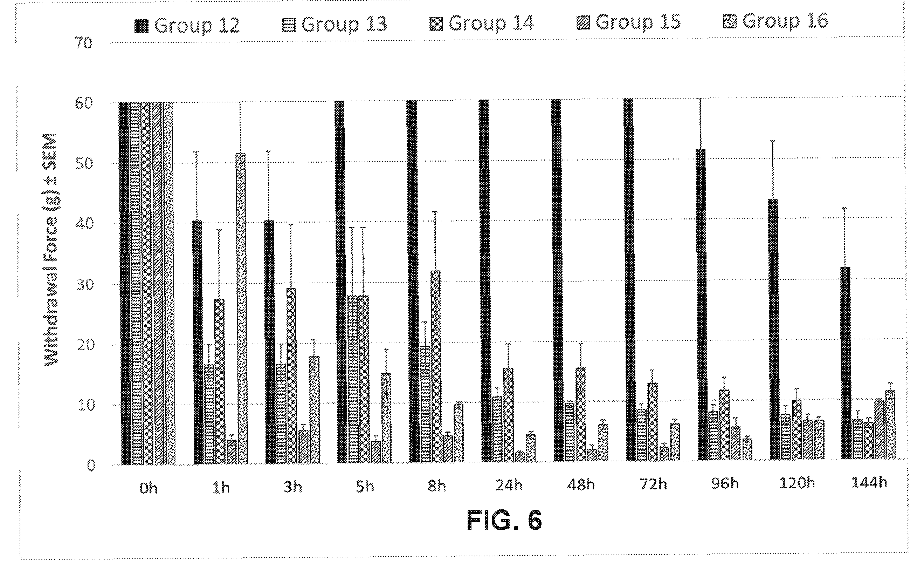

FIG. 6 is a bar graph of withdrawal force, in gram force, as a function of time, in hours and days, after administration *in vivo* to animals of a composition comprised of the test compositions denoted as follows: (i) an extended release formulation of bupivacaine,meloxicam, and aprepitant, dosed with 3.4 mL once during surgery by injection into the wound margins (Group 12, bars with solid fill), (ii) an extended release formulation of bupivacaine and aprepitant, dosed with 3.4 mL once during surgery by injection into the wound margins (Group 13, bars with horizontal fill), (iii) an extended release formulation of bupivacaine, a low concentration of meloxicam, and aprepitant, dosed with 3.4 mL once during surgery by injection into the wound margins (Group 14, historical data, bars with checkerboard fill), (iv) an extended release formulation of aprepitant, dosed with 3.4 mL once during surgery by injection into the wound margins (Group 15, bars with diagonal fill), and (v) an extended release formulation of meloxicam and aprepitant, dosed with 3.4 mL once during surgery by injection into the wound margins (Group 16, bars with dotted fill).

### DETAILED DESCRIPTION

Various aspects now will be described more fully hereinafter. Such aspects may, however, be embodied in many different forms and should not be construed as limited to the embodiments set forth herein; rather, these embodiments are provided so that this disclosure will be thorough and complete, and will fully convey its scope to those skilled in the art.

### I. DEFINITIONS

As used in this specification, the singular forms "a," "an," and "the" include plural referents unless the context clearly dictates otherwise. Thus, for example, reference to a "polymer" includes a single polymer as well as two or more of the same or different polymers, reference to an "excipient" includes a single excipient as well as two or more of the same or different excipients, and the like.

Where a range of values is provided, it is intended that each intervening value between the upper and lower limit of that range and any other stated or intervening value in that stated range is encompassed within the disclosure. For example, if a range of 1 µm to 8 µm is stated, it is intended that 2 µm, 3 µm, 4 µm, 5 µm, 6 µm, and 7 µm are also explicitly disclosed, as well as the range of values greater than or equal to 1 µm and the range of values less than or equal to 8 µm. As used herein, the term "about" means ±5%, ±10%, or ±20% of the value being modified.

The term "emulsion" or "emulsion formulation" means a colloidal dispersion of two immiscible liquids in the form of droplets, whose diameter, in general, is between 10 nanometers and 100 microns. An emulsion is denoted by the symbol O/W (oil-in-water) if the continuous phase is an aqueous solution and by W/O (water-in-oil) if the continuous phase is an oil. Other examples of emulsions such as O/W/O (oil-in-water-oil) include oil droplets contained within aqueous droplets dispersed in a continuous oil phase.

"Physically stable" emulsions will meet the criteria under USP <729>, which defines universal limits for (1) mean droplet size not exceeding 500 nm or 0.5 µm and (2) the population of large-diameter fat globules, expressed as the volume-weighted percentage of fat greater than 5 µm (PFAT5) not exceeding 0.05%, at 5 °C or room temperature for a designated storage time period. In addition, physically stable emulsions will have no visible NK-1 receptor antagonist crystals upon storage at 5 °C or room temperature for a designated time period. Crystals are considered visible when viewed at magnification of 4X to 10X. An emulsion is physically stable if it meets the criteria under USP <729> and NK-1 receptor antagonist crystals are not visible upon storage at 5 °C or room temperature for a time period equal to or at least 1 week, 2 weeks, 4 weeks, 1 month, 2 months, 6 months, 1 year or 2 years.

"Chemically stable" emulsions of the disclosure are ones in which the concentration of the active component (i.e., the drug being delivered) does not change by more than about 20% under appropriate storage conditions for at least 1 month. In certain embodiments, the concentration the NK-1 receptor antagonist in an emulsion of the present disclosure does not change by more than about 5%, 10%, 15% or 20% under appropriate storage conditions for at least 1, 2, 3, 4, 5, 6, 9, 12, 15, 18, or 24 months.

In one example, the stable emulsion compositions of the disclosure are stable over a wide range of temperatures, e.g., -20 °C to 40 °C. The compositions of the disclosure may be stored at about 5 °C to about 25 °C.

An "emulsifier" refers to a compound that deters the separation of the injectable emulsion into individual oil and aqueous phases. Emulsifiers useful in the present disclosure generally are (1) compatible with the other ingredients of the stable emulsions of the present disclosure, (2) do not interfere with the stability or efficacy of the drugs contained in the emulsions, (3) are stable and do not deteriorate in the preparation, and (4) are non-toxic.

Suitable emulsifiers include, but are not limited to, propylene glycol mono- and di-fatty acid esters, polyoxyethylene sorbitan fatty acid esters, polyoxyethylene fatty acid esters, polyoxyethylene-polyoxypropylene co-polymers and block co-polymers, salts of fatty alcohol sulphates, sorbitan fatty acid esters, esters of polyethylene-glycol glycerol ethers, oil and wax based emulsifiers, glycerol monostearate, glycerine sorbitan fatty acid esters and phospholipids.

A "phospholipid" refers to a triester of glycerol in which the secondary alcohol and one of the primary alcohols has been esterified with fatty acids and the other primary alcohol has been esterified with a phosphate group. Exemplary phospholipids useful in the present invention include, but are not limited to, phosphatidyl chlorine, lecithin (a mixture of choline ester of phosphorylated diacylglyceride), phosphatidylethanolamine, phosphatidylglycerol, phosphatidic acid with about 4 to about 22 carbon atoms, and more generally from about 10 to about 18 carbon atoms and varying degrees of saturation. The phospholipids can have any combination of fatty acid as its fatty acyl side chain, for example, the phospholipids can have a saturated fatty acid such as a decanoic acid, lauric acid, myristic acid, palmitic acid, stearic acid, icosanoic acid, (a C20 saturated fatty acid); sodium behenic acid, or an unsaturated fatty acid such as myristoleic acid, palmitoleic acid, oleic acid, sodium linoleic acid, alpha linolenic acid, sodium arachidonic acid, eicosapentanoic acid, and the like. The two fatty acyl residues on the phospholipids may be the same or they may be different fatty acids. The phospholipid component of the drug delivery composition can be either a single phospholipid or a mixture of several phospholipids. The phospholipids should be acceptable for the chosen route of administration.

In one aspect, the phospholipids used as emulsifiers in the present invention are naturally occurring phospholipids from a natural origin. For example, naturally occurring lecithin is a mixture of the diglycerides of stearic, palmitic, and oleic acids, linked to the choline ester of phosphoric acid, commonly called phosphatidylcholine, and can be obtained from a variety of sources such as eggs and soya beans. Soy lecithin and egg lecithin (including hydrogenated versions of these compounds) have been characterized in various compositions and are generally recognized to be safe, have combined emulsification and solubilization properties, and tend to be broken down into innocuous substances more rapidly than most synthetic surfactants.

The term "lecithin" includes a complex mixture of acetone-insoluble phosphatides, of which phosphatidylcholine is a significant component. The term lecithin is also used as a synonym for phosphatidylcholine. Useful lecithins include, but are not limited to, egg yolk-, egg-, soybean-, and corn-derived lecithin. In some embodiments, the emulsifier is lecithin, such as egg yolk-derived lecithin. The terms egg lecithin and egg yolk derived lecithin are used interchangeably throughout. The compositions described herein preferably comprise lecithin as an emulsifier.

The amount of phospholipids, by weight, in the emulsions of the present disclosure may be within a range of about 10 wt/wt% to about 20 wt/wt%, 11 wt/wt% to 19 wt/wt%, 11 wt/wt% to 15 wt/wt%, 12 wt/wt% to 13 wt/wt%, 13 wt/wt% to 14 wt/wt%, 13 wt/wt% to 20 wt/wt%, or 12 wt/wt% to 18 wt/wt%. In certain embodiments, the phospholipids in the emulsions are at a concentration, by weight, about 11 wt/wt%, 12 wt/wt%, 12.5 wt/wt%, 13 wt/wt%, 13.5 wt/wt%, 14 wt/wt%, 14.5 wt/wt%, or 15 wt/wt%.

"Oil" refers to an organic liquid of mineral, vegetable, animal, essential or synthetic origin, including, for example, aliphatic or wax-based hydrocarbons, aromatic hydrocarbons or mixed aliphatic and aromatic hydrocarbons.

The term "buffer" or "buffered" as used herein means a solution containing both a weak acid and its conjugate base, whose pH changes only slightly upon addition of acid or base. As used herein, the phrase "buffering agent" means a species whose inclusion in a solution provides a buffered solution. Buffers are well-known in the art and readily available. Buffers for use according to the methods and compositions described herein include but are not limited to phosphate, citrate, Tris, carbonate, succinate, maleate, borate, MES, Bis-Tris, ADA, aces, PIPES, MOPSO, Bis-Tri Propane, BES, MOPS, TES, HEPES, DIPSO, MOBS, TAPSO, Trizma, HEPPSO, POPSO, TEA, EPPS, Tricine, Gly-Gly, Bicine, GEPBS, TAPS, AMPD, TABS, AMPSO, CHES, CAPSO, AMP, CAPS, and CABS.

The term "therapeutic agent" describes any natural or synthetic compound which has a biological activity. A "therapeutically effective amount" means the amount that, when administered to an animal or subject for treating or preventing a disorder, condition, or disease, is sufficient to effect treatment for that disorder, condition, or disease.

The term "substantially" in reference to a certain feature or entity means to a significant degree or nearly completely (i.e. to a degree of 85% or greater) in reference to the feature or entity.

Bioerodible", "bioerodibility" and "biodegradable", which are used interchangeably herein, refer to the degradation, disassembly or digestion of a polymer by action of a biological environment, including the action of living organisms and most notably at physiological pH and temperature. As an example, a principal mechanism for bioerosion of a polyorthoester is hydrolysis of linkages between and within the units of the polyorthoester.

A "polymer susceptible to hydrolysis" such as a polyorthoester refers to a polymer that is capable of degradation, disassembly or digestion through reaction with water molecules. Such a polymer contains hydrolyzable groups in the polymer. Examples of polymers susceptible to hydrolysis may include, but are not limited to, polymers described herein, and those described in U.S. Pat. Nos. 4,079,038, 4,093,709, 4,131,648, 4,138,344, 4,180,646, 4,304,767, 4,957,998, 4,946,931, 5,968,543, 6,613,335, and 8,252,304, and U.S. Patent Publication No. 2007/0265329.

"Molecular mass" in the context of a polymer such as a polyorthoester, refers to the nominal average molecular mass of a polymer, typically determined by size exclusion chromatography, light scattering techniques, or velocity. Molecular weight can be expressed as either a number-average molecular weight or a weight-average molecular weight. Unless otherwise indicated, all references to molecular weight herein refer to the weight-average molecular weight. Both molecular weight determinations, number-average and weight-average, can be measured using gel permeation chromatographic or other liquid chromatographic techniques. Other methods for measuring molecular weight values can also be used, such as the measurement of colligative properties (e.g., freezing-point depression, boiling-point elevation, or osmotic pressure) to determine number-average molecular weight or the use of light scattering techniques, ultracentrifugation or viscometry to determine weight-average molecular weight. The polymers of the invention are typically polydisperse (i.e., number-average molecular weight and weight-average molecular weight of the polymers are not equal), possessing low polydispersity values such as less than about 3.0, less than about 2.75, less than about 2.25, less than about 1.5, and less than about 1.03.

"Semi-solid" denotes the mechano-physical state of a material that is flowable under moderate stress. A semi-solid material will generally have a viscosity between about 1,000 and 3,000,000 cps (mPa•s) at 37 °C, especially between about 1,000 and 50,000 cps (mPa•s) at 37 °C. Viscosity may be measured using a Brookfield Viscometer DV-II Pro with a CPA-44PSYZ cup and measured at 37 °C. Viscosity measurements of formulations with less than 8,000 cP (mPa•s) are measured at using a CPA-40Z spindle and the system may be verified using 1,000cps silicone oil Brookfield Viscosity Standard. Viscosity measurements for formulations above 8,000 cP (mPa•s) may be evaluated using a CPA-52Z spindle and standardized using the 30,000cps silicone oil Brookfield Viscosity Standard.

An "active agent" or "active ingredient" refers to any compound or mixture of compounds which produces a beneficial or useful result. Active agents are distinguishable from such components as vehicles, carriers, diluents, lubricants, binders and other formulating aids, and encapsulating or otherwise protective components. Examples of active agents are pharmaceutical, agricultural or cosmetic agents.

A "small molecule" is a molecule, typically a drug, having a molecular weight of less than about 900 daltons.

A "local anesthetic" is a drug that causes reversible local anesthesia, generally for inducing absence of pain sensation.

A "caine" type drug as used herein refers to an amino type local anesthetic which are basic, such as bupivacaine, levobupivacaine, ropivacaine, etidocaine, lidocaine, mepivacaine, prilocaine, tetracaine and the like.

"Pharmaceutically acceptable salt" denotes a salt form of a drug having at least one group suitable for salt formation that causes no significant adverse toxicological effects to the patient. Pharmaceutically acceptable salts include salts prepared by reaction with an inorganic acid, an organic acid, a basic amino acid, or an acidic amino acid, depending upon the nature of the functional group(s) in the drug. Suitable pharmaceutically acceptable salts include acid addition salts which may, for example, be formed by mixing a solution of a basic drug with a solution of an acid capable of forming a pharmaceutically acceptable salt form of the basic drug, such as hydrochloric acid, iodic acid, fumaric acid, maleic acid, succinic acid, acetic acid, citric acid, tartaric acid, carbonic acid, phosphoric acid, sulfuric acid and the like. Typical anions for basic drugs, when in protonated form, include chloride, sulfate, bromide, mesylate, maleate, citrate and phosphate. Suitably pharmaceutically acceptable salt forms are found in, e.g., Handbook of Pharmaceutical Salts: Properties, Selection and Use, Weinheim/Zarich:Wiley-VCH/VHCA, 2002; P. H. Stahl and C. G. Wermuth, Eds.

An "organic acid" is an organic molecule having at least one carboxylic acid group that generally possesses a molecular weight that is less than about 300 daltons. An organic acid may have 2 or more carboxylic acid groups, e.g, 2, 3, or 4, carboxylic acid groups. The organic acid may be aliphatic or aromatic, and may optionally contain additional non-basic substituents such as hydroxyl, ester, halo, and the like. Aliphatic organic acids may also contain one or more elements of unsaturation, e.g., a double or a triple bond. Exemplary organic acids include ethanoic acid, propanoic acid, butanoic acid, pentanoic acid, benzoic acid, acetyl salicylic acid, citric acid, fumaric acid, maleic acid, salicylic acid, succinic acid, oxalic acid, malonic acid, glutaric acid, adipic acid, pimelic acid, and so forth.

Mole percent (also designated as mol%) of an organic acid as used herein is relative to the molar amount of the corresponding amino-containing drug. As an example, a composition comprising 100 mmols of an amino-containing drug such as ropivacaine and 10 mmols of an organic acid contains 10 mol percent of the organic acid.

"Polyorthoester-compatible" refers to, in one particular aspect of the properties of the polyorthoester, the properties of an excipient or additive which, when mixed with the polyorthoester, forms a single phase and does not cause any physical or chemical changes to the polyorthoester.

A "therapeutically effective amount" means the amount that, when administered to an animal for treatment of a disease, is sufficient to effect treatment for that disease.

"Treating" or "treatment" of a disease includes preventing the disease from occurring in an animal that may be predisposed to the disease but does not yet experience or exhibit symptoms of the disease (prophylactic treatment), inhibiting the disease (slowing or arresting its development), providing relief from the symptoms or side-effects of the disease (including palliative treatment), and relieving the disease (causing regression of the disease).

Optional" or "optionally" means that the subsequently described circumstance may or may not occur, so that the description includes instances where the circumstance occurs and instances where it does not.

The term "substantially" in reference to a certain feature or entity means to a significant degree or nearly completely (i.e. to a degree of 85% or greater) in reference to the feature or entity.

The term "about", particularly in reference to a given quantity, is meant to encompass deviations of plus or minus five percent.

### II. PHARMACEUTICAL COMPOSITION COMPRISING PAIN MEDICATION AND NK-1 RECEPTOR ANTAGONSIT

In one aspect, compositions comprising a local anesthetic, an NK-1 receptor antagonist, and a delivery vehicle are provided. In another aspect, compositions comprising a local anesthetic, an NK-1 receptor antagonist, a NSAID, and a delivery vehicle are provided. In another aspect, compositions comprising an NK-1 receptor antagonist, a NSAID, and a delivery vehicle are provided.

### A. Local Anesthetic

The composition comprises a local anesthetic of the caine type. Local anesthetics belonging to this class include bupivacaine, levobupivacaine, dibucaine, mepivacaine, procaine, lidocaine, tetracaine, ropivacaine, and the like. These compounds are alkaline-amides possessing pKb values ranging from 5.8 to 6.4. That is, the drugs contain protonizable tertiary amine functions. For example, the pKa values of ropivacaine, lidocaine, and bupivacaine are 8.1, 7.7 and 8.1, respectively. The amide-type drugs are provided in the compositions either in their neutral, base form or as their corresponding acid-addition salts, or as a mixture of both forms.

In one embodiment, the caine type local anesthetic is added to the composition in its free base form. The caine type anesthetic may be provided as a racemic mixture, i.e., containing equal amounts of the R and S enantiomers, or may be provided as a single enantiomer, or may be provided as an unequal mixture of enantiomers in which one enantiomer is in excess.

In one particular embodiment, the composition comprises bupivacaine as the local anesthetic. In a further embodiment, the composition comprises as the active agent ropivacaine.

In yet one or more additional embodiments, the composition comprises any one or more of the caine type local anesthetics described above such as, for example, levobupivacaine, dibucaine, mepivacaine, procaine, lidocaine, tetracaine, and the like. In still another embodiment, the amide-type local anesthetic is selected from the group consisting of bupivacaine, ropivacaine, levobupivacaine, dibucaine, mepivacaine, procaine, lidocaine, and tetracaine.

The composition may also comprise in addition to the caine type local anesthetic and the NK-1 receptor antagonist (described below), one or more additional active agents.

The caine type local anesthetic is dissolved or dispersed into the composition as provided herein. The concentration of the caine local anesthetic in the composition may vary from about 0.1 wt% to 1 wt%, 0.1 wt% to 10 wt%, 0.25 wt% to 0.75 wt%, 0.25 wt% to 2.5 wt%, 0.25 wt% to 3.5 wt%, 0.25 wt% to 5 wt%, 1.5 wt% to 5 wt%, 1.5 wt% to 3.5 wt%, 1.5 wt% to 3.0 wt%, 2 wt% to 3 wt%, 2.1 wt% to 2.9 wt%, 2.2 wt% to 2.8 wt%), 2.3 wt% to 2.7 wt%, 2.4 wt% to 2.6 wt%, 2.25 wt% to 2.75 wt%, 1 wt% to 30 wt%, 1 wt % to 10 wt %, 10 wt% to 20 wt%, 1 wt% to 5 wt%, 2 wt% to 5 wt%, 2 wt% to 4 wt%, 2 wt° o to 3 wt%, 10 wt% to 15 wt%, or 15 wt% to 20 wt% and may be about 0.25 wt%, 0.5 wt%, 0.751 wt%, 1.1 wt%, 1.2 wt%, 1.3 wt%, 1.4 wt%, 1.5 wt%, 1.6 wt%, 1.7 wt%, 1.8 wt%, 1.9 wt%, 2 wt%, 2.1 wt%, 2.2 wt%, 2.3 wt%, 2.4 wt%, 2.5 wt%, 2.6 wt%, 2.7 wt%, 2.8 wt%, 2.9 wt%, 3 wt%, 3.1 wt%, 3.2 wt%, 3.3 wt%, 3.4 wt%, 3.5 wt%, 3.6 wt%, 3.7 wt%, 3.8 wt%, 3.9 wt%, 4 wt%, 4.1 wt%, 4.2 wt%, 4.3 wt%, 4.4 wt%, 4.5 wt%, 4.6 wt%, 4.7 wt%, 4.8 wt%, 4.9 wt%, 5 wt%, 5 wt%, 5.1 wt%, 5.2 wt%, 5.3 wt%, 5.4 wt%, 5.5 wt%, 5.6 wt%, 5.7 wt%, 5.8 wt%, 5.9 wt%, 6 wt%, 6.1 wt%, 6.2 wt%, 6.3 wt%, 6.4 wt%, 6.5 wt%, 6.6 wt%, 6.7 wt%, 6.8 wt%, 6.9 wt%, 7 wt%, 7.1 wt%, 7.2 wt%, 7.3 wt%, 7.4 wt%, 7.5 wt%, 7.6 wt%, 7.7 wt%, 7.8 wt%, 7.9 wt%, 8 wt%, 8.1 wt%, 8.2 wt%, 8.3 wt%, 8.4 wt%, 8.5 wt%, 8.6 wt%, 8.7 wt%, 8.8 wt%, 8.9 wt%, 9 wt%. 9.1 wt%, 9.2 wt%, 9.3 wt%, 9.4 wt%, 9.5 wt%, 9.6 wt%, 9.7 wt%, 9.8 wt%, 9.9 wt%, 10 wt%, 11 wt%, 11.1 wt%, 11.2 wt%, 11.3 wt%, 11.4 wt%, 11.5 wt%, 11.6 wt%, 11.7 wt%, 11.8 wt%, 11.9 wt%, 12 wt%, 12.1 wt%, 12.2 wt%, 12.3 wt%, 12.4 wt%, 12.5 wt%, 12.6 wt%, 12.7 wt%, 12.8 wt%, 12.9 wt%, 13 wt%, 13.1 wt%, 13.2 wt%, 13.3 wt%, 13.4 wt%, 13.5 wt%, 13.6 wt%, 13.7 wt%, 13.8 wt%, 13.9 wt%, 14 wt%, 14.1 wt%, 14.2 wt%, 14.3 wt%, 14.4 wt%, 14.5 wt%, 14.6 wt%, 14.7 wt%, 14.8 wt%, 14.9wt%, 15 wt%, 15 wt%, 15.1 wt%, 15.2 wt%, 15.3 wt%, 15.4 wt%, 5.5 wt%, 15.6 wt%, 15.7 wt%, 15.8 wt%, 15.9 wt%, 16 wt%, 16.1 wt%, 16.2 wt%, 16.3 wt%, 16.4 wt%, 16.5 wt%, 16.6 wt%, 16.7 wt%, 16.8 wt%, 16.9 wt%, 17 wt%, 17.1 wt%, 17.2 wt%, 17.3 wt%, 17.4. wt%, 17.5 wt%, 17.6 wt%, 17.7 wt%, 17.8 wt%, 17.9 wt%, 18 wt%, 18.1 wt%, 18.2 wt%, 18.3 wt%, 18.4 wt%, 18.5 wt%, 18.6 wt%, 18.7 wt%, 18.8 wt%, 18.9 wt%, 19 wt%, 19.1 wt%, 19.2 wt%, 19.3 wt%, 19.4 wt%, 19.5 wt%, 19.6 wt%, 19.7 wt%, 19.8 wt%, 19.9 wt%, 20 wt%, 21 wt%, 22 wt%, 23 wt%, 24 wt%, 25 wt%, 26 wt %, 27 wt%, 28 wt%, 29 wt% and 30 wt%.

In one embodiment, the caine type local anesthetic is present in the composition at between about 0.01 wt % and about 7.5 wt%. In another embodiment, the caine type local anesthetic is present in the composition at between about 0.1 wt % and about 7.5 wt%, or between about 0.1 wt % and about 5.5 wt%, or between about 0.25 wt % and about 5.2 wt%, or between about 0.25 wt % and about 5.0 wt%.

### B. NK-1 Receptor Antagonist and NSAID

Suitable NK-1 receptor antagonists for use in the presently described pharmaceutical compositions include RP 67580 ((3aR.7aR)-Octahydro-2-[1-imino-2-(2-methoxyphenyl)ethyl]-7,7-diphenyl-4H-isoindol)), WIN 51078 (17-β-Hydroxy-17-α-ethynyl-5-α-androstano[3,2- b]pyrimido[1.2-a]benzimidazole), 1-733,060, (((2S,3S)-3-[[3,5-bis(Trifluoromethyl) phenyl]methoxy]-2-phenylpiperidine hydrochloride), 1-703,606 (cis-2-(Diphenylmethel)-N- ([2-iodophenyl]methyl)-1-azabicyclo(2.2.2)octan-3-amine) MDL 105,212 (R)-1-[2-[3-(3,4- dichlorophenyl)-1-(3,4,5-trimethoxybenzoyl)-pyrrolidin-3-yl]-ethyl]-4-phenylpiperidine-4-carboxamide hydrochloride), serlopitant, maropitant, Antagonist D, aprepitant, fosaprepitant, R116301, CGP49823, CP-96345, CP-99994, GR-203040, MDL-103392, 1-760735, SDZ-NKT-343, nolpitanitium (SR-140333), AV608, rolapitant, SCH 900978, AV608, GSK424887 (GlaxoSmithKline), GSK206136 (GlaxoSmithKline), GR-205171, CP-99994, TAK 637 ((S)-7-(3,5-Bis-trifluoromethyl-benzyl)-9-methyl-5-p-tolyl-8,9,10,11-tetrahydro-7H-1,7,11a-triaza-cycloocta[b]naphthalene-6,12-dione), LY303870 ([(R)-1-[N-(2-methoxybenzyl)acetylamino]-3-(1H-indol-3-yl)-2-[N-(2-(4- (piperidin-1-yl)piperidin-1-yl)acetyl)amino]propane]), LY686017 ((2-chloro-phenyl)-{2-[5-pyridin-4-yl-1-(3,5-bistrifluoromethyl-benzyl)-1H-[1,2,3]triazol-4-yl]-pyridin-3-yl}-methanone), E-6006, casopitant/GW679769 ((2R,4S)-4-(4-acetylpiperazin-1-yl)-N-[(1R)-1-[3,5-bis(trifluoromethyl)phenyl)ethyl]-2-(4-fluoro-2-methylphenyl)-N-methylpiperidine-1-carboxamide), vestipitant, orvepitant and orvepitant maleate, befetupitant, netupitant, ezlopitant, CP-122721, MPC-4505 (Myriad Genetics, Inc.), CP-122721 (Pfizer, Inc.), CJ-1 2,255 (Pfizer, Inc.), SRR 240600 (Sanofi-Aventis), or TA-5538 (Tanabe Seiyaku Co.) including all pharmaceutically acceptable salts thereof.

In some embodiments, the NK-1 receptor antagonist is selected from aprepitant, rolapitant, netupitant, lanepitant, vestipitant, orvepitant maleate, casopitant, ezlopitant, serlopitant, befetupitant and maropitant, or a pharmaceutically acceptable salt thereof. In one embodiment, the NK-1 receptor antagonist is aprepitant.

The NK-1 receptor antagonist is dissolved or dispersed into the composition as provided herein. The concentration of the NK-1 receptor antagonist in the composition may vary from about 0.01 wt% to 1 wt%, 0.01 wt% to 5 wt%, 0.01 wt% to 10 wt%, 0.1 wt% to 1 wt%, 0.1 wt% to 2.5 wt%, 0.1 wt% to 5 wt%, 0.2 wt% to 1 wt%, 0.2 wt% to 2 wt%, 0.2 wt% to 3.5 wt%, 0.2 wt% to 5 wt%, 2.1 wt% to 2.9 wt%, 2.2 wt% to 2.8 wt%, 2.3 wt% to 2.7 wt%, 2.4 wt% to 2.6 wt%, 2.25 wt% to 2.75 wt%, 1 wt% to 30 wt%, 1 wt % to 10 wt %, 10 wt% to 20 wt%, 1 wt% to 5 wt%, 2 wt% to 5 wt%, 2 wt% to 4 wt%, 2 wt% to 3 wt%, 10 wt% to 15 wt%, or 15 vvt%) to 20 wt% and may be about 0.0 1wt%, 0.02 wt%, 0.03 wt%, 0.04 wt%, 0.05 wt%, 0.06 wt%, 0.07 wt%, 0.08 wt%, 0.09 wt%, 0.1 wt%, 0.1 wt%0.2 wt%, 0.3 wt%, 0.4 wt%, 0.5 wt%, 0.6 wt%, 0.7 wt%, 0.8 wt%, 0.9 wt%, 1 wt%, 1.1 wt%, 1.2 wt%, 1.3 wt%, 1.4 wt%, 1.5 wt%, 1.6 wt%, 1.7 wt%, 1.8 wt%, 1.9 wt%, 2 wt%, 2.1 wt%, 2.2 wt%, 2.3 wt%, 2.4 wt%, 2.5 wt%, 2.6 wt%, 2.7 wt%, 2.8 wt%, 2.9 wt%, 3 wt%, 3.1 wt%, 3.2 wt%, 3.3 wt%, 3.4 wt%, 3.5 wt%, 3.6 wt%, 3.7 wt%, 3.8 wt%, 3.9 wt%, 4 wt%, 4.1 wt%, 4.2 wt%, 4.3 wt%, 4.4 wt%, 4.5 wt%, 4.6 wt%, 4.7 wt%, 4.8 wt%, 4.9 wt%, 5 wt%, 5.1 wt%, 5.2. wt%, 5.3 wt%, 5.4 wt%, 5.5 wt%, 5.6 wt%, 5.7 wt%, 5.8 wt%, 5.9 wt%, 6 wt%, 6.1 wt%, 6.2 wt%, 6.3 wt%, 6.4 wt%, 6.5 wt%, 6.6 wt%, 6.7 wt%, 6.8 wt%, 6.9 wt%, 7 wt%, 7.1 wt%, 7.2 wt%, 7.3 wt%, 7.4 wt%, 7.5 wt%, 7.6 wt%, 7.7 wt%, 7.8 wt%, 7.9 wt%, 8 wt%, 8.1 wt%, 8.2 wt%, 8.3 wt%, 8.4 wt%, 8.5 wt%, 8.6 wt%, 8.7 wt%, 8.8 wt%, 8.9 wt%, 9 wt%, 9.1 wt%, 9.2 wt%, 9.3 wt%, 9.4 wt%, 9.5 wt%, 9.6 wt%, 9.7 wt%, 9.8 wt%, 9.9 wt%, 10 wt%.

In one embodiment, the NK-1 receptor antagonist is present in the composition in an amount which is above 0.001 percent and less than 5 wt%, less than 2.5 wt%, less than 1 wt%, less than 0.5 wt% percent, less than or less than 0.3wt%.

In one embodiment, the composition comprises a caine type local anesthetic and an NK-1 receptor antagonist the weight ratio of local anesthetic: NK-1 receptor antagonist ranges from about 100:1 to 1:1, 100:1 to 2.5:1, 100:1 to 7.5:1, 100:1 to 10:1, 100:1 to 1:100, 75:1 to 1:1, 75:1 to 2.5:1, 75:1 to 7.5:1, 75:1 to 10:1, 75:1 to 1:75, 50:1 to 1:1, 50:1 to 2.5:1, 50:1 to 7.5:1, 50:1 to 10:1, 50:1 to 1:50, 25:1 to 1:1, 25:1 to 2.5:1, 25:1 to 7.5:1, 25:1 to 10:1, 25:1 to 1:25, 10:1 to 1:1, 10:1 to 2.5:1, 10:1 to 7.5:1, 10:1 to 10:1, 10:1 to 1:10, In a particular embodiment, the caine type local anesthetic is bupivacaine and the NK -1 receptor antagonist is aprepitant. In an alternative particular embodiment, the amide-type local anesthetic is ropivacaine and the NK-1 receptor antagonist is aprepitant.

NSAID is selected from the group consisting of diflunisal, indomethacin, tolmetin, sulindac, etodolac, ketorolac, diclofenac, nabumetone, ibuprofen, dexibuprofen, naproxen, fenoprofen, ketoprofen, dexketoprofen, flurbiprofen, oxaprozin, loxoprofen, mefenamic acid, meclofenamic acid, flufenamic acid, tolfenamic acid, meloxicam, piroxicam, tenoxicam, droxicam, lomoxicam, isoxicam, celecoxib, rofecoxib, and valdecoxib. In some embodiments, the NSAID is meloxicam.

The concentration of the NSAID in the composition may vary from about 0.01 wt% to 10 wt%, 0.01 wt % to 5wt %, 0.01 wt% to 3 wt%, 0.01 wt% to 1 wt%, 0.10 wt% to 10 wt%, 0.1wt % to 5wt %, 0.1 wt% to 3 wt%, 0.1 wt% to 1 wt%, and may be 0.01 wt%, 0.011 wt%, 0.012 wt%, 0.013 wt%, 0.014 wt%, 0.015 wt%, 0.016 wt%, 0.017 wt%, 0.018 wt%, 0.019 wt%, 0.02 wt%, 0.021 wt%,0.022 wt%, 0.023 wt%, 0.024 wt%, 0.025 wt%, 0.026 wt%, 0.027 wt%, 0.028 wt%, 0.029 wt%, 0.030 wt%, 0.031 wt%, 0.032 wt%, 0.033 wt%, 0.034 wt%, 0.035 wt%, 0.036 wt%, 0.037 wt%, 0.038 wt%, 0.039 wt%, 0.040 wt%, 0.041 wt%, 0.042 wt%, 0.043 wt%, 0.044 wt%, 0.045 wt%, 0.046 wt%, 0.047 wt%, 0.048 wt%, 0.049 wt%, 0.05 wt%, 0.051 wt%, 0.052 wt%, 0.033 wt%, 0.054 wt%, 0.055 wt%, 0.056 wt%, 0.057 wt%, 0.058 wt%, 0.059 wt%, 0.06 wt%, 0.061 wt%, 0.062 wt%, 0.063 wt%, 0.064 wt%, 0.065 wt%, 0.066 wt%, 0.067 wt%, 0.068 wt%, 0.069 wt%, 0.07 wt%, 0.071 wt%, 0.077 wt%, 0.073 wt%, 0.074 wt%, 0.075 wt%, 0.076 wt%, 0.077 wt%, 0.078 wt%, 0.079 wt%, 0.08 wt%, 0.081 wt%, 0.082 wt%, 0.083 wt%, 0.084 wt%, 0.085 wt%, 0.086 wt%, 0.087 wt%, 0.088 wt%, 0.089 wt%, 0.09 wt%, 0.091 wt%, 0.092 wt%, 0.093 wt%, 0.094 wt%, 0.095 wt%, 0.096 wt%, 0.097 wt%, 0.098 wt%, 0.099 wt%, 0.1 wt%, 0.11 wt%, 0.12 wt%, 0.13 wt%, 0.14 wt%, 0.15 wt%, 0.16 wt%, 0.17 wt%, 0.18 wt%, 0.19 wt%, 0.2 wt%, 0.21 wt%,0.22 wt%, 0.23 wt%, 0.24 wt%, 0.25 wt%, 0.26 wt%, 0.27 wt%, 0.28 wt%, 0.29 wt%, 0.30 wt%, 0.31 wt%, 0.32 wt%, 0.33 wt%, 0.34 wt%, 0.35 wt%, 0.36 wt%, 0.37 wt%, 0.38 wt%, 0.39wt%, 0.40 wt%, 0.41 wt%, 0.42 wt%, 0.43 wt%, 0.44 wt%, 0.45 wt%, 0.46 wt%, 0.47 wt%, 0.48 wt%, 0.49wt%, 0.5 wt%, 0.5.1 wt%, 0.52 wt%, 0.53 wt%, 0.54 wt%, 0.55 wt%, 0.56 wt%, 0.57 wt%, 0.58 wt%, 0.59 wt%, 0.6 wt%, 0.61 wt%, 0.62 wt%, 0.63 wt%, 0.64 wt%, 0.65 wt%, 0.66 wt%, 0.67 wt%, 0.68 wt%, 0.69wt%, 0.7 wt%, 0.71 wt%, 0.72 wt%, 0.73 wt%, 0.74 wt%, 0.75 wt%, 0.76 wt%, 0.77 wt%, 0.78 wt%, 0.79 wt%, 0.8 wt%, 0.81 wt%, 0.82 wt%, 0.83 wt%, 0.84 wt%, 0.85 wt%, 0.86 wt%, 0.87 wt%, 0.88 wt%, 0.89 wt%, 0.9 wt%, 0.91 wt%, 0.92 wt%, 0.93 wt%, 0.94 wt%, 0.95 wt%, 0.96 wt%, 0.97 wt%, 0.98 wt%, 0.99 wt%, 1.0 wt%, 1.01 wt%, 1.02 wt%, 1.03 wt%, 1.04 wt%, 1.05 wt%, 1.06 wt%, 1.07 wt%, 1.08 wt%, 1.09wt%, 1.1 wt%, 1.11 wt%, 1.12 wt%, 1.13 wt%, 1.14 wt%, 1.15 wt%, 1.16 wt%, 1.17 wt%, 1.18 wt%, 1.19 wt%, 1.2 wt%, 1.21 wt%, 1.22 wt%, 1.23 wt%, 1.24 wt%, 1.25 wt%, 1.26 wt%, 1.27 wt%, 1.28 wt%, 1.29 wt%, 1.30 wt%, 1.31 wt%, 1.32 wt%, 1.33 wt%, 1.34 wt%, 1.35 wt%, 1.36 wt%, 1.37 wt%, 1.38 wt%, 1.39 wt%, 1.40 wt%, 1.41 wt%, 1.42 wt%, 1.43 wt%, 1.44 wt%, 1.45 wt%, 1.46 wt%, 1.47 wt%, 1.48 wt%, 1.49wt%, 1.5 wt%, 1.5.1 wt%, 1.52 wt%, 1.53 wt%, 1.54 wt%, 1.55 wt%, 1.56 wt%, 1.57 wt%, 1.58 wt%, 1.59 wt%, 1.6 wt%, 1.61 wt%, 1.62 wt%, 1.63 wt%, 1.64 wt%, 1.65 wt%, 1.66 wt%, 1.67 wt%, 1.68 wt%, 1.69 wt%, 1.7 wt%, 1.71 wt%, 1.72 wt%, 1.73 wt%, 1.74 wt%, 1.75 wt%, 1.76 wt%, 1.77 wt%, 1.78 wt%, 1.79wt%, 1.8 wt%, 1.81 wt%, 1.82 wt%, 1.83 wt%, 1.84 wt%, 1.85 wt%, 1.86 wt%, 1.87 wt%, 1.88 wt%, 1.89wt%, 1.9 wt%, 1.91 wt%, 1.92 wt%, 1.93 wt%, 1.94 wt%, 1.95 wt%, 1.96 wt%, 1.97 wt%, 1.98 wt%, 1.99 wt%, 2.00 wt%, 2.01 wt%, 2.02 wt%, 2.03 wt%, 2.04 wt%, 2.05 wt%, 2.06 wt%, 2.07 wt%, 2.08 wt%, 2.09 wt%, 2.1 wt%, 2.11 wt%, 2.12 wt%, 2.13 wt%, 2.14 wt%, 2.15 wt%, 2.16 wt%, 2.17 wt%, 2.18 wt%, 2.19 wt%, 2.20 wt%, 2.21 wt%,2.22 wt%, 2.23 wt%, 2.24 wt%, 2.25 wt%, 2.26 wt%, 2.27 wt%, 2.28 wt%, 2.29 wt%, 2.30 wt%, 2.31 wt%, 2.32 wt%, 2.33 wt%, 2.34 wt%, 2.35 wt%, 2.36 wt%, 2.37 wt%, 2.38 wt%, 2.39 wt%, 2.40 wt%, 2.41 wt%, 2.42 wt%, 2.43 wt%, 2.44 wt%, 2.45 wt%, 2.46 wt%, 2.47 wt%, 2.48 wt%, 2.49 wt%, 2.5 wt%, 2.5.1 wt%, 2.52 wt%, 2.53 wt%, 2.54 wt%, 2.55 wt%, 2.56 wt%, 2.57 wt%, 2.58 wt%, 2.59 wt%, 2.6 wt%, 2.61 wt%, 2.62 wt%, 2.63 wt%, 2.64 wt%, 2.65 wt%, 2.66 wt%, 2.67 wt%, 2.68 wt%, 2.69wt%, 2.7 wt%, 2.71 wt%, 2.72 wt%, 2.73 wt%, 2.74 wt%, 2.75 wt%, 2.76 wt%, 2.77 wt%, 2.78 wt%, 2.79wt%, 2.8 wt%, 2.81 wt%, 2.82 wt%, 2.83 wt%, 2.84 wt%, 2.85 wt%, 2.86 wt%, 2.87 wt%, 2.88 wt%, 2.89 wt%, 2.9 wt%, 2..91 wt%, 2.92 wt%, 2.93 wt%, 2.94 wt%, 2.95 wt%, 2.96 wt%, 2.97 wt%, 2.98 wt%, 2.99wt%, 3.0 wt%, 3.01 wt%, 3.02 wt%, 3.03 wt%, 3.04 wt%, 1.05 wt%, 3.06 wt%, 3.07 wt%, 3.08 wt%, 3.09 wt%, 3.1 wt%, 3.11 wt%, 3.12 wt%, 3.13 wt%, 3.14 wt%, 3.15 wt%, 3.16 wt%, 3.17 wt%, 3.18 wt%, 3.19 wt%, 3.20 wt%, 3.21 wt%,3.22 wt%, 3.23 wt%, 3.24 wt%, 3.25 wt%, 3.26 wt%, 3.27 wt%, 3.28 wt%, 3.29 wt%, 3.30 wt%, 3.31 wt%, 3.32 wt%, 3.33 wt%, 3.34 wt%, 3.35 wt%, 3.36 wt%, 3.17 wt%, 3.38 wt%, 3.39 wt%, 3.40 wt%, 3.41 wt%, 3.42 wt%, 3.43 wt%, 3.44 wt%, 3.45 wt%, 3.46 wt%, 3.47 wt%, 3.48 wt%, 3.49 wt%, 3.5 wt%, 3.5.1 wt%, 3.52 wt%, 3.53 wt%, 3.54 wt%, 3.55 wt%, 3.56 wt%, 3.57 wt%, 3.58 wt%, 3.59wt%, 3.6 wt%, 3.61 wt%, 3.62 wt%, 3.63 wt%, 3.64 wt%, 3.65 wt%, 3.66 wt%, 3.67 wt%, 3.68 wt%, 3.69wt%, 3.7 wt%, 3.71 wt%, 3.72 wt%, 3.73 wt%, 3.74 wt%, 3.75 wt%, 3.76 wt%, 3.77 wt%, 3.78 wt%, 3.79wt%, 3.8 wt%, 3.81 wt%, 3.82 wt%, 3.83 wt%, 3.84 wt%, 3.85 wt%, 3.86 wt%, 3.87 wt%, 3.88 wt%, 3.89wt%, 3.9 wt%, 3.91 wt%, 3.92 wt%, 3.93 wt%, 3.94 wt%, 3.95 wt%, 3.96 wt%, 3.97 wt%, 3.98 wt%, 3.99wt%, 4.0 wt%, 4.25 wt%, 4.5 wt%, 4.75 wt%, 5.0 wt%, 5.25 wt%, 5.5 wt%, 5.75 wt%, 6.0 wt%, 6.25 wt%, 6.5 wt%, 6.75 wt%, 7.0 wt%, 7.25 wt%, 7.5 wt%, 7.75 wt%, 8.0 wt%, 8.25 wt%, 8.5 wt%, 8.75 wt%, 9.0 wt%, 9.25 wt%, 9.5 wt%, 9.75 wt%, or 10.0 wt%.

### C. Delivery Vehicles

The composition additionally comprises a delivery vehicle. In one embodiment, the delivery vehicle is a sustained-release vehicle, and exemplary vehicles include polymeric formulations, liposomes, microspheres, implantable device or non-polymeric formulations. Examples of these vehicles will now be described.

### 1. Liposomes

Liposomes are small vesicles composed of lipids arranged in spherical bilayers. Liposomes are usually classified as small unilamellar vesicles (SUV), large unilamellar vesicles (LUV), multilamellar vesicles (MLV) or multivesicular liposomes (MVL). SUVs and LUVs, by definition, have only one bilayer, whereas MLVs contain many concentric bilayers (see, e.g., Stryer, Biochemistry, 2d Edition, W.H. Freeman & Co., p. 213 (1981)). MVLs were first reported by Kim et al. (Biochim, Biophys. Acta, 728:339-348, 1983) and contain multiple, non-concentric aqueous chambers per particle (*See,* USPNs 6,132,766 and 8,182,835).

Liposomes suitable for use in the composition of the present invention include those composed primarily of vesicle-forming lipids. Vesicle-forming lipids can form spontaneously into bilayer vesicles in water, as exemplified by the phospholipids. The liposomes can also include other lipids incorporated into the lipid bilayers, e.g., cholesterol, with the hydrophobic moiety in contact with the interior, hydrophobic region of the bilayer membrane, and the head group moiety oriented toward the exterior, polar surface of the bilayer membrane.

The vesicle-forming lipids can have two hydrocarbon chains, typically acyl chains, and a head group, either polar or nonpolar. There are a variety of synthetic vesicle-forming lipids and naturally-occurring vesicle-forming lipids, including the phospholipids, such as phosphatidylcholine, phosphatidylethanolamine, phosphatidic acid, phosphatidylinositol, and sphingomyelin, where the two hydrocarbon chains are typically between about 14-22 carbon atoms in length, and have varying degrees of unsaturation. The above-described lipids and phospholipids whose acyl chains have varying degrees of saturation can be obtained commercially or prepared according to published methods. Other suitable lipids include glycolipids and sterols, such as cholesterol.

In one embodiment, the vesicle-forming lipid is selected to achieve a specified degree of fluidity or rigidity, to control the stability of the liposome in serum and to control the rate of release of the entrapped agent in the liposome. Liposomes may be prepared by a variety of techniques (see, e.g., Szoka, F., Jr., et al., Ann. Rev. Biophys. Bioeng. 9:467 (1980); U.S. Pat. No. 5,631,018). It will be appreciated that lipid-based delivery vehicles other than liposomes are contemplated, such as micelles and emulsions.

In one embodiment, the amide-type local anesthetic and the enolic-acid NSAID are entrapped in an aqueous space of the liposome or in a lipid layer of the liposome.

### 2. Microspheres/Microparticles/Microcapsules

In another embodiment, the delivery vehicle is a microspheres, microparticles or microcapsules. Microspheres in the form of spherical polymer matrices with interconnected pores in which an active agent is incorporated are described, for example, in USPN 4,818,542. Microparticles comprised of one or more polymers in which the active agents are incorporated or associated can be fabricated from biodegradable or non-biodegradable polymers that are suitable for in vivo use, such as poly(vinylpyrrolidone) and poly(acrylamide). The microspheres or microparticles can be administered as part of a formulation that forms a depot in situ or as part of an implant. The active agents are released from the microspheres or microparticles in a controlled fashion, to provide the desired therapeutic efficacy. In one embodiment, the sustained-release delivery vehicle is a microsphere comprised of a bioerodible or biodegradable polymer. In another embodiment, the amide-type local anesthetic and the enolic-acid NSAID are entrapped in the microsphere.

### 3. Implantable Devices

Implantable devices with a reservoir in which the active agents are contained and controllably-released are known in the medical arts. In one embodiment, an osmotic, mechanical, or electromechanical device is provided for implantation and sustained release of the active agents. Examples of implantable devices are set forth in USPNs 7,655,254; 8,603,051; and 8,603,076 and US Publication No. 2003/0032947.

### 4. Non-Polymeric Formulations

The delivery vehicle can also take the form of a non-polymeric, pharmaceutically acceptable carrier. For example, the non-polymeric formulation can comprise sucrose acetate isobutyrate as a non-polymeric, pharmaceutically acceptable carrier and an optional solvent, such as benzyl alcohol. The non-polymeric formulation can be a liquid. This liquid, non-polymeric formulation provides sustained local anesthesia to a subject after administration for a period of about 24-36 hours, 36-48 hours, 48-60 hours, 60-72 hours, 3-4 days or 3-5 days. In one embodiment, the delivery vehicle is comprised of between about 50-80 wt% sucrose acetate isobutyrate and between about 5-25 wt% benzyl alcohol, alternatively between 55-75 wt% sucrose acetate isobutyrate and between about 15-25 wt% benzyl alcohol, with the remainder to 100 wt% being the active agents. Exemplary non-polymeric formulations of this type are described in EP 1809329.

In some embodiments, the liquid non-polymeric carrier is a liquid carrier material having a viscosity of about less than 50,000 mPa-s at 37°C, measured using a viscometer. Alternatively, the carrier has a viscosity of less than about 10,000 mPa-s when measured at 37°C using a viscometer. In another embodiment, the liquid non-polymeric carrier is a liquid carrier material having a viscosity of about less than 5,000 mPa-s at 37°C. In yet another embodiment, the liquid non-polymeric carrier is a liquid carrier material having a viscosity of about less than 2,500 mPa-s at 37°C.

In another embodiment, the non-polymeric formulation is an aqueous solution.

### 5. Polymeric Formulations

Exemplary polymeric formulations as the sustained-release delivery vehicle include those comprised of bioerodible or biodegradable polymers. The vehicle when comprised of a bioerodible or biodegradable polymer can be a solid or a semi-solid vehicle. Bioerodible and/or biodegradable polymers are known in the art, and include but are not limited to polylactides, polyglycolides, poly(lactic-co-glycolic acid) copolymers, polycaprolactones, poly-3-hydroxybutyrate, and polyorthoesters. Semisolid polymers exist either in a glassy or viscous liquid state. Semisolid polymers typically display a glass transition temperature (Tg) below room temperature. Below the Tg, semisolid polymers can be considered to exist in a glassy state, while above the Tg, the polyorthoester can be considered to exist in a liquid state. Semisolid polyorthoester polymers are not thermoplastic polymers.

In one embodiment, a bioerodible or biodegradable polymer is selected to provide a certain rate of degradation or erosion to achieve a desired release rate of the enolic acid-type NSAID and the amide- or anilide type anesthetic. The delivery vehicle and active agents can be formulated to provide a semi-solid or solid composition. By way of example, in one embodiment, a semi-solid delivery vehicle comprised of a polyorthoester is provided, and some examples are set forth herein. In another embodiment, the polymeric delivery vehicle forms an implant or depot *in situ.*

In another embodiment, a solid delivery vehicle comprised of a biodegradable or bioerodible polymer is provided, where the solid vehicle is in the form of a rod or disk. Rods and disks are suitable for implantation into a patient, and the biodegradable or bioerodible polymer in which the active agents are incorporated can formulated to tailor the release of active agent. For example, the rod or disk can be formulated from different polymers with different rates of biodegradability or polymers of differing molecular weights can be used, as well as additives or excipients can be added to active agent-polymer matrix to tailor the rate of agent release. The rod or disk can also comprise materials commonly used in sutures and/or capable of being used in sutures, including the biodegradable polymers noted above as well as polyglactin and copolymers of glycolide with trimethylene carbonate (TMC) (polyglyconate).

In one embodiment, the delivery vehicle is comprised of a polyorthoester.

Polyorthoesters useful for the compositions provided herein are generally composed of alternating residues resulting from reaction of a diketene acetal and a diol, where each adjacent pair of diketene acetal derived residues is separated by the residue of a reacted diol. The polyorthoester may comprise α-hydroxy acid-containing subunits, i.e., subunits derived from an α-hydroxy acid or a cyclic diester thereof, such as subunits comprising glycolide, lactide, or combinations thereof, i.e., poly(lactide-co-glycolide), including all ratios of lactide to glycolide, e.g., 75:25, 65:35, 50:50, etc. Such subunits are also referred to as latent acid subunits; these latent acid subunits also fall within the more general "diol" classification as used herein, due to their terminal hydroxyl groups. Polyorthoesters can be prepared as described, for example, in U.S. Patent Nos. 4,549,010 and 5,968,543. Exemplary polyorthoesters suitable for use in the compositions provided herein are described in U.S. Patent No. 8,252,304.

The mole percentage of α-hydroxy acid containing subunits, R¹, generally ranges from 0 to 20 mol% of the total diol components (R¹ and R³ as provided below). In one or more embodiments, the mole percentage of α-hydroxy acid containing subunits in the polyorthoester formulation is at least about 0.01 mole percent. Exemplary percentages of α-hydroxy acid containing subunits in the polymer are from about 0 to about 50 mole percent, or from about 0 to about 25 mole percent, or from about 0.05 to about 30 mole percent, or from about 0.1 to about 25 mole percent. For example, in one embodiment, the percentage of α-hydroxy acid containing subunits in the polymer is from about 0 to about 50 mole percent. In another embodiment, the percentage of α-hydroxy acid containing subunits in the polymer is from about 0 to about 30 mole percent. In yet another particular embodiment, the percentage of α-hydroxy acid containing subunits in the polymer is from about 0.05 to about 25 mole percent. In yet another embodiment, the percentage of α-hydroxy acid containing subunits in the polymer is from about 0.1 to about 25 mole percent, about 0.1 to about 50 mole percent, about 10 to about 40 mole percent, about 5 to about 25 mole percent, about 10 to about 25 mole percent, or about 15 to about 25 mole percent. As an illustration, the percentage of α-hydroxy acid containing subunits may be 0, 1, 2, 3, 4, 5, 6, 7, 8, 9, 10, 11, 12, 13, 14, 15, 16, 17, 18, 19, 20, 21, 22, 23, 24, 24, 26, 27, 28, 29 or 30 mole percent, including any and all ranges lying therein, formed by combination of any one lower mole percentage number with any higher mole percentage number. In one embodiment, the percentage of α-hydroxy acid containing subunits is less than 40 mole percent or less than 30 mole percent.

More particularly, a poly(orthoester) for use in the compositions and delivery systems provided herein is described by the following formula: 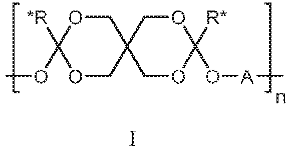 where: R* is a C₁₋₄ alkyl (e.g., C1, C2, C3 or C4 alkyl), n is an integer ranging from 5 to 400, and A in each subunit is R¹ or R³. That is, in any monomer unit 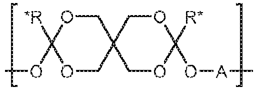 of the polymer of Formula I, A may be either R¹ or R³.

In a particular embodiment, R* is ethyl (i.e., C2 alkyl). A subunit in accordance with formula I, wherein R* is ethyl, corresponds to a subunit resulting from reaction of a diol as provided herein with 3,9-di(ethylidene)-2,4,8,10-tetraoxaspiro[5.5]undecane (DETOSU), a diketene acetal having the structure:

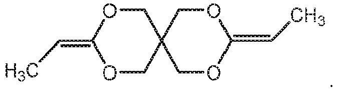

In reference to Formula I, as described previously, A may correspond to R¹. R¹ is 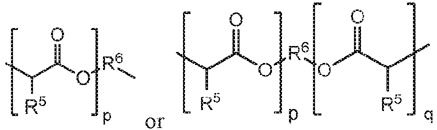 where p and q are each independently integers that range from between about 1 to 20 (e.g., are each independently selected from 1, 2, 3, 4, 5, 6, 7, 8, 9, 10, 11, 12, 13, 14, 15, 16, 17, 18, 19, and 20), each R⁵ is independently hydrogen or C₁₋₄ alkyl (e.g., is H, or C1, C2, C3, or C4 alkyl); and R⁶ is: 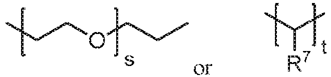 where s is an integer from 0 to 10 (e.g., is selected from 0, 1, 2, 3, 4, 5, 6, 7, 8, 9, 10); t is an integer from 2 to 30; and R⁷ is hydrogen or C₁₋₄ alkyl (e.g., is H or C1, C2, C3, or C4 alkyl). In one or more particular embodiments, R⁷ is H. The R¹ subunits are α-hydroxy acid-containing subunits, i.e., subunits derived from an α-hydroxy acid or a cyclic diester thereof.

In reference to Formula I, A may also correspond to R³, where R³ is: 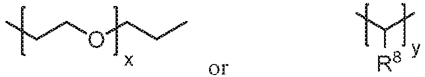 and x is an integer ranging from 1 to 100, and is, in certain particular instances, selected from 1, 2, 3, 4, and 5; y is an integer in a range from 2 to 30; and R⁸ is hydrogen or C₁₋₄ alkyl (C1, C2, C3 or C4 alkyl).

In a particular embodiment, R⁸ is H.

In some embodiments,, the poly(orthoester) is one in which A is R¹ or R³, where R¹ is 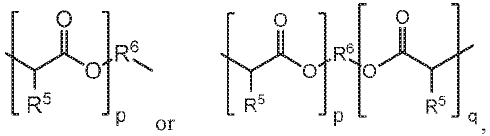 where p and q are each independently integers that range from between about 1 and 20, where the average number of p or the average number of the sum of p and q (p + q) is between about 1 and 7 (e.g., 1, 2, 3, 4, 5, 6, 7) when R¹ is present in the poly(orthoester) polymer; x and s are each independently an integer ranging from 0 to 10; and t and y are each independently an integer ranging from 2 to 30. In one or more particular embodiments, R⁵ is H.

Additional particular poly(orthoesters) are those in which A is R¹ or R³, where R¹ is 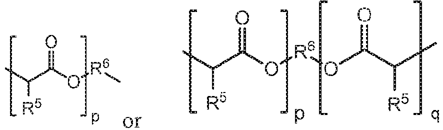 and p and q are each independently integers that vary from between about 1 and 20, or between about 1 and 15, or between about 1 and 10, where the average number of p or the average number of the sum of p and q (i.e., p + q) is between about 1 and 7 when R¹ is present in the poly(orthoester) polymer. Additionally, particular ranges of x and s (in reference to the particular embodiment above or in reference to any polyorthoester as provided herein) are those in which each is independently an integer ranging from 0 to 7 or from 1 to 5. Similarly, particular ranges for t and y are those in which each independently varies from 2 to 10.

Particular polyorthoesters are those in which R⁵ is hydrogen or methyl.

In certain particular embodiments, s and x are each independently selected from 1, 2, 3, 4, 5, 6, 7 and 8. In some particular embodiments, s is 2. In some other particular embodiments, x is 2.

An exemplary polyorthoester comprises alternating residues of 3,9-diethyl-3,9-2,4,8,10-tetraoxaspiro[5.5]undecane-3,9-diyl and A : 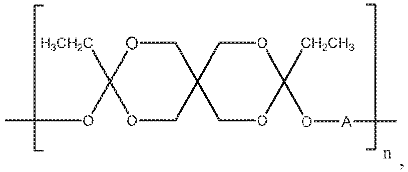 where A is as described above.

Polyorthoesters such as those described herein can be prepared by reacting an illustrative diketene acetal, 3,9-di(ethylidene)-2,4,8,10-tetraoxaspiro[5.5]undecane (DETOSU), 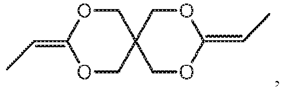 with one or more diols as described above, such as HO-R¹-OH or HO-R³-OH. Illustrative diols include oligoethylene glycols such as triethylene glycol (TEG), oligoethylene glycols modified at one or both termini with an α-hydroxy acid such as an oligoethylene glycol diglycolide or an oligoethylene glycol dilactide, organic diols having a hydrocarbyl core of from 2 to 30 carbon atoms such as 1,6-hexanediol, 1,10-decanediol, cis/trans 1,4-cyclohexane dimethanol, para-menthane-3,8-diol, 1,4-butanediol, 1,5-pentanediol, 1,7-heptanediol, 1,8-octanediol, 1,10-decanediol, 1,12-dodecanediol, and cyclic equivalents thereof, where the hydroxyl groups can be at any two positions within the cycloalkyl or alkylene ring. An organic diol can possess from 2 to 20 carbon atoms. The organic diol can be linear, branched or cyclic, and may also be saturated or unsaturated. Generally, unsaturated diols will possess from 1-3 elements of unsaturation. A particular poly(orthoester) will contain from about from 10 to 50 total mole percent of subunits derived from one or more organic diols having a hydrocarbyl core.

Diols such as HO-R¹-OH are prepared as described in U.S. Patent No. 5, 968,543 and in Heller et al., J. Polymer Sci., Polymer Letters Ed. 18:293-297 (1980). For example, a diol of the formula HO-R¹-OH comprising a polyester moiety can be prepared by reacting a diol of the formula HO-R³-OH with between 0.5 and 10 molar equivalents of a cyclic diester of an α-hydroxy acid such as lactide or glycolide, and allowing the reaction to proceed at 100-200° C for about 12 hours to about 48 hours. Suitable solvents for the reaction include organic solvents such as dimethylacetamide, dimethyl sulfoxide, dimethylformamide, acetonitrile, pyrrolidone, tetrahydrofuran, and methylbutyl ether. Although the diol product. is generally referred to herein as a discrete and simplified entity, e.g., TEG diglycolide (and diol reaction products such as TEG diglycolide), it will be understood by those of skill in the art that due to the reactive nature of the reactants, e.g., ring opening of the glycolide, the diol is actually a complex mixture resulting from the reaction, such that the term, TEG diglycolide (or any other term referring a similar product), generally refers to the average or overall nature of the product.

A particular polyorthoester is prepared by reacting 3,9-di(ethylidene)-2,4,8,10-tetraoxaspiro[5.5]undecane (DETOSU) with one or more reactive diols. Generally, the polyorthoester is prepared by reacting DETOSU with two or more reactive diols under anhydrous conditions. A particular polyorthoester is prepared by reacting DETOSU with triethylene glycol and triethylene glycol diglycolide as described in U.S. Patent No. 8,252,305. A particular polyorthoester prepared from DETOSU-triethylene glycol- triethylene glycol diglycolide possesses the following molar ratios of components: 90:80:20, although the relative ratios of components can be suitably varied as described above.

A polyorthoester formed by the reaction of DETOSU with TEG and TEG diglycolide can generally be described as possessing the following subunits, where R¹ corresponds to the diolate portion derived from triethylene glycol diglycolide (formed by reaction of glycolide with TEG) and R³ corresponds to the diolate portion derived from triethylene glycol: where A is R¹, and R¹ is 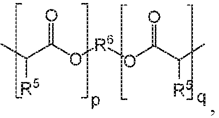 where R⁵ is H and R⁶ is 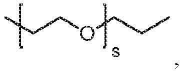 the resulting component of the polyorthoester is: 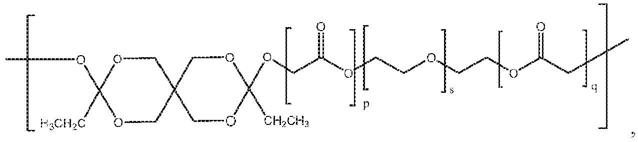 where the sum of p and q is, on average, 2 and s is 2; and when A is R³, and R³ is 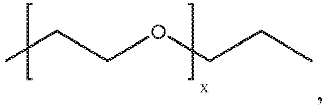 where x is 2, the resulting subunit or component of the polyorthoester is: 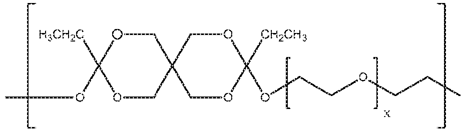 Structures corresponding to polyorthoesters prepared from the various α-hydroxy acid-containing subunits and additional diols described herein can be readily envisioned.

Exemplary polyorthoesters possess a weight average molecular weight of about 1000 Da to about 200,000 Da, for example from about 2,500 Da to about 100,000 Da or from about 3,500 Da to about 20,000 Da or from about 4,000 Da to about 10,000 Da or from about 5,000 Da to about 8,000 Da. Illustrative molecular weights, in Da, are 2500, 5000, 5500, 6000, 6500, 7000, 7500, 8000, 8500, 9000, 9500, 10,000, 20,000, 30,000, 40,000, 50,000, 60,000, 70,000, 80,000, 90,000, 100,000, 120,000, 150,000, 175,000 and 200,000, and ranges therein, wherein exemplary ranges include those formed by combining any one lower molecular weight as described above with any one higher molecular weight as provided above, relative to the selected lower molecular weight.

In one particular embodiment related to the polyorthoester in the delivery system, the polyorthoester has a molecular weight ranging from about 2,500 daltons to 10,000 daltons.

In one embodiment, the poly(orthoesters) described in this section are semi-solids both at room temperature and at temperatures above room temperature. In one embodiment, polyorthoesters containing 80 to 100 mole% R³, where R³ is 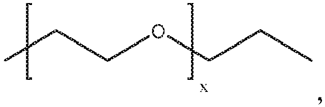 where x is 2, are semisolid polymers at both room temperature and at temperatures above room temperature. Semisolid polymers exist either in a glassy or viscous liquid state. Semisolid polymers typically display a glass transition temperature (Tg) below room temperature. Below the Tg, semisolid polymers can be considered to exist in a glassy state, while above the Tg, the polyorthoester can be considered to exist in a liquid state. Semisolid polyorthoester polymers are not thermoplastic polymers.

Generally, polyorthoesters in accordance with any one of the following formulae, Formula I, Formula II, Formula III or Formula IV, are suitable for use in the compositions and/or delivery vehicles provided herein: 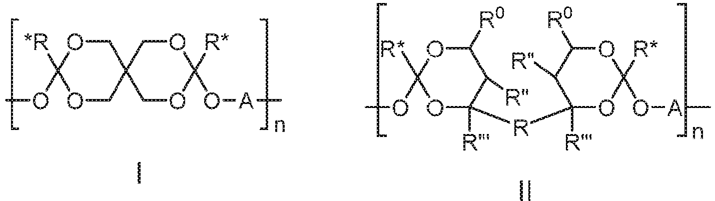 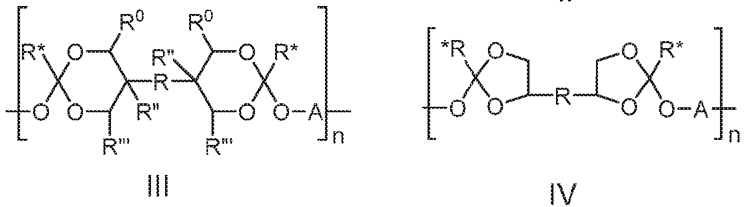 In reference to formulas I-IV,
R is a bond, -(CH₂)ₐ-, or -(CH₂)_{b}-O-(CH₂)_{c}-; where a is an integer from 1 to 12 (e.g., selected from 1, 2, 3, 4, 5, 6, 7, 8, 9, 10, 11, and 12), and b and c are independently integers from 1 to 5 (e.g., selected from 1, 2, 3, 4, and 5);
R* is a C₁₋₄ alkyl;
R°, R" and R‴ are each independently H or C₁₋₄ alkyl;
n is an integer of at least 5; and
A is a diol.

For example, the compositions and delivery vehicles described herein may be comprised of a polyorthoester of Formula I, Formula II, Formula III or Formula IV, where:
R is a bond, -(CH₂)ₐ-, or -(CH₂)_{b}-O-(CH₂)_{c}-; where a is an integer of 1 to 12, and b and c are independently integers of 1 to 5;
R* is a C₁₋₄ alkyl;
R°, R" and R‴ are each independently H or C₁₋₄ alkyl;
n is an integer of at least 5; and
A is R¹, R², R³, or R⁴, where
R¹ is an α-hydroxy acid containing subunit as described in the preceding paragraphs;
R⁵ is hydrogen or C₁₋₄ alkyl (e.g., methyl, ethyl, propyl, butyl, isopropyl, isobutyl, sec-butyl); and
R⁶ is selected from the group consisting of: 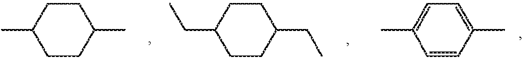 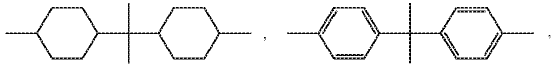 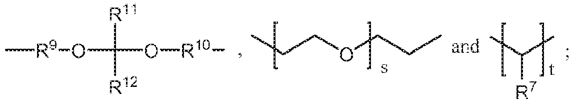 where:
   s is an integer ranging from 0 to 10;
   t is an integer ranging from 2 to 30; and
   R⁷ is hydrogen or C₁₋₄ alkyl;
   R² is: 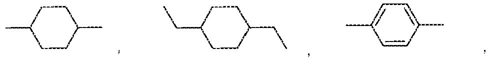 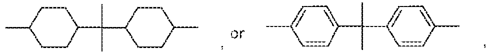
   R³ is: 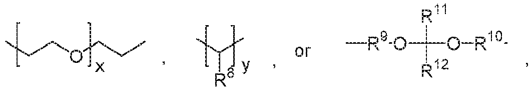 where:
      x is an integer ranging from 0 to 200;
      y is an integer ranging from 2 to 30;
      R⁸ is hydrogen or C₁₋₄ alkyl;
      R⁹ and R¹⁰ are independently C₁₋₁₂ alkylene;
      R¹¹ is hydrogen or C₁₋₆ alkyl and R¹² is C₁₋₆ alkyl; or R¹¹ and R¹² together are C₃₋₁₀ alkylene; and
      R⁴ is the residue of a diol containing at least one functional group independently selected from an amide, an imide, a urea, and a urethane (carbmate) group.

In certain instances, the polyorthoester is one according to any one of Formulae I-IV in which A is R¹, R³, or R⁴, where
R³ is selected from: 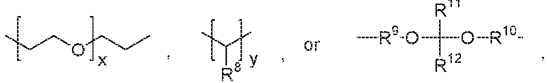 where:
x is an integer of 0 to 100;
y is an integer of 2 to 30;
R⁸ is hydrogen or C₁₋₄ alkyl;
R⁹ and R¹⁰ are independently C₁₋₁₂ alkylene;
R¹¹ is hydrogen or C₁₋₆ alkyl and R¹² is C₁₋₆ alkyl; or R¹¹ and R¹² together are C₃₋₁₀ alkylene;
R⁴ is a residual of a diol containing at least one functional group independently selected from amide, imide, urea and urethane groups; and R⁵ is hydrogen or C₁₋₄ alkyl.

In one particular embodiment of the polyorthoester, the fraction of the A units that are of the formula R¹ is between 0 and 20 mole percent.

One exemplary polyorthoester is described by formula I, II, III or IV, where: none of the units have A equal to R²;
R³ is: 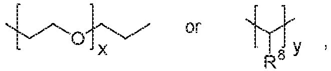 where:
x is an integer of 1 to 100;
y is an integer of 2 to 30; and
R⁶ is: 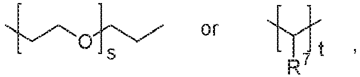 where:
   s is an integer of 1 to 10;
   t is an integer of 2 to 30; and
   R⁵, R⁷, and R⁸ are independently hydrogen or methyl.

An additional representative polyorthoester of Formula I, II, III or IV, is one in which R³ and R⁶ are both -(CH₂-CH₂-O)₂-(CH₂-CH₂)-; R⁵ is methyl; and where p and q are each independently selected from 1, 2, 3, 4, 5, 6, 7, 8, 9, 10, 11, 12, 13, 14, 15, 16, 17, 18, 19, and 20.

In another embodiment of a polyorthoester of Formula I, II, III or IV, R³ and R⁶ are both - (CH₂-CH₂-O)₉-(CH₂-CH₂)-; R⁵ is methyl; and p or the sum of p and q is on average 2.

In another variation, the polyorthoester is of Formula I, II, III or IV, R is -(CH₂)_{b}-O-(CH₂)_{c}-; where b and c are both 2; R* is a C₂ alkyl.

Additional representative polyorthoesters of Formula I, II, III or IV, are those in which R⁵ is hydrogen or methyl; R⁶ is 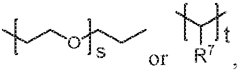 where *s* is an integer from 1 to 10, or in some embodiments s is selected from 1, 2, 3, or 4; *t* is an integer from 2 to 30, particularly selected from 2, 3, 4, 5, 6, 7, 8, 9 and 10; R⁷ is hydrogen or methyl; and R³ is 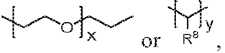 where x is an integer from 1 to 10, or in some embodiments is selected from 1, 2, 3, or 4; *y* is an integer from 2 to 30, particularly selected from 2, 3, 4, 5, 6, 7, 8, 9 and 10; R⁸ is hydrogen or methyl; R⁴ is selected from a residue of an aliphatic diol having from 2-20 carbon atoms (e.g., selected from 2, 3, 4, 5, 6, 7, 8, 9, 10, 11, 12, 13, 14, 15, 16, 17, 18, 19, and 20 carbon atoms), and in some embodiments R⁴ has from 2 to 10 carbon atoms, interrupted by one or two amide, imide, urea, or urethane groups. In some cases, the proportion of subunits in the polyorthoester in which A is R¹ is from about 0.01-50 mole percent. In certain instances, the proportion of subunits in the polyorthoester in which A is R¹ is from about 0 to about 30 mole percent, or from about 0.1 to 25 mole percent. Illustrative mole percentages include 10, 15, 20 and 25 mole percent of subunits in the polyorthoester in which A is R¹. In one embodiment, the mole percent is 20. Additionally, in one or more embodiments, the proportion of subunits in which A is R² is less than about 20 percent, less than about 10 percent, or less than about 5 percent, and the proportion of subunits in which A is R⁴ is less than 20 percent, less than about 10 percent or less than 5 percent.

The polyorthoester, as shown in Formula I, Formula II, Formula III and Formula IV, in certain embodiments, is one of alternating residues of a diketene acetal and a diol, with each adjacent pair of diketene acetal residues being separated by the residue of one polyol, such as a diol.

Methods of manufacturing the polyorthoesters are well known in the art, and are described, e.g., in U.S. Patent Nos. 6,613,355 and 8,252,304.

### 6. Optional Solvents and Excipients

The composition may additionally comprise one or more pharmaceutically acceptable excipients, and some examples are now set forth.

In the embodiment wherein the delivery vehicle is a polymeric formulation, and in particular where the polymer is a polyorthoester, the delivery vehicle may optionally comprise an organic acid, such as that described in co-owned U.S. Patent Application No. 61/982,300, filed April 21, 2014. The organic acid facilitates release of the active agent, such as caine type local anesthetic, from the vehicle or composition, in particular, during the early stages of delivery (e.g., days 1-3 post-administration). Generally, the organic acid is a carboxylic acid. Most suitable are organic acids having a molecular weight less than about 300 daltons. Representative organic acids include, e.g., fumaric or maleic acid, ethanoic acid, propanoic acid, butanoic acid, pentanoic acid, hexanoic acid, heptanoic acid, benzoic acid, salicylic acid and acetyl salicylic acid, oxalic acid, malonic acid, succinic acid, glutaric acid, adipic acid, pimelic acid, and so forth.

The delivery vehicle may comprise from about 0.01 wt% to 0.6 wt% or from about 0.01 wt% to 0.2 wt% of a di-carboxylic acid. The amount of the organic acid additive comprised in the vehicle will depend, at least in part, upon the identity of the particular active agent, the amount of active agent contained in the vehicle, the particular polyorthoester, amount thereof, and desired delivery profile.

For a given organic acid, vehicles comprising a greater amount of the organic acid exhibit a faster release rate which is typically most pronounced during the first 1-3 days following administration and eliminate crystallization of the active agents from the homogeneous composition.

In another embodiment, the delivery vehicle in the form of a semi-solid polyorthoester polymeric formulation may also contain one or more liquid excipients. The excipient can be a pharmaceutically-acceptable polyorthoester compatible liquid excipient. Such excipients are liquid at room temperature and are readily miscible with polyorthoesters. Exemplary polyorthoester compatible liquid excipients include both protic and aprotic solvents. Protic liquid excipients include polyethylene glycol having a molecular weight between about 200 Da and 4,000 Da, or a polyethylene glycol derivative or co-polymer having a molecular weight between about 200 Da and 4,000 Da, e.g., an endcapped PEG such as monomethoxypolyethylene glycol, or a mono-, di- or triglyceride of a C2-C19 aliphatic carboxylic acid or a mixture of such acids, and alkoxylated tetrahydrofurfuryl alcohols. Additional suitable liquid excipients include C1-C4 alkyl ethers of alkoxylated tetrahydrofurfuryl alcohols, and C2-C19 aliphatic carboxylic acid esters, or the like. A particular excipient for semi-solid vehicles is monomethoxy-PEG, having a molecular weight selected from 400, 450, 500, 550, 600 and 650 Da.

Additional liquid excipients include aprotic solvents. Aprotic solvents suitable for use, as well as exemplary polyorthoester vehicles comprising an aprotic solvent are described in U.S. Patent Application Publication No. 2014/0275046. Examples of hydrophilic biocompatible, aprotic organic solvents include, for example, amides such as N-methyl-2-pyrrolidone (NMP), 2-pyrrolidone, N-ethyl-2-pyrrolidone, N-cycylohexyl-2-pyrrolidone, dimethyl acetamide, and dimethyl formamide; esters of monobasic acids such as methyl lactate, ethyl lactate, and methyl acetate; sulfoxides such as dimethyl sulfoxide and decylmethylsulfoxide; lactones such as e-caprolactone and butyrolactone; ketones such as acetone and methyl ethyl ketone; and ethers such as dimethyl isosorbide and tetrahydrofuran.

An exemplary semi-solid composition comprises a polyorthoester, a liquid excipient such as NMP or DMSO, a caine type local anesthetic such as bupivacaine or ropivacaine, and an NK-1 receptor antagonist such as aprepitant and optionally an organic acid additive such as maleic acid. The relative concentrations of the components of the semi-solid composition will vary depending upon the amount of the caine local anesthetic(s), NK-1 receptor antagonist, polyorthoester, polyorthoester-compatible liquid excipient, and organic acid additive, if present. The weight percent of the polyorthoester compatible liquid excipient can range from about 10-50 weight percent, or from about 10-40 weight percent, or from 10-30 weight percent, or from 10-25 weight percent, or from 1 to 20 weight percent, or from 5-15 weight percent, or from 5-10 weight percent. Exemplary amounts of the polyorthoester-compatible liquid excipient are about 5, 6, 7, 8, 9, 10, 12, 15, 20, 25, 30, 35, 40, 45 or 50 weight percent.

In another embodiment, the compositions described herein and in particular the semi-solid composition comprising a polyorthoester, a liquid excipient such as NMP or DMSO, a caine type local anesthetic such as bupivacaine or ropivacaine, and a NK-1 receptor antagonist such as apreptiant and optionally an organic acid additive, additionally comprises a viscosity reducing triglyceride solvent such as those disclosed in this application.

The delivery vehicle in the form of a semi-solid polymeric formulation can be prepared by mixing or blending the active agents, the polymer, such as the polyorthoester, an optional polymeric/polyorthoester-compatible liquid excipient, and any other additional additives or excipients as desired. The mixing or blending can be performed by any suitable method, generally at a temperature less than about 50°C, e.g., at room temperature, although in certain instances, depending upon the nature of the materials, mixing or blending may be carried out at higher temperatures, e.g., from about 25 to 100°C. The mixing or blending is generally carried out in the absence of additional solvents, to obtain a homogeneous, flowable and non-tacky vehicle at room temperature.

The polymeric-compatible liquid excipient is typically added to the compositions in an amount ranging from about 5 percent to about 70 percent by weight, relative to the total weight of the composition. The liquid excipient may be present in the composition in an amount ranging from about 5 percent to about 50 percent by weight. In other embodiments, the liquid excipient is present in the composition in an amount ranging from about 5-10 wt%, 5-15 wt%, 10-60 wt%, 15-60 wt%, 15-50 wt%, 20-60 wt%, 25-50 wt%, 30-70 wt%, 30-60 wt%, 30-50 wt%, 35-70 wt%, 35-60 wt% or 35-50 wt%.

The rate of release of the active agent (e.g., drug) can be controlled by adjusting the composition and amount of the polymer and/or by the selection and quantity of the optional additives/excipients. The chemical structure of the polymer (i.e., the type of monomer used or the ratio of monomers for copolymers or terpolymers, the end groups on the polymer chains, and the molecular weight of the polymer) will determine the hydrophilicity or lipophilicity of the polymer material as well as contribute to the degradation time of the polymer depot. More hydrophilic polymers (e.g., polyorthoesters wherein the diol monomer is hydrophilic, e.g., triethylene glycol, tetraethylene glycol, or polyethylene glycol and the like) are used in applications where faster release rates and shorter durations of release are needed. The composition includes the delivery vehicle and the active agents in an amount effective to provide the desired therapeutic effect over the release period.

While the singular form is used to describe the polyorthoester and other composition components in this application, it is understood that more than one polyorthoester and/or more than one caine type local anesthetic or NK-1 receptor antagonist selected from the groups described above may be used in the delivery system. In some embodiments of the herein described compositions, the compositions further comprise one or more additional excipients. In one embodiment, a particular excipient is one that does not influence the release of the active agents from the composition.

It is also understood that while not required, other pharmaceutically acceptable inert agents such as coloring agents and preservatives may also be incorporated into the composition.

### 7. Aqueous Compositions Comprising a Caine and NK-1 Receptor Antagonist

As described herein, it was discovered that administering a combination of a caine type local anesthetic and a NK-1 receptor antagonist provides a surprisingly effective level and duration of pain relief in a subject. Based upon the disclosures and guidance provided herein, a person having ordinary skill in the art would understand that the combination of a caine type local anesthetic and a NK-1 receptor antagonist would also be more effective than an equal amount of the caine type local anesthetic or the NK-1 receptor antagonist administered alone. Accordingly, also disclosed are aqueous solutions comprising a caine type local anesthetic and a NK-1 receptor antagonist. In a particular embodiment, the NK-1 receptor antagonist in the aqueous composition is aprepitant. In a more particular embodiment, the aqueous composition comprises aprepitant and bupivacaine.

Caine type local anesthetics which are suitable for the aqueous combination are commercially available, for example, as injectable solutions and include but are not limited to lidocaine, mepivacaine, bupivacaine, and etidocaine. Pharmaceutically acceptable solutions of an NK-1 receptor antagonist such as aprepitant are disclosed, for example, in U.S. Patent Application No. 15/012,532 and U.S. Pat. No. 9,561,229. Accordingly, a pharmaceutically acceptable solution of, for example, aprepitant, can be mixed with a solution of the caine-type local anesthetic prior to administration to a subject. For example, the mixing can be done less than an hour prior to administration or within 2 hours, 4 hours, 6 hours, 8 hours, 10 hours, 12 hours, 14 hours, 16 hours, 18 hours, 20 hours, 22 hours or 24 hours prior to administration. The mixture of the caine type local anesthetic with the NK-1 receptor antagonist provides a pain relief which is more effective than the same amount of either the caine ty pe local anesthetic or the NK-1 receptor antagonist alone. Greater efficacy in providing pain relief of such a combination formulation can be measured, for example, using the von Frey assay, wherein pain tolerance of a subject will be greater when administered a combination of meloxicam and the amide-type local anesthetic than when administered either active agent alone.

Accordingly, in one embodiment, an aqueous pharmaceutical composition comprising a therapeutically effective amount of aprepitant and a therapeutically effective amount of a caine type local anesthetic is contemplated. In one embodiment, administration of the composition to a subject provides pain relief to the subject for a period of about 4 days to about 6 days after administration.

In another embodiment, an aqueous solution comprising a therapeutically effective amount of aprepitant is provided, wherein the solution is suitable for adding to a pharmaceutical solution comprising a therapeutically effective amount of a caine type local anesthetic to generate a mixed solution which is suitable for administering to a subject in need thereof. In one embodiment, administration of the mixed solution to the subject provides pain relief to the subject for a period of about 4 hours to about 12 hours after the administration, alternatively for a period of about 4-24 hours, or 2-4 hours, or 2-6 hours, or 3-5 hours.

In one embodiment, the mixed solution is for use in a method for treating a subject in pain, wherein the method comprises mixing a pharmaceutical solution of aprepitant with a pharmaceutical solution of a caine type local anesthetic to prepare a mixed solution, and administering the mixed solution to the subject within 24 hours of preparing the mixed solution. The method may also comprise prophylactically treating a subject for pain.

In one embodiment, a composition comprising aprepitant, bupivacaine or ropivacaine, meloxicam, and a polyorthoester is provided. In some embodiments, the polyorthoester is any of Formula I -IV. In some embodiments, the composition further comprises at least one selected from aprotic solvent, organic acid, and a viscosity reducing agent. In some embodiment, the composition comprises aprepitant, bupivacaine, meloxicam, maleic acid, DMSO or NMP, triacetin, and a polyorthoester.

### III. METHOD OF TREATING POST-SURGICAL PAIN

The present disclosure is also directed to a method of treating post-surgical pain in a patient in need thereof. The method comprises administering to a patient in need thereof an NK-1 receptor antagonist in combination with a pain medication.

In some embodiments, the NK-1 receptor and the pain medication are administered concurrently, wherein the NK-1 receptor antagonist is administered in a pharmaceutical composition which also comprises the pain medication and a pharmaceutically acceptable excipient. In some embodiments, the NK-1 receptor and the pain medication are administered concurrently, wherein the NK-1 receptor antagonist is administered in a pharmaceutical composition that is separate from the composition comprising the pain medication.

In some embodiments, the NK-1 receptor and the pain medication are administered sequentially, wherein the NK-1 receptor antagonist and the pain medication are in separate pharmaceutical compositions comprising a delivery vehicle, each of which may further comprise a pharmaceutically acceptable excipient.

In some embodiments, the NK-1 receptor antagonist is administered to the patient in need thereof before the surgery and the pain medication is administered during the surgery. In some embodiments, the pain medication is administered to the patient in need thereof before the surgery and the NK-1 receptor antagonist is administered during the surgery. In some embodiments, both the NK-1 receptor antagonist and the pain medication are administered to the patient in need thereof post surgery. In some embodiments, both the NK-1 receptor antagonist and the pain medication are administered before the surgery.

In some embodiments, the method disclosed herein comprises administering to patient in need of post-surgical pain treatment a NK-1 receptor antagonist in combination with a pain medication selected from local anesthetics, opioids, NSAIDs, anticonvulsants, serotonin and norepinephrine reuptake inhibitors (SNRIs), acetaminophen, and tricyclic antidepressants. In some embodiments, the method disclosed herein comprises administering to a patient in need of post-surgical pain treatment a NK-1 receptor antagonist in combination with a local anesthetic.

In some embodiments, the method disclosed herein comprises administering to patient in need of post-surgical pain treatment a NK-1 receptor antagonist in combination with two pain medications selected from local anesthetics, opioids, NSAIDs, anticonvulsants, serotonin and norepinephrine reuptake inhibitors (SNRIs), acetaminophen, and tricyclic antidepressants. In some embodiments, the method disclosed herein comprises administering to a patient in need of post-surgical pain treatment a NK-1 receptor antagonist in combination with acetaminophen and an opioid. In some embodiments, the method disclosed herein comprises administering to a patient in need of post-surgical pain treatment a NK-1 receptor antagonist in combination with a local anesthetic and a NSAID.

In some embodiments, the method further comprises administration of the NK-1 receptor antagonist daily after surgery for 1, 2, 3, 4, 5, or 6 days.

In some embodiments, the method disclosed herein comprises administering to a patient in need of post-surgical pain treatment (1) a NK-1 receptor antagonist before (such as once) and after surgery daily for 1-6 days and (2) pain medication during surgery. In some emboidments, the NK-1 receptor antagonist is apreptiant. In some embodiments the pain medication includes both a local anesthetic, such as bupivacaine or ropivacaine and a NSAID, such as meloxicam.

In some embodiments, the method disclosed herein comprises administering to a patient in need of post-surgical pain treatment (1) a NK-1 receptor antagonist and a NSAID before (such as once) and after surgery once daily for 1-6 days and (2) a local anesthetic during surgery. In some embodiments, the NK-1 receptor antagonist is apreptiant. In some emboidments, the NSAID is meloxicam. In some embodiments, the local anesthetic is bupivacaine or ropivacaine.

In some embodiments, the administration of the NK-1 receptor antagonist is via systemic administration, such as intravenous administration or oral administration. In some embodiments, the administration of the NK-1 receptor antagonist is via local administration, such as topical administration.

In some embodiments, the administration of the pain medication is via systemic administration, such as oral or intravenous administration. In some embodiments, the administration of the pain medication is via local administration, such as topical administration.

In some embodiments, the administration of the composition comprising NK-1 receptor antagonist and tha pain medication is via systemic administration, such as oral or intravenous administration of the composition. In some embodiments, the administration of the composition is via local administration, such as topical administration of the composition.

### A. NK-1 RECEPTOR ANTAGONIST

Suitable NK-1 receptor antagonists for use in the method include RP 67580 ((3aR,7aR)-Octahydro-2-[1-imino-2-(2-methoxyphenyl)ethyl]-7,7-diphenyl-4H-isoindol)), WIN 51078 (17-β-Hydroxy-17-α-ethynyl-5-α-androstano[3,2- b]pyrimido[1,2-a]benzimidazole), 1-733,060, (((2S,3S)-3-[[3,5-bis(Trifluoromethyl) phenyl]methoxy]-2-phenylpiperidine hydrochloride), 1-703,606 (cis-2-(Diphenylmethel)-N- ([2-iodophenyl]methyl)-1-azabicyclo(2.2.2)octan-3-amine) MDL 105,212 (R)-1-[2-[3-(3,4- dichlorophenyl)-1-(3,4,5-trimethoxybenzoyl)-pyrrolidin-3-yl]-ethyl]-4-phenylpiperidine-4-carboxamide hydrochloride), serlopitant, maropitant, Antagonist D, aprepitant, fosaprepitant, R116301, CGP49823, CP-96345, CP-99994, GR-203040, MDL-103392, 1-760735, SDZ-NKT-343, nolpitanitium (SR-140333), AV608, rolapitant, SCH 900978, AV608, GSK424887 (GlaxoSmithKline), GSK206136 (GlaxoSmithKline), GR-205171, CP-99994, TAK 637 ((S)-7-(3,5-Bis-trifluoromethyl-benzyl)-9-methyl-5-p-tolyl-8,9,10,11-tetrahydro-7H-1,7,11a-triaza-cycloocta[b]naphthalene-6,12-dione), LY303870 ([(R)-1-[N-(2-methoxybenzyl)acetylamino]-3-(1H-indol-3-yl)-2-[N-(2-(4- (piperidin-1-yl)piperidin-1-yl)acetyl)amino]propane]), LY686017 ((2-chloro-phenyl)-{2-[5-pyridin-4-yl-1-(3,5-bistrifluoromethyl-benzyl)-1H-[1,2,3]triazol-4-yl]-pyridin-3-yl}-methanone), E-6006, casopitant/GW679769 ((2R,4S)-4-(4-acetylpiperazin-1-yl)-N-1(1R)-1-[3,5-bis(trifluoromethyl)phenyl)ethyl]-2-(4-fluoro-2-methylphenyl)-N-methylpiperidine-1-carboxamide), vestipitant, orvepitant and orvepitant maleate, befetupitant, netupitant, ezlopitant, CP-122721, MPC-4505 (Myriad Genetics, Inc.), CP-122721 (Pfizer, Inc.), CJ-1 2,255 (Pfizer, Inc.), SRR 240600 (Sanofi-Aventis), or TA-5538 (Tanabe Seiyaku Co.) including all pharmaceutically acceptable salts thereof.

### B. PAIN MEDICATION

Pain medication used in the method for treating post-surgical pain as disclosed herein refers to an active agent for the treatment of pain. The active agent is selected from local anesthetics, opioids, NSAIDs, anticonvulsants, serotonin and norepinephrine reuptake inhibitors (SNRIs), acetaminophen, and tricyclic antidepressants.

The local anesthetics can be of the "caine" classification, which include, for example, benzocaine, chloroprocaine, cocaine, cyclomethycaine, dimethocaine, piperocaine, propoxycaine, procaine, proparacaine, tetracaine, articaine, bupivacaine, dibucaine, levobupivacine, lidocaine, mepivacaine, prolocaine, ropivacaine, and trimecaine. In some embodiments, the local anesthetics are selected from bupivacaine, ropivacaine, levobupivacaine, dibucaine, mepivacaine, procaine, lidocaine, and tetracaine. Other local anesthetics include, for example, saxitoxin, neosaxitoxin, tetrodotoxin, menthol, eugenol, and spilanthol. In the method disclosed herein, the local anesthetics can be administered in the form and/or dose regime of a pharmaceutical composition that is FDA-approved.

Opioids are a type of narcotic pain medication. They work by binding to opioid receptors in the brain, spinal cord, and other areas of the body, reducing the pain messages to the brain and feelings of pain. Opioids analgesics include, for example, buprenorphine, butorphanol, dextromoramide, dezocine, dextropropoxyphene, diamorphine, fentanyl, alfentanil, sufentanil, hydrocodone, hydromorphone, ketobemidone, levomethadyl, mepiridine, methadone, morphine, nalbuphine, opium, oxycodone, papaveretum, pentazocine, pethidine, phenoperidine, piritramide, dextropropoxyphene, remifentanil, tilidine, tramadol, codeine, dihydrocodeine, meptazinol, dezocine, eptazocine, and flupirtine. In the method disclosed herein, the opioids can be administered in the form and/or dose regime of a pharmaceutical composition that is FDA-approved.

NSAIDs work by inhibiting the activity of cyclooxygenase. They can be classified into, for example, salicylates, propionic acid derivatives, acetic acid derivatives, enolic acid derivatives, anthranilic acid derivtives, selective cox-2 inhibitors, sulfonanilides, and others such as clonixin, licofelone, and H-harpagide. Exemplary NSAIDs include ketoprofen, diclofenac, diflunisal, etodolac, fenoprofen, flurbiprofen, ibuprofen, indomethacin, tolmetin, piroxicam, celecoxib, meloxicam, nabumetone, naproxen, oxaprozin, rofecoxib, sulindac, valdecoxib, aspirin, and acetaminophen. In some embodiments, the NSAID is selected from coxib derivatives including, for example, celecoxib, rofecoxib, and valdecoxib. In some embodiments, the NSAID is selected from enolic acid derivatives including, for example, piroxicam, meloxicam, tenoxicam, droxicam, lornoxicam, isoxicam, and phenylbutazone. In some embodiments, the NSAID is ibuprofen. In the method disclosed herein, the NSAIDs can be administered in the form and/or dose regime of a pharmaceutical composition that is FDA- approved. For example, the NSAID can administered as Caldolor^{®}, which is an intravenous injection form of ibuprofen. Further for example, the NSAID is administered as Voltaren^{®} Gel, which is a topical gel form of diclofenac.

Anticonvulsants, such as phenobarbital, clonazepam, valproic acid, topiramate, gabapentin, pregabalin and tiagabine, can have antihyperalgesic and antinociceptive activities. In the method disclosed herein, the anticonvulsants can be administered in the form and/or dose regime of a pharmaceutical composition that is FDA- approved. For example, gabapentin can be administered as Gralise^{®}, which is the tablet form of gabapentin; and pregabalin can be administered as Lyrica^{®}, which is the capsule or oral solution form of pregabalin.

SNRIs are a class of medications effective for the treatment of depression. SNRIs are also used to treat conditions such as long-term pain (Tom, the literature says they are used to treat nerve pain). SNRIs approved by the FDA for treating depression include desvenlafaxine, duloxetine, levomilnacipran, and venlafaxine. In the method disclosed herein, the SNRIs can be administered in the form and/or dose regime of a pharmaceutical composition that is FDA-approved. For example, duloxetine can be administered as Cymbalta^{®}, which is the capsule form of duloxetine.

Tricyclic antidepressants have also been found to be effective for pain treatment. They include amitriptyline, imipramine, clomipramine, doxepin, nortriptyline, and desipramine. In the method disclosed herein, the tricyclic antidepressant can be administered in the form and/or dose regime of a pharmaceutical composition that is FDA-approved.

Acetaminophen is used for pain management for both mild to moderate pain and moderate to severe pain, for example, used for postoperative pain relief. In the method disclosed herein, acetaminophen can be administered as Ofirmev^{®}, which is the injection form of acetaminophen approved by the FDA.

In the method disclosed herein, the NK-1 receptor antagonist is administered in the form of a pharmaceutical composition further comprising a pharmaceutically acceptable excipient. In some embodiments, the NK-1 receptor antagonist is administered in a pharmaceutical composition which also comprises the pain medication. In some embodiments, the NK-1 receptor antagonist is administered in a pharmaceutical composition which also comprises a caine type local anesthetic as disclosed above.

### C. NK-1 Receptor Antagonist and Pain Medication Administered in Separate Compositions

In some embodiments, the NK-1 receptor antagonist is administered in a pharmaceutical composition separate from the pharmaceutical composition comprising the pain medication, i.e., the pharmaceutical composition comprising the NK-1 receptor antagonist does not comprise the pain medication. In these embodiments, the pharmaceutical composition comprising the NK-1 receptor antagonist can be an oil-in-water emulsion described U.S. Patent Application No. 15/012,532.

### 1. Pharmaceutical Composition Comprising NK-1 Receptor Antagonist Without Pain Medication

In some embodiments, the NK-1 receptor antagonist is administered in the form of a pharmaceutical composition that is an oil-in-water emulsion comprising the NK-1 receptor antagonist, an emulsifier, an oil, a co-surfactant, a tonicity agent, a pH modifier, and water.

The emulsion can be for intravenous injection and remain both physically and chemically stable during the shelf life of the formulation.

In some embodiments, the oil is selected from the group consisting of coconut oil, olive oil, soybean oil, safflower oil, triglycerides, octyl and decyl glycerate, ethyl oleate, glyceryl linoleate, ethyl linoleate, glyceryl oleate, cholesteryl oleate/linoleate or a mixture thereof. In other embodiments, the oil is hydrolyzed. In still other embodiments, the oil is structurally modified.

In some embodiments, the emulsifier comprises a phospholipid. In another embodiment, the emulsifier is selected from the group consisting of egg phospholipids, soy phospholipids, phosphatidylcholines, phosphatidylethanolamines, phosphatidylserines, phosphatidylglycerols, phosphatidylinositols, phosphatidic acids, mixed chain phospholipids, lysophospholipids, hydrogenated phospholipids, partially hydrogenated phospholipids, and mixtures thereof.

In some embodiments, the co-surfactant comprises an alcohol. In other embodiments, the co-surfactant is ethanol.

In some embodiments, the pH modifier comprises an oleate or pharmaceutically acceptable salt thereof. In other embodiments, the oleate is sodium, potassium or ammonium oleate. In yet other embodiments, the pH modifier is sodium oleate or a pharmaceutically acceptable salt thereof.

In some embodiments, the pH modifier comprises a buffer. In other embodiments, the buffer is selected from the group consisting of phosphate buffer, citrate buffer, Tris buffer, carbonate buffer, succinate buffer, maleate buffer and borate buffer. In still other embodiments, the buffer is selected from the group, phosphate buffered saline (PBS), modified PBS (PBS-mod) and citrate buffer.

In some embodiments, the pH modifier comprises an oleate and a buffer. In other embodiments, the oleate is sodium oleate and the buffer is Tris buffer.

In some embodiments, the pH modifier is selected from the group consisting of sodium hydroxide, potassium hydroxide, magnesium hydroxide, sodium carbonate, Tris, sodium linoleate, sodium oleate, potassium oleate, ammonium oleate, potassium carbonate, potassium linoleate, and mixtures thereof

In some embodiments, the composition comprises about 5 wt/wt% (weight/weight %) to 15 wtiwt%, 5 wt/wt% to 10 wt/wt%, 7 wt/wt% to 10 wt/wt%, 8 wt/wt% to 9 wt/wt%, or 9 wt/wt% to 10 wt/wt% oil. In another embodiment, the composition comprises about 8 wt/wt5, 8.5 wt/wt%, 9 wt/wt%, 9.5 wt/wt%, 10 wt/wt%, or 10.5 wt/wt%, oil. In still other embodiments, the oil is soybean oil.

In some embodiments, the composition comprises about 10 wt/wt% to 20 wt/wt%, 12 wt/wt% to 17 wt/wt%, 13 wt/wt% to 16 wt/wt%, 13 wt/wt% to 15 wt/wt%, 14 wt/wt% to 15 wt/wt%, or 13 wt/wt% to 14 wt/wt% emulsifier. In other embodiments, the composition comprises about 13 wt/wt%, 13.5 wt/wt%, 14 wt/wt%, 14.5 wt/wt%, 15 wt/wt%, 16 wt/wt%, 17 wt/wt%, 18 wt/wt%, 19 wt/wt% or 20 wt/wt% emulsifier. In still other embodiments, the emulsifier is a lecithin. In other embodiments, the lecithin is an egg yolk lecithin.

In some embodiments, the composition comprises about 0.05 wt/wt% to 1.5 wt/wt%, 0.1 wt/wt% to 1.0 wt/wt%, 0.2 wt/wt% to 0.8 wt/wt%, 0.3 wt/wt% to 0.7 wt/wt%, 0.4 wt/wt% to 0.6 wt/wt%, 0.4 wt/wt% to 0.5 wt/wt% oleate or salt thereof. In other embodiments, the composition comprises about 0.05 wt/wt%, 0.1 wt/wt%, 0.2 wt/wt%, 0.3 wt/wt%, 0.4 wt/wt%, 0.45 wt/wt%, 0.5 wt/wt%, 0.6 wt/wt%, 0.7 wt/wt%, 0.8 wt/wt%, 0.9 wt/wt%, 1.0 wt/wt% or 1.5 wt/wt% oleate or salt thereof. In still other embodiments, the oleate is sodium oleate. In still another embodiment, the oleate or sodium oleate is the pH modifier.

In some embodiments, the composition comprises about 20 wt/wt% to 50 wt/wt%, 30 wt/wt% to 50 wt/wt%, 35 wt/wt% to 45 wt/wt%, 30 wt/wt% to 45 wt/wt%, 37 wt/wt% to 42 wt/wt%, 38 wt/wt% to 40 wt/wt%, 30 wt/wt%, 31 wt/wt%, 32 wt/wt%, 33 wt/wt%, 34 wt/wt%, 35 wt/wt%, 36 wt/wt%, 37 wt/wt%, 38 wt/wt%, 39 wt/wt%, 40 wt/wt%, 41 wt/wt%, 42 wt/wt%, 43 wt/wt%, 44 wt/wt%, 45 wt/wt%, 46 wt/wt%, 47 wt/wt%, 48 wt/wt%, 49 wt/wt%, 50 wt/wt% of oil expressed as a percentage of the weight of the oil per the sum of weight of oil, emulsifier and oleate in a unit of the composition. In other embodiments, the oil is soybean oil.

In some embodiments, the composition comprises about 20 wt/wt% to 50 wt/wt%, 30 wt/wt% to 50 wt/wt%, 35 wt/wt% to 45 wt/wt%, 30 wt/wt% to 45 wt/wt%, 37 wt/wt% to 42 wt/wt%, 38 wt/wt% to 40 wt/wt%, 30 wt/wt%, 31 wt/wt%, 32 wt/wt%, 33 wt/wt%, 34 wt/wt%, 35 wt/wt%, 36 wt/wt%, 37 wt/wt%, 38 wt/wt%, 39 wt/wt%, 40 wt/wt%, 41 wt/wt%, 42 wt/wt%, 43 wt/wt%, 44 wt/wt%, 45 wt/wt%, 46 wt/wt%, 47 wt/wt%, 48 wt/wt%, 49 wt/wt%, 50 wt/wt% of oil expressed as a percentage of the weight of the oil per the sum of weight of oil and emulsifier in a unit of the composition. In other embodiments, the oil is soybean oil.

In some embodiments, the composition comprises about 40 wt/wt% to 80 wt/wt%, 50 wt/wt% to 70 wt/wt%, 55 wt/wt% to 65 wt/wt%, 57 wt/wt% to 63 wt/wt%, 58 to 60 wt/wt%, 35 wt/wt% to 40 wt/wt%, 30 wt/wt% to 40 wt/wt%, 50 wt/wt%, 51 wt/wt%, 52 wt/wt%, 53 wt/wt%, 54 wt/wt%, 55 wt/wt%, 56 wt/wt%, 57 wt/wt%, 58 wt/wt%, 59 wt/wt%, 60 wt/wt%, 61 wt/wt%, 62 wt/wt%, 63 wt/wt%, 64 wt/wt%, 65 wt/wt%, 66 wt/wt%, 67 wt/wt%, 68 wt/wt%, 69 wt/wt%, 70 wt/wt% of emulsifier expressed as a percentage of the weight of emulsifier per the sum of weight of oil, emulsifier and oleate in a unit of the composition. In other embodiments, the emulsifier is a lecithin. In still other embodiments, the lecithin is an egg yolk lecithin.

In some embodiments, the composition comprises about 40 wt/wt% to 80 wt/wt%, 50 wt/wt% to 70 wt/wt%, 55 wt/wt% to 65 wt/wt%, 57 wt/wt% to 63 wt/wt%, 58 to 60 wt/wt%, 35 wt/wt% to 40 wt/wt%, 30 wt/wt% to 40 wt/wt%, 50 wt/wt%, 51 wt/wt%, 52 wt/wt%, 53 wt/wt%, 54 wt/wt%, 55 wt/wt%, 56 wt/wt%, 57 wt/wt%, 58 wt/wt%, 59 wt/wt%, 60 wt/wt%, 61 wt/wt%, 62 wt/wt%, 63 wt/wt%, 64 wt/wt%, 65 wt/wt%, 66 wt/wt%, 67 wt/wt%, 68 wt/wt%, 69 wt/wt%, 70 wt/wt% of emulsifier expressed as a percentage of the weight of emulsifier per the sum of weight of oil and emulsifier in a unit of the composition. In other embodiments, the emulsifier is a lecithin. In still other embodiments, the lecithin is an egg yolk lecithin.

In some embodiments, the ratio of oil to NK-1 receptor antagonist (wt%:wt%) in the composition ranges from about 5:1 to 20:1, 5:1 to 15:1, 5:1 to 10:1, 11:1 to 20:1, 11:1 to 15:1, 12:1 to 16:1, 12:1 to 14:1, 11:1 to 15:1, 12:1 to 14:1, 12.5:1 to 13.5:1, 13:1 to 14:1, or 12:1 to 15:1. In other embodiments, the ratio of oil to NK-1 receptor antagonist (wt%:wt%) in the composition is about 11:1 to 20:1, 11:1 to 15:1, 12:1 to 16:1, 12:1 to 14:1, 5:1, 6:1, 7:1, 8:1, 9:1, 10:1, 11:1, 11.5:1, 12:1, 12.5:1, 13:1, 13.5:1, 14:1, 14.5:1 or 15:1, 15.5:1, 16:1.

In some embodiments, the ratio of emulsifier to NK-1 receptor antagonist (wt%:wt%) in the composition ranges from about 10:1 to 30:1, 10:1 to 20:1, 15:1 to 30:1, 20:1 to 25:1, 18:1 to 22:1, 19:1 to 20:1, or 10:1 to 30:1. In other embodiments, the ratio of emulsifier: NK-1 receptor antagonist (wt%:wt%) in the composition is about 10:1, 11:1, 13:1, 14:1, 15:1, 18:1, 19:1, 20:1, 21:1, 22:1 23:1, 24:1, 25*:*1*,* or 30:1.

In some embodiments, the ratio of (emulsifier plus oil) to NK-1 receptor antagonist (wt%:wt%) in the composition ranges from about 20:1 to 40:1, 25:1 to 35:1, 30:1 to 35:1, or 32:1 to 34:1. In other embodiments, the ratio of (emulsifier plus oil) to NK-1 receptor antagonist is about 25:1, 26:1, 27:1, 28:1, 29:1 30:1, 31:1, 32:1, 33:1, 34:1, 35:1, 36:1, 37:1, 38:1 or 40:1.

In some embodiments, the ratio of emulsifier to oil (wt%:wt%) in the composition ranges from about 0.5:1, to 4:1, 1:1 to 2:1, 1.25:1 to 1.75: 1, or 1.4:1 to 1.6:1. In other embodiments, the ratio of emulsifier to oil (wt%:wt%) in the composition is about 0.5:1, 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, 2:1, 1.05:1, 1.15:1, 1.25:1, 1.35:1, 1.45:1, 1.55:1, 1.65:1, 1.75:1, 1.85:1, or 1.95:1.

In some embodiments, a therapeutic dose of the pharmaceutical composition comprises about 1 to 4 g, 1.5 to 3 g, 1.8 to 2.8 g, 2.3 to 2.8 g, 1.8 to 2.3 g, 1 g, 1.1 g, 1.2 g, 1.3 g, 1.4 g, 1.5 g, 1.6 g, 1.7 g, 1.8 g. 1.9 g, 2 g, 2.1 g, 2.2 g, 2.3 g, 2.4 g, 2.5 g, 2.6 g, 2.7 g, 2.8 g. 2.9 g, 3 g, 3.1 g, 3.2 g, 3.3 g, 3.4 g, 3.5 g, 3.6 g, 3.7 g, 3.8 g. 3.9 g, 4 g emulsifier. In other embodiments, the emulsifier is a lecithin. In still other embodiments, the emulsifier is egg yolk lecithin.

In some embodiments, a therapeutic dose of the pharmaceutical composition comprises about 0.5 to 3 g, 1 to 2.5 g, 1 to 2 g, 1 to 1.5 g, 1.5 g to 2 g, 0.5g 0.6 g, 0.7 g, 0.8 g, 0.9 g, 1 g, 1.1 g, 1.2 g, 1.3 g, 1.4 g, 1.5 g, 1.6 g, 1.7 g, 1.8 g. 1.9 g, 2 g, 2.1 g, 2.2 g, 2.3 g, 2.4 g, 2.5 g oil. In other embodiments, the oil is soybean oil.

In some embodiments, a therapeutic dose of the pharmaceutical composition comprises about 50 to 600 mg, 100 to 600 mg, 100 to 500 mg, 100 to 400 mg, 100 to 300 mg, 100 to 200 mg, 200 to 400 mg, 50 to 250 mg, 75 to 200 mg, 100 to 150 mg, 100 mg, 110 mg, 120 mg, 130 mg, 140 mg, 150 mg, 160 mg, 170 mg, 180 mg, 190 mg, 200 mg, 250 mg, 300 mg, 350 mg, 400 mg, 450 mg, 500 mg, or 600 mg of the NK-1 receptor antagonist.

In some embodiments, the composition comprises about 0 wt/wt% to 10 wt/wt%, 1 wt/wt% to 9 wt/wt%, 2 wt/wt% to 6 wt/wt%, 2 wt/wt% to 4 wt/wt% or 2 wt/wt% to 3 wt/wt% co-surfactant. In other embodiments, the composition comprises less than 10 wt/wt%, less than 9 wt/wt%, less than 8 wt/wt%, less than 7, less than 6 wt/wt%, less than 5 wt/wt%, less than 4 wt/wt%, less than 3 wt/wt%, less than 2 wt/wt% or less than 1 wt/wt% co-surfactant.

In some embodiments, the composition comprises about 0 wt/wt% to 10 wt/wt%, 1 wt/wt% to 9 wt/wt%, or 2 wt/wt% to 6 wt/wt% ethanol. In other embodiments, the composition comprises less than 10 wt/wt%, less than 9 wt/wt%, less than 8 wt/wt%, less than 7, less than 6 wt/wt%, less than 5 wt/wt%, less than 4 wt/wt%, less than 3 wt/wt%, less than 2 wt/wt% or less than 1 wt/wt% ethanol.

In some embodiments, the emulsion further comprises an osmotic agent.

In some embodiments, the emulsion further comprises a buffer.

In some embodiments, the emulsion comprises a buffer but does not comprise a pH modifier which is different from the buffer. In other embodiments, the buffer functions as both a pH modifier agent and a buffer.

In some embodiments, when the emulsion comprises a buffer, the emulsion contains no tonicity agent.

In some embodiments, the buffer is selected from the group consisting of phosphate buffer, citrate buffer, Tris buffer, carbonate buffer, succinate buffer, maleate buffer and borate buffer. In other embodiments, the buffer is selected from the group, phosphate buffered saline (PBS), modified PBS (PBS-mod) and citrate buffer.

In some embodiments, the emulsion comprises a buffer and is a substantially isotonic oil in water emulsion.

In some embodiments, the osmotic agent is selected from the group consisting of glycerol, sorbitol, xylitol, mannitol, glucose, trehalose, maltose, sucrose, raffinose, lactose, dextran, polyethylene glycol, or propylene glycol. In other embodiments, the osmotic agent is an inorganic salt such as sodium chloride and mixtures thereof.

In some embodiments, the composition has a pH of about 6 to 9, 7 to 9, 7.5 to 9, 7.5 to 8.5, 8 to 9, 6 to 8, 7 to 8, or 6, 7, 8 or 9.

In some embodiments, the composition comprises about 0 wt/wt% to 25 wt/wt%, 2 wt/wt% to 20 wt/wt%, 3 wt/wt% to 15 wt/wt%, or 3 wt/wt% to 8 wt/wt% tonicity agent. In other embodiments, the composition comprises about 1 wt/wt%, 2 wt/wt%, 3 wt/wt%, 4 wt/wt%, 5 wt/wt%, 6 wt/wt%, 7 wt/wt%, 8 wt/wt%, 9 wt/wt%, or 10 wt/wt%, 11 wt/wt%, 12 wt/wt%, 13 wt/wt%, 14 wt/wt%, 15 wt/wt%, 16 wt/wt%, 17 wt/wt%, 18 wt/wt%, 19 wt/wt%, or 20 wt/wt%, 21 wt/wt%, 22 wt/wt%, 23 wt/wt%, 24 wt/wt%, 25 wt/wt% tonicity agent. In still other embodiments, the composition comprises no tonicity agent.

In some embodiments, the composition comprises about 0 wt/wt% to 25 wt/wt%, 2 wt/wt% to 20 wt/wt%, 3 wt/wt% to 15 wt/wt%, or 3 wt/wt% to 8 wt/wt% osmotic agent. In other embodiments, the composition comprises about 1 wt/wt%, 2 wt/wt%, 3 wt/wt%, 4 wt/wt%, 5 wt/wt%, 6 wt/wt%, 7 wt/wt%, 8 wt/wt%, 9 wt/wt%, or 10 wt/wt%, 11 wt/wt%, 12 wt/wt%, 13 wt/wt%, 14 wt/wt%, 15 wt/wt%, 16 wt/wt%, 17 wt/wt%, 18 wt/wt%, 19 wt/wt%, or 20 wt/wt%, 21 wt/wt%, 22 wt/wt%, 23 wt/wt%, 24 wt/wt%, 25 wt/wt% osmotic agent. In still other embodiments, the composition comprises no osmotic agent.

In some embodiments, the aqueous phase comprises a dose of dexamethasone sodium phosphate in a therapeutic dose of the pharmaceutical composition. In other embodiments, the dose of dexamethasone sodium phosphate ranges from about 0.5 mg to 30 mg, 0.5 mg to 25 mg, 1 mg to 20 mg, 10 mg to 20 mg, or 3 mg to 16 mg. In still other embodiments, the dose of dexamethasone sodium phosphate is about 9 mg or 16 mg in a therapeutic dose of the pharmaceutical composition. In these embodiments comprising a dose of dexamethasone, the therapeutic dose of the pharmaceutical composition comprises about 50 to 600 mg, 100 to 600 mg, 100 to 500 mg, 100 to 400 mg, 100 to 300 mg, 100 to 200 mg, 200 to 400 mg, 50 to 250 mg, 75 to 200 mg, 100 to 150 mg, 100 mg, 110 mg, 120 mg, 130 mg, 140 mg, 150 mg, 160 mg, 170 mg, 180 mg, 190 mg, 200 mg, 250 mg, 300 mg, 350 mg, 400 mg, 450 mg, 500 mg, or 600 mg of the NK-1 receptor antagonist.

In some embodiments, the emulsion comprises a dose of dexamethasone in a therapeutic dose of the pharmaceutical composition. In other embodiments, the dose of dexamethasone ranges from about 0.5 mg to 30 mg, 0.5 mg to 20 mg, 1 mg to 18 mg, 10 mg to 20 mg, or 3 mg to 16 mg. In other embodiments, the dose of dexamethasone is about 8 mg or 12 mg in a therapeutic dose of the pharmaceutical composition. In these embodiments comprising a dose of dexamethasone, the therapeutic dose of the pharmaceutical composition comprises about 50 to 600 mg, 100 to 600 mg, 100 to 500 mg, 100 to 400 mg, 100 to 300 mg, 100 to 200 mg, 200 to 400 mg, 50 to 250 mg, 75 to 200 mg, 100 to 150 mg, 100 mg, 110 mg, 120 mg, 130 mg, 140 mg, 150 mg, 160 mg, 170 mg, 180 mg, 190 mg, 200 mg, 250 mg, 300 mg, 150 mg, 400 mg, 450 mg, 500 mg, or 600 mg of the NK-1 receptor antagonist

In some embodiments, the emulsion comprises about 0.002 wt/wt% to 0.2 wt/wt%, 0.003 wt/wt% to 0.16 wt/wt%, 0.02 wt/wt% to 0.1 wt/wt% dexamethasone sodium phosphate.

In some embodiments, the composition is a stable system maintaining an intensity-weighted mean particle size as determined by dynamic light scattering (DLS) of about 50 nm to 1000 nm, 50 to 500 nm, 50 nm to 400 nm, 50 nm to 300 nm, 50 nm to 200 nm or 50 nm to 100 nm. In another embodiment, the average droplet size is maintained below 500 nm for a period of at least 1 month, 3 months, 6 months, 9 months, 12 months, 2 years or 3 years at room temperature. In other embodiments, the average droplet size is maintained below 500 nm for a period of at least 1 month, 3 months, 6 months, 9 months, 12 months, 2 years or 3 years at 5 °C.

In some embodiments, the NK-1 receptor antagonist is aprepitant. In some embodiments, the NK-1 receptor antagonist is selected from aprepitant, rolapitant, netupitant, lanepitant, vestipitant, orvepitant maleate, casopitant, ezlopitant, serlopitant, befetupitant and maropitant, or a pharmaceutically acceptable salt thereof.

In some embodiments, the NK-1 receptor antagonist is selected from the group consisting of rolapitant, netupitant, casopitant, ezlopitant, vestipitant, serlopitant, maropitant, and orvepitant.

### 2. Pharmaceutical Compositions Comprising Local Anesthetic Without NK-1 Receptor Antagonist

In the method disclosed herein, pain medication is administered in the form of a pharmaceutical composition further comprising a pharmaceutically acceptable excipient. In some embodiments, the pain medication is in the form of a pharmaceutical composition which also comprises the NK-1 receptor antagonist. For example, the method comprises admistering a composition comprising a local anesthetic, NSAID, and NK-1 receptor antagonist in an extended release formulation to the surgical site.

In some embodiments, the pain medication is in a pharmaceutical composition that is separate from the composition comprising the NK-1 receptor antagonist. In some embodiments, the pain medication is in a pharmaceutical composition that is separate from the composition comprising the NK-1 receptor antagonist, wherein the pharmaceutical composition comprises a polyorthester, an organic acid and a pain medication, such as a local anesthetic comprising an amino group, such as the pharmaceutical composition disclosed in U.S. Patent Application No. 14/691,491.

In some embodiments, the pain medication comprising an amino group is a caine type anesthetic. In some embodiments, the caine type anesthetic is selected from bupivacaine, ropivacaine, levobupivacaine, dibucaine, mepivacaine, procaine, lidocaine, and tetracaine. In some embodiments, the pain medication is bupivacaine or ropivacaine.

The concentration of the caine type anesthetic in the composition may vary from about 1 wt% to 30 wt%, or from about 1 wt% to 10 wt%, or from about 10 wt% to 20 wt%, or from about 2 wt% to 5 wt%, or from about 10 wt% to 15%, or from about 15 wt% to 20wt%.

In one or more embodiments related to the foregoing, the caine type anethetic, is added to the composition in its free base form (i.e., is not in the form of an ammonium salt).

In yet some additional embodiments, the organic acid is a C2 - C12 carboxylic acid. In some other embodiments, the organic acid is a C2-C12 dicarboxylic acid. In some other embodiments, the organic acid is a C2-C8 carboxylic acid. In some other embodiments, the organic acid is a C2-C8 dicarboxylic acid.

In yet some further embodiments, the organic acid is selected from the group consisting of oxalic acid, malonic acid, succinic acid, glutaric acid, adipic acid, pimelic acid, fumaric acid, maleic acid, citric acid, acetylsalicylic acid and salicylic acid.

In one or more embodiments, the organic acid is comprised within the formulation in an amount less than an equimolar amount relative to the caine type anesthetic, such that the caine type anesthetic is present as a mixed salt-base. In some particular embodiments, the composition comprises from about 1 - 95 mole percent of the organic acid relative to the caine type anesthetic.

In certain particular embodiments, the organic acid is a mono-carboxylic acid.

In one or more related embodiments, the composition comprises from about 10-80 mole percent of a mono-carboxylic acid relative to the caine type anesthetic.

In one or more additional embodiments, the organic acid is a mono-carboxylic acid or a di-carboxylic acid.

In one or more additional embodiments, the organic acid is a di-carboxylic acid or a tricarboxylic acid.

In some additional embodiments, the composition comprises from about 10-40 mole percent of a di-carboxylic acid relative to the caine type anesthetic.

In some embodiments, the organic acid is a C2-C8, aliphatic, unsubstituted or substituted, saturated straight-chain di-carboxylic acid. In certain related embodiments, the di-carboxylic acid is selected from the group consisting of oxalic acid, malonic acid, succinic acid, glutaric acid, adipic acid, and pimelic acid.

In yet some further embodiments, the organic acid is a C2-C8 aliphatic, unsubstituted or substituted, di-carboxylic acid containing one or more elements of unsaturation (e.g., one or more double or triple bonds). In one or more particular embodiments, the organic acid is either fumaric acid or maleic acid.

In yet one or more further embodiments, the organic acid is a C2-C8 tricarboxylic acid such as the hydroxy-substituted tricarboxylic acid, citric acid.

In yet a further embodiment, the organic acid is an aromatic carboxylic acid. In a particular embodiment, the organic acid is benzoic acid or a substituted benzoic acid having, e.g., from 7 to 14 carbon atoms. Examples include acetylsalicylic acid and salicylic acid.

In yet another one or more embodiments, the organic acid is maleic acid.

In some embodiments, the composition is a semi-solid.

In some embodiments, the polyorthoester is represented by the structure shown as Formula I, 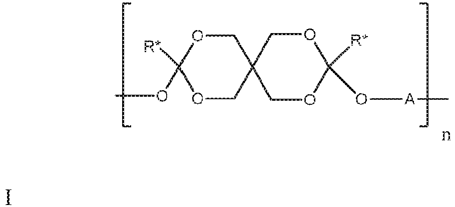 where: R* is a methyl, ethyl, propyl or butyl, n is the number of repeating units and is an integer ranging from 5 to 400, and A in each subunit is R¹ or R³.

In some embodiments directed to Formula I, R* is ethyl.

In yet some additional embodiments directed to Formula III, A corresponds to R¹, where R¹ is 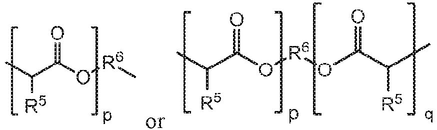 where p and q are each independently integers ranging from about 1 to 20, each R⁵ is independently hydrogen or C₁₋₄ alkyl; and R⁶ is: 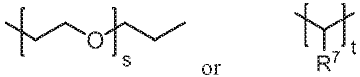 where s is an integer from 0 to 10; t is an integer from 2 to 30; and R⁷ is hydrogen or C₁₋₄ alkyl.

In some other embodiments related to Formula I, R7 is C1, C2, C3, or C4 alkyl. In some particular embodiments, R⁷ is H.

In yet still other embodiments, the R¹ subunits are α-hydroxy acid-containing subunits.

In yet other embodiments, p and q are each independently selected from 1, 2, 3, 4, 5, 6, 7, 8, 9, 10, 11, 12, 13, 14, 15, 16, 17, 18, 19, and 20.

In yet another embodiment, R5 is independently hydrogen, or C1, C2, C3, or C4 alkyl.

In some embodiments, A corresponds to R³, where R³ is: 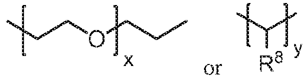 and x is an integer ranging from 1 to 100. In another embodiment, x is selected from 0, 1, 2, 3, 4, and 5; y is an integer in a range from 2 to 30; and R⁸ is hydrogen or C₁₋₄ alkyl. In still another embodiment, R⁸ is a C1, C2, C3 or C4 alkyl. In another embodiment, R⁸ is H.

In some embodiments, the polyorthoester is one of Formula I, II, III or IV, and in particular of Formula I, in which A is R¹ or R³, where R¹ is 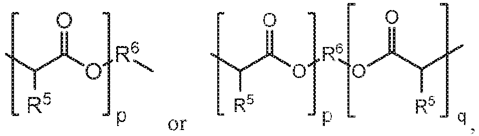 where p and q are each independently selected from 1, 2, 3, 4, 5, 6, 7, 8, 9, 10, 11, 12, 13, 14, 15, 16, 17, 18, 19, and 20 in any repeating unit, where the average number of p or the average number of the sum of p and q (p + q) is between about 1 and 7; x and s are each independently an integer ranging from 0 to 10; and t and y are each independently an integer ranging from 2 to 30. In yet additional embodiments, the sum of p and q is 1, 2, 3, 4, 5, 6 or 7 in any repeating unit of R¹. In yet some further embodiments, R⁵ is H.

In yet further embodiments, A is R¹ or R³, where R¹ is 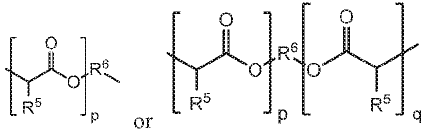 and p and q are each independently integers ranging from about 1 and 20, about 1 and 15, or about 1 and 10 in any repeating unit of R¹, where the average number of p or the average number of the sum of p and q (i.e., p + q) is between about 1 and 7. In another one or more embodiments, x and *s* each independently range from 0 to about 7 or from 1 to about 5. In still another embodiment, t and y each independently range from 2 to 10.

In one embodiment, R⁵ is hydrogen or methyl.

In one embodiment, s and x are each independently selected from 1, 2, 3, 4, 5, 6, 7 and 8. In some particular embodiments, s is 2. In still yet further embodiments, x is 2.

In one embodiment, the polyorthoester comprises alternating residues of 3,9-diethyl-3,9-2,4,8,10-tetraoxaspiro[5.5]undecane-3,9-diyl and A : 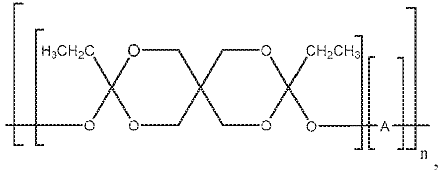 where A is as described above.

In yet one or more additional embodiments, the composition comprising a polyorthoester, an organic acid, and a caine type anesthetic further comprises a solvent. The solvent may be either protic or aprotic in nature.

In yet one or more further embodiments, the caine type anesthetic is solubilized in the composition.

In some embodiments, the composition comprises a polyorthoester, maleic acid, and a caine type anesthetic.

In some embodiments, the pain medication is in the form of a pharmaceutical composition that is separate from the composition comprising the NK-1 receptor antagonist, wherein the pharmaceutical composition comprises a delivery vehicle and a local anesthetic and NSAID as the pain medication, such as the pharmaceutical composition disclosed in U.S. Patent Application No. 15/331,767.

In some embodiments, the local anesthetic is a caine type local anesthetic. In some embodiments, the caine type type local anesthetic is selected from bupivacaine, ropivacaine, levobupivacaine, dibucaine, mepivacaine, procaine, lidocaine, and tetracaine. In some embodiments, the caine type local anesthetic is bupivacone or ropivacine.

In some embodiments, the ratio of amide-type local anesthetic: NSAID ranges from about 10:1 to 50:1, 15:1 to 50:1, 20:1 to 50:1, 25:1 to 50:1, 15:1 to 45:1, 20:1 to 45:1, 25:1 to 45:1, 30:1 to 45:1, 20:1 to 30:1, 20:1 to 35:1, 20:1 to 40:1, or 30:1 to 35:1. In another embodiment, the ratio of amide-type local anesthetic: NSAID is about 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1,44:1, 45:1, 46:1, 47:1, 48:1, 49:1, 50:1, 51:1, 52:1, 53:1, 54:1, 55:1, 56:1, 57:1, 58:1, 59:1, or 60:1.

In some embodiments, the composition comprises bupivacaine and meloxicam, wherein the ratio of bupivacaine:meloxicam ranges from about 10:1 to 50:1, 15:1 to 50:1, 20:1 to 50:1, 25:1 to 50:1, 15:1 to 45:1, 20:1 to 45:1, 25:1 to 45:1, 30:1 to 45:1, 20:1 to 30:1, 20:1 to 35:1, 20:1 to 40:1, or 30:1 to 35:1.. In another embodiment, the ratio of bupivacaine:meloxicam is about 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1,44:1, 45:1, 46:1, 47:1, 48:1, 49:1, 50:1, 51:1, 52:1, 53:1,54:1, 55:1, 56:1, 57:1, 58:1, 59:1, or 60:1.

In some embodiments, the composition comprises ropivacaine and meloxicam.

In some embodiments, the composition comprises ropivacaine and meloxicam, wherein the ratio of ropivacaine:meloxicam ranges from about 10:1 to 50:1, 15:1 to 50:1, 20:1 to 50:1, 25:1 to 50:1, 15:1 to 45:1, 20:1 to 45:1, 25:1 to 45:1, 30:1 to 45:1, 20:1 to 30:1, 20:1 to 35:1, 20:1 to 40:1, or 30:1 to 35:1.. In another embodiment, the ratio of ropivacaine:meloxicam is about1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1,44:1, 45:1, 46:1, 47:1, 48:1, 49:1, 50:1, 51:1, 32:1, 53:1,54:1, 55:1, 56:1, 57:1, 58:1, 59:1, or 60:1.

In some embodiments, the composition comprises the caine type local anesthetic in an amount of about 0.1 to 8 wt%, 1 to 7 wt%, 1 to 6 wt%, 1 to 5 wt%, or 1 to 4 wt%, 1 to 3 wt%, 1.5 to 5 wt%, 1.5 to 3.5 wt%, 1.5 to 3.0 wt%, 2 to 3 wt%, 2.1 to 2.9 wt%, 2.2 to 2.8 wt%, 2.3 to 2.7 wt%, 2.4 to 2.6 wt%, 2.25 to 2.75, 0.25 to 0.75 wt%, 0.25 to 2.5 wt%, 0.25 to 3.5 wt%, or 0.25 to 5 wt% by weight of the composition. In another embodiment, the composition further comprises an NSAID, preferably meloxicam, wherein the meloxicam is present in an amount ranging from about 0.005 to 0.006 wt%, 0.005 to 0.007 wt%, 0.005 to 0.008 wt%, 0.005 to 0.009 wt%, 0.005 to 1 wt%, 0.005 to 0.75 wt%, 0.005 to 0.5 wt%, 0.005 to 0.25 wt%, 0.005 to 0.125 wt%, 0.01 to 1 wt%, 0.01 to 0.75 wt%, 0.01 to 0.5 wt%, 0.01 to 0.25 wt%, 0.01 to 0.125 wt%, 0.020 to 0.100 wt%, 0.030 to 0.100 wt%, 0.040 to 0.100 wt%, 0.050 to 0.090 wt%, 0.060 to 0.080 wt%, 0.070 to 0.080 wt% by weight of the composition.

In some embodiments, the composition is an aqueous based solution.

In some embodiments, the composition is injectable.

In some embodiments, the composition is suitable for administration as an intramuscular injection, transdermally, topically, as a subcutaneous injection, as a perineural injection or to a wound.

In some embodiments, the delivery vehicle is a sustained-release delivery vehicle.

In some embodiments, the sustained-release delivery vehicle is a polymeric composition, a liposomal composition, a microsphere composition, a non-polymeric composition or an implantable device.

In some embodiments, the sustained release delivery vehicle is not a microsphere composition.

In some embodiments the sustained release delivery vehicle is not a liposomal composition.

In some embodiments the sustained release delivery vehicle is not a non-polymeric composition.

In some embodiments the sustained release delivery vehicle is not an implantable device.

In some embodiments, the sustained-release delivery vehicle is not a polymeric composition.

In some embodiments, the delivery vehicle is not a sustained-release vehicle. In another embodiment, the delivery vehicle is an immediate-release vehicle. In still another embodiment, at least about 80% of the anesthetic and the NSAID are released from the delivery vehicle within a time period of about 15 minutes, 30 minutes, 45 minutes, 60 minutes, 90 minutes or 2 hours. In yet another embodiment, the release of the anesthetic and the NSAID is determined in an in vitro dissolution test at 37 °C.

In some embodiments, the composition has a viscosity of less than 10,000 mPa-s when viscosity is measured at 37 °C using a viscometer. In another embodiment, the composition has a viscosity of less than 5,000 mPa-s when viscosity if measured at 37 °C using a viscometer. In yet another embodiment, the composition has a viscosity ranging from about 2500 mPa-s to 10,000 mPa-s when measured at 37 °C using a viscometer. In some embodiments, the viscometer is a cone and plate vi scometer.

In yet another embodiment, the sustained-release delivery vehicle is a liposome selected from the group consisting of small unilamellar vesicles (SUV), large unilamellar vesicles (LUV), multilamellar vesicles (MLV) and multivesicular liposomes (MVL).

In some embodiments, the amide-type local anesthetic is entrapped in an aqueous space of the liposome or in a lipid layer of the liposome.

In some embodiments, the non-steroidal anti-inflammatory drug (NSAID) is entrapped in an aqueous space of the liposome or in a lipid layer of the liposome.

In still another embodiment, the sustained-release delivery vehicle is a microsphere comprised of a bioerodible or biodegradable polymer.

In some embodiments, the amide-type local anesthetic and the non-steroidal anti-inflammatory drug (NSAID) are entrapped in the microsphere.

In some embodiments, the implantable device is an osmotic pump with a reservoir comprising the amide-type local anesthetic and the non-steroidal anti-inflammatory drug (NSAID).

In some embodiments, the sustained-release delivery vehicle is a non-polymeric formulation comprising sucrose acetate isobutyrate.

In some embodiments, the sustained-release delivery vehicle is a polymeric formulation in the form of a semi-solid polymer formulation comprising a polymer, the caine type local anesthetic and the non-steroidal anti-inflammatory drug (NSAID).

In some embodiments, the polymer is a bioerodible or biodegradable polymer.

In yet another embodiment, the polymer formulation forms an implant or depot in situ.

In still another embodiment, the polymer is selected from the group consisting of polylactides, polyglycolides, poly(lactic-co-glycolic acid) copolymers, polycaprolactones, poly-3-hydroxybutyrates, and polyorthoesters.

In a further embodiment, the sustained-release delivery vehicle is a polymeric formulation in the form of a semi-solid polymer formulation comprising a polyorthoester, the caine type local anesthetic and the non-steroidal anti-inflammatory drug (NSAID).

In some embodiments, the caine type type local anesthetic is selected from the group consisting of bupivacaine, ropivacaine, levobupivacaine, dibucaine, mepivacaine, procaine, lidocaine, and tetracaine. In another embodiment, the amide-type local anesthetic is selected from bupivacaine and ropivacaine.

In some embodiments, the amide-type local anesthetic is ropivacaine.

In some embodiments, the amide-type local anesthetic is bupivacaine.

In some embodiments related to any one or more of the foregoing embodiments, the non-steroidal anti-inflammatory drug (NSAID) is an enolic-acid NSAID. Exemplary enolic-acid NSAIDs include meloxicam, piroxicam, tenoxicam, droxicam, lornoxicam, and isoxicam.

In some embodiments, the enolic-acid NSAID is meloxicam.

In some embodiments related to any one or more of the foregoing embodiments, the composition is a semi-solid or solid composition.

In some embodiments, the delivery vehicle is a sustained-release delivery vehicle. In some embodiments, the sustained-release vehicle is a polymeric vehicle or formulation.

In another embodiment, the sustained-release polymeric vehicle is a solid or semi-solid vehicle comprising a bioerodible or biodegradable polymer.

In an embodiment, the biodegradable or bioerodible polymeric formulation comprises a polymer selected from the group consisting of polylactide, polyglycolide, a poly(lactic-co-glycolic acid) copolymer, polycaprolactone, poly-3-hydroxybutyrate, or a polyorthoester.

In some embodiments, the polyorthoester is selected from the polyorthoesters represented by Formula III set forth herein.

In an alternative embodiment, the sustained-release vehicle does not comprise a polylactide, polyglycolide, a poly(lactic-co-glycolic acid) copolymer, polycaprolactone, poly-3-hydroxybutyrate, or a polyorthoester. In another embodiment, the sustained-release vehicle does not comprise a polyorthoester. In still another embodiment, the sustained-release vehicle does not comprise a polyorthoester represented by Formula I.

In yet an additional embodiment, the composition or delivery vehicle further comprises a solvent. The solvent may be either protic or aprotic in nature. In some embodiments, the composition comprises as the delivery vehicle a polyorthoester and a solvent.

In another embodiment, the sustained-release delivery vehicle is selected from the group consisting of microspheres, microparticles, and homogeneous or heterogeneous matrix depots. In some embodiments, the microsphere, microparticle or depot vehicle is biodegradable or bioerodible.

In another embodiment, the sustained-release delivery vehicle is a liposomal formulation or a lipid-based formulation.

In another embodiment, the sustained-release formulation is a polymeric-based solid or semi-solid implant where the caine type local anesthetic and the enolic-acid NSAID are dispersed in the polymeric-based implant. In some embodiments, the implant is a solid polymeric-based vehicle in the form of a suture or a staple.

A post-operative model was employed to study the pain relief profiles provided by the method described herein. The data unexpectedly shows that surprisingly enhanced pain relief can be obtained when both the NK-lantagonist and pain medication are administered after the surgery.

The method disclosed herein comprises administering to a patient in need of post-surgical pain treatment, (1) a composition comprising NK-1 receptor antagonist as disclosed herein before surgery and daily thereafter for 1-6 days and (2) a composition comprises a local anesthetic (such as bupivacaine or ropivaciane) and NSAID (such as meloxicam) prior to, during or at the end of surgery. In some embodiments, the composition comprising NK-1 receptor antagonist is administered systemically such as orally or by IV. In some embedments, the composition comprises a local anesthetic and NSAID is in the form of an extended release as disclosed herein. In some embodiments, the extended release composition comprises a local anesthetic and NSAID is administered into the surgical site. In some emboidments, the NK-1 receptor antagonist is aprepitant. In some emboidments, the local anesthetic is bupivacaine. In some embodiments, the NSAID is meloxicam.

The method disclosed herein comprises administering to a patient in need of post-surgical pain treatment a composition comprising NK-1 receptor antagonist, a NSAID, a local anesthetic, and a delivery vehicle as disclosed herein. The administration is conducted prior to, during or at the end of surgery. The method may further comprises administration of a compostion comprising NK-1 receptor antagonist daily after surgery for 1-6 days. In some embodiments, the composition comprises an NK-1 receptor antagonist, a NSAID, and a local anesthetic, is in the form of an extended release composition as disclosed herein. In some embodiments, the extended release composition comprising NK-1 receptor antagonist, a NSAID, a local anesthetic is administered into the surgical site. In some embodiments, the NK-1 receptor antagonist is aprepitant. In some embodiments, the local anesthetic is bupivacaine. In some embodiments, the NSAID is meloxicam.

The method disclosed herein comprises administering to a patient in need of post-surgical pain treatment, (1) NK-1 receptor antagonist and a NSAID as disclosed herein before surgery and daily thereafter for 1-6 days orally, IV, subcutaneously or other means to provide systemic exposure and (2) a composition comprising a local anesthetic prior to, or during or at the end of surgery . In some embodiemnts, the NK-1 receptor antagonist and the NSAID are provided in a single compostion. In some embodiemnts, the NK-1 receptor antagonist and the NSAID are provided in separate compostions. In some embodiment, the composition comprising a local anesthetic is in the form of an extended release. In some embodiments, the extended release composition comprising the local anesthetic is administered into the surgical site. In some embodiments, the composition comprises an NK-1 receptor antagonist and NSAID is in the form of an extended release compostion as disclosed herein. In some embodiments, the NK-1 receptor antagonist is aprepitant. In some embodiments, the local anesthetic is bupivacaine. In some embodiments, the NSAID is meloxicam.

Example 1 describes three studies conducted to evaluate the capacity to reduce post-surgical incisional (post-operative or POP) pain in a porcine model system. Using a post-operative (POP) pain porcine model system, where a 7 cm long skin and fascia incision was made in the left flank under general anesthesia to pigs, NK-1 receptor antagonist alone or in combination with pain medication (i.e., bupivacaine and meloxicam) were applied to the wound. The skin incision was then closed using sterile sutures. Post-operative pain was assessed using the von Frey methodology with results shown in FIG.1.

FIG.1 is a bar graph of withdrawal force, in grain force, as a function of time, in hours, after surgery, measured for *in vivo* administrations to pigs of a composition comprised of aprepitant once prior to surgery and daily for 5 days after surgery (Group 1, diagonal line fill) or further in combination with administration of a composition comprising bupivacaine and meloxicam once during surgery (Group 2, dotted fill) or for *in vivo* administrations to pigs of a composition comprised of aprepitant once prior to surgery in combination with administration of a composition comprising bupivacaine and meloxicam once during surgery (Group 3, vertical line fill). The efficacy of the use of aprepitant in combination with bupicacaine and meloxicam were longer lasting and provided deeper analgesic effect than did aprepitant alone.

Example 2 describes studies conducted to evaluate analgesic effect of (1) co-adminsitration of apprepitant with a bupivacaine composition (2) co-adminsitration of apprepitant with with a meloxicam composition, and (3) co-administration of aprepitant at a dose lower than that of Example 1 (3.0 mg/kg in Example 2 vs. 6.0 mg/kg in Example 1) with a composition comprising both bupivacaine and meloxicam. The studies used the porcine model system as described in Example 1. Post-operative pain was assessed using the von Frey methodology with results shown in FIG.2.

FIG. 2 shows that (1) co-adminsitration of apprepitant with meloxicam, e.g., 6.0 mg/kg apprepitant by slow IV push 30 minutes prior to surgery and daily thereafter for 5 days following von Frey fiber evaluation and 4.0 g of an extended release formulation of meloxicam into the margins of the surgical incision prior to suturing, had no analgesic effect (Group 4), (2) co-adminsitration of apprepitant with bupivacaine, e.g., 6.0 mg/kg apprepitant by slow IV push 30 minutes prior to surgery and daily thereafter for 5 days following von Frey fiber evaluation and 4.0 g of an extended release formuation of bupivacaine into the margins of the surgical incision prior to suturing, only had the analgesic activity consistent with bupivacaine alone (Group 3), and (3) co-administration of aprepitant at a lower dose with a composition comprising both bupivacaine and meloxicam, e.g., 3.0 mg/kg aprepitant by slow IV push 30 minutes prior to surgery and daily thereafter for 5 days following von Frey fiber evaluation and 4.0 g of the composition comprising bupivacaine and meloxicam, administered to the wound margins prior to suturing, maintained good analgesic effect for 5 treatment days (Group 5).

Example 3 describes studies conducted to evaluate analgesic effect of an even lower dosing of aprepitant (e.g., 1.5 mg/kg) and of less frequent administration of aprepitant. The studies used the porcine model system as described in Example 1. Post-operative pain was assessed using the von Frey methodology with results shown in FIG.3.

FIG. 3 is a bar graph of withdrawal force, in gram force, as a function of time, in hours, after administration *in vivo* to animals of a composition comprised of the test compositions denoted as follows: (i) IV aprepitant dosed once prior to surgery and daily for 5 days after surgery at a dose of 1.5 mg/kg and an extended release formulation of bupivacaine and meloxicam dosed 3.4 mL once during surgery by injection into the wound margins (Group 6, bars with solid fill), (ii) ) IV aprepitant dosed once prior to surgery and once on days 2 and 3 after surgery at a dose of 6.0 mg/kg and an extended release formulation of bupivacaine and meloxicam dosed 3.4 mL once during surgery by injection into the wound margins (Group 7, bars with diagonal line fill), (iii) IV aprepitant dosed once prior to surgery and once on days 3, 4, and 5 after surgery at a dose of 6.0 mg/kg and an extended release formulation of bupivacaine and meloxicam dosed 3.4 mL once during surgery by injection into the wound margins (Group 8, bars with horizontal line fill); and (iv) oral suspension of meloxicam dosed once at 0.5 mg/kg prior to surgery and IV aprepitant dosed once prior to surgery and once daily for 5 days after surgery at a dose of 6.0 mg/kg and an extended release formulation of bupivacaine dosed 3.4 mL once during surgery by injection into the wound margins (Group 9, bars with dotted fill). When aprepitant was dosed at 1.5 mg/kg, good analgesi effect was observed for 3 treatment days but dimimished significantly on days 4 and 5. Group 7 animals that were dosed on days 1-3 show enhancement of analgesia followed by, a somewhat unexpected, decline in analgesia on Day 4. Group 8 animals, where dosing was initiated on Day 3, show no enhancement of analgesia. Therefore, it appears that the dosing of CINVANTI must be started prior to surgery and must be dosed daily thereafter.

Example 4 evaluated the effect of bupivacaine, meloxicam and aprepitant formulated into extended release formulations containing all three active agents for administration into the surgical incision. The studies used the porcine model system as described in Example 1. Post-operative pain was assessed using the von Frey methodology with results shown in FIG.5.

FIG. 5 is a bar graph of withdrawal force, in gram force, as a function of time, in hours, after administration *in vivo* to animals of two compositions comprised of a combination of bupivacaine, meloxicam with one formulated with a high concentration of aprepitant and one with a low concentration of aprepitant. Both combination formulations of bupivacaine, meloxicam, aprepitant administered as a single wound site admistration during surgery provided nearly complete analgesia for the duration of the study.

FIG. 4 compares the analgesic effect of aprepitant dosed at 1.5, 3.0, 6.0 mg/kg based on data from Examples 1-3. These results demonstrate a dose response with respect to apprepitant with the dose of 3mg/kg providing enhanced analgesia for the duration of apprepitant dosing and the 1.5 mg/kg providing enhancement of analgesia during the 3-day period that the composition comprising bupivacaine and meloxicam is normally providing analgesia.

Example 5 evaluted various combinations of bupivacaine, meloxicam, and aprepitant. Extended release formulations of meloxicam and aprepitant, aprepitant alone, bupviaciane, a low concentration meloxicam and aprepitant, and bupivacaine and aprepitant were compared to an extended release formuation of bupivacaine, meloxicam, and aprepitant. None of the tested combination formulations demonstrated analgesia comparable to the combination of bupivacaine, meloxicam, and aprepitant.

FIG. 6 is a bar graph comparing the analgesic effect of varioius combinations of bupivacaine, meloxicam and aprepitant described in Example 5. The combination formulation of bupivacaine, meloxicam, and aprepitant (Group 12, solid fill bars) administered as a single wound site admistration during surgery provided excellent analgesia for the duration of the study. In contrast, the formulations of bupivacaine and aprepitant (Group 13, bars with horizontal lines), bupivacaine, a low concentration meloxicam, and aprepitant (group 14, bars with checkerboard), aprepitant alone (Group 15, bars with diagonal lines), and meloxicam and aprepitant (Group 16, bars with dotted fill) provided modest analgesia during the first 24 hours for bupivacaine containing formulations and almost no analgesia from 24 hours to 114 hours.

### EXAMPLES

The following examples are illustrative in nature and are in no way intended to be limiting.

### Example 1

### Post-Surgical Pain Reduction Comparison

Various methods were evaluated for their capacity to reduce post-surgical incisional (post-operative or POP) pain in a porcine model system. In this model, a 7 cm long skin and fascia incision was made in the left flank under general anesthesia. Aprepitant and/or bupivacaine and meloxicam were applied to the wound. The skin incision was then closed using sterile sutures. All studies described below evaluated 4 pigs per group.

Post-operative pain was assessed using the von Frey methodology. Von Frey filaments (Ugo Basile) were applied at approximately ~0.5 cm proximal to the incision line to the surface of the flank skin. Filaments of increasing diameter (thicker fibers equate to a higher gram force while thinner fibers equate to a lower gram force) were applied until the animal withdrew from the stimuli (the act of moving away from the stimuli). Each filament was applied 3-5 times. If withdrawal was not achieved, a thicker filament was applied. The maximum force filament was 60 g. If a withdrawal was achieved, a thinner filament was applied. By alternating the filament thickness, the gram force required to achieve withdrawal reaction was determined and recorded. The greater the force that was applied, the more effective the analgesia.

Several studies evaluated emulsion compositions containing aprepitant (diluted 1:7) with saline, administered by intravenous infusion dosed at 6 mg/kg as a slow bolus via an IV catheter in the ear. In one study 8 piglets divided into 2 groups of 4 piglets, received one dose of aprepitant prior to surgery followed by daily dosing thereafter for 5 days. One group of 4 recieved no further study medication and 1 group of 4 also received a single administration of an extended release combination of bupivacaine and meloxicam into the wound site during surgery. In a second study 4 piglets received a single dose of aprepitant prior to surgery and also received a single administration of an extended release combination of bupivacaine and meloxicam into the wound site during surgery.

The extended release bupivacaine and meloxicam was administered following creation of an incision. Each test composition was administered by injecting subcutaneously into the lateral margins of the wound. Table 1 summaries the test groups and compositions. Analgesia was evaluated by response in the von Frey test, as described above. The baseline (pre-surgery) withdrawal score for the von Frey test was 60 g.

**Table 1:**

| **Group** | **Administration of NK-1** | **Administration of Extended Release Bupivacaine and Meloxicam** |
|---|---|---|
| **1** | 6 mg/kg, slow bolus Prior to surgery and daily for 5 days thereafter | None |
| **2** | 6 mg/kg, slow bolus Prior to surgery and daily for 5 days thereafter | 3.4 mL by subcutaneous Injection Around Wound |
| **3** | 6 mg/kg, slow bolus Prior to surgery only | 2.6 mL by subcutaneous Injection Around Wound |

The mean von Frey response for the animals in each test group is shown in Figure 1, where withdrawal force, in gram force is shown as a function of time, in hours, after administration *in vivo* to pigs. The test compositions are denoted as follows: (i) IV aprepitant dosed once prior to surgery and daily for 5 days after surgery at a dose of 6mg/kg (Group 1, bars diagonal line fill ); (ii) IV aprepitant dosed once prior to surgery and daily for 5 days after surgery at a dose of 6mg/kg and an extended release formulation of bupivacaine and meloxicam dosed once during surgery by injection into the wound margins (Group 2, bars dotted fill); (iii) IV aprepitant dosed once 30 minutes before surgery at a dose of 6mg/kg and an extended release formulation of bupivacaine and meloxicam dosed once during surgery by injection into the wound margins (Group 3, bars vertical line fill). Compositions tested are presented in the Tables 2 and 3 below.

**Table 2: Composition comprising aprepitant (CINVANTI^{®})**

| Component | Aprepitant | Egg Lecithin, Lipoid E 80 | Soybean Oil | Ethanol | Sucrose | Sodium Oleate | Water for Injection |
|---|---|---|---|---|---|---|---|
| Concentration (% w/w) | 0.716 | 14.31 | 9.54 | 2.82 | 5.34 | 0.48 | 66.79 |

**Table 3: Composition comprising bupivacaine and meloxicam**

| Component | Bupivacaine | Meloxicam | Maleic acid | POE | DMSO | Triacetin |
|---|---|---|---|---|---|---|
| Concentration (% w/w) | 2.5 | 0.075 | 0.05 | 62.38 | 10 | 25 |

There was little analgesic response for animals treated daily with IV aprepitant. In contrast the combination treatement of daily IV aprepitant and a single wound site admistration of extended release bupivacaine and meloxicam during surgery provided nearly complete analgesia for the duration of the study. The group dosed with aprepitant only prior to surgery and a single wound site admistration of extended release bupivacaine and meloxicam during surgery demonstrated very good analgesia on Day 1 but lesser analgesia at 24 hours and beyond.

### Example 2

### Post-Surgical Pain Reduction Using Lower Dose of Apprepitant

By following similar procedures described in Example 1, the analgesic effect of a lower dose of aprepitant (i.e., lower than 6.0 mg/kg) was evaluated. The analgesic effect of co-administration of aprepitant with an extended release formulation of meloxicam or an extended release formulation of bupivacaine was also evaluated.

Table 4 summaries the test groups and compositions. Analgesia was evaluated by response in the von Frey test, as described above. The baseline (pre-surgery) withdrawal score for the von Frey test was 60 g.

**Table 4:**

| **Group No.** | **No. of Animals** | **Treatment** | **Dosing*** | **Dosing Location** |
|---|---|---|---|---|
| 3 | 4 | Extended release bupivacaine composition | 3.4 mL (4.0 g) | 4 injections on each side of the wound (8 total) |
| | | Apprepitant composition | 6.0 mg/kg | IV - once prior to surgery and daily for 5 days after surgery |
| 4 | 4 | Extended release meloxicam composition | 3.4 mL (4.0 g) | 4 injections on each side of the wound (8 total) |
| | | Apprepitant composition | 6.0 mg/kg | IV - once prior to surgery and daily for 5 days after surgery |
| 5 | 4 | Extended release bupivacaine+meloxicam composition | 3.4 mL (4.0 g) | 4 injections on each side of the wound (8 total) |
| | | Apprepitant composition | 3.0 mg/kg | IV - once prior to surgery and daily for 5 days after surgery |

The apprepitant composition in Table 4 is the same as the composition comprising aprepitant of Table 2. The bupivacaine+meloxicam composition in Table 4 is the same composition comprising bupivacaine and meloxicam of Table 3. The bupivacaine composition and the meloxicam composition in Table 4 are presented in Table 5 and 6, respectively:

**Table 5: Extended release bupivacaine composition**

| Component | Bupivacaine | Maleic acid | POE | DMSO | Triacetin |
|---|---|---|---|---|---|
| Concentration (% w/w) | 2.5 | 0.05 | 62.45 | 10 | 25 |

**Table 6: Extended release meloxicam composition**

| Component | Meloxicam | POE | DMSO | Triacetin |
|---|---|---|---|---|
| Concentration (% w/w) | 0.075 | 64.9 | 10 | 25 |

The mean von Frey response for the animals in each test group is shown in FIG. 2, where withdrawal force, in gram force is shown as a function of time, in hours, after administration *in vivo* to pigs. The test compositions are denoted as follows: (i) IV aprepitant dosed once prior to surgery and daily for 5 days after surgery at a dose of 3.0 mg/kg and an extended release formulation of bupivacaine and meloxicam dosed 3.4 mL once during surgery by injection into the wound margins (Group 5, bars with diagonal line fill), (ii) IV aprepitant dosed once prior to surgery and daily for 5 days after surgery at a dose of 6.0 mg/kg and an extended release formulation of bupivacaine dosed 3.4 mL once during surgery by injection into the wound margins (Group 3, bars with dotted fill), and (iii) IV aprepitant dosed once prior to surgery and daily for 5 days after surgery at a dose of 6.0 mg/kg and an extended release formulation of meloxicam dosed 3.4 mL once during surgery by injection into the wound margins (Group 4, bars with with vertical line fill).

Group 4 showed no analgesic activity. Group 3 showed analgesic activity consistent with bupivacaine composition alone. These results indicate that the analgesic effect of apprepitant were only observed when co-administered with both bupivacaine and meloxicam. Group 5 showed good analgesia for the 5 treatment days but diminished significantly on Days 6 and 7.

### Example 3

### Post-Surgical Pain Reduction With Differing Apprepitant Administration Times

Another study, following similar experimental proceudres describe in Example 1, evaluated the potential analgesic effect of a lower dose of apprepitant than was evaluated in Example 1 and 2. To evaluate if the effect of apprepitant was durable after an extended period of dosing, this study also evaluated the effect of 3 days of apprepitant dosing co-administered with an extended release formulation of bupivacaine and meloxicam. Conversely, this study also evaluated the effect of delaying dosing aprepitant for several days after the surgery and dosing of the extended release formulation of bupivacaine and meloxicam. Finally, oral dosing of meloxicam in combination with local delivery of an extended release formulation of bupivacaine and IV administration of appreitant was evaluated to determine if the effect of locally delivered meloxicam could be replicated by systemically delivered meloxicam.

Group 6 was dosed with 1.5 mg/kg apprepitant by slow IV push 30 minutes prior to surgery and daily thereafter for 5 days following von Frey fiber evaluation and the surgical incision was dosed with 3.4 mL of the extended release formulation of bupivacaine and meloxicam administered to the wound margins prior to suturing.

Groups 7 animals were dosed with 6 mg/kg apprepitant by slow IV push 30 minutes prior to surgery and on days 2 and 3 (24 and 48 h) following von Frey fiber evaluation. Groups 8 animals were dosed with 6 mg/kg apprepitant by slow IV push starting on day 3 with additional dosing on days 4 and 5 (48, 72, and 96 h) following von Frey fiber evaluation. Animals in both groups were also dosed with 3.4 mL of the extended release formulation of bupivacaine and meloxicam into the margins of the surgical incision prior to suturing.

Group 9 animals were dosed with 0.5 mg/kg meloxicam (Loxicom oral suspension) and 6 mg/kg aprepitant by slow IV push 30 minutes prior to surgery and daily thereafter for 5 days following von Frey fiber evaluation. Animals in this group were also dosed with 3.4 mL of an extended release formulation of bupivacaine into the margins of the surgical incision prior to suturing.

Table 7 summaries the test groups and compositions. Analgesia. was evaluated by response in the von Frey test, as described above. The baseline (pre-surgery) withdrawal score for the von Frey test was 60 g.

**Table 7:**

| **Group No.** | **No. of Animals** | **Treatment** | **Dosing*** | **Dosing Location** |
|---|---|---|---|---|
| 6 | 4 | Bupivacaine +meloxicam compsition | 3.4 mL (4.0 g) | 4 injections on each side of the wound (8 total) |
| | | Aprepitant composition | 1.5 mg/kg | IV Once Daily on Days 1, 2, 3, 4 ,5 and 6 |
| 7 | 4 | Bupivacaine +meloxicam compsition | 3.4 mL (4.0 g) | 4 injections on each side of the wound (8 total) |
| | | Aprepitant composition | 6.0 mg/kg | IV Once daily on Days 1, 2 and 3 |
| 8 | 4 | Bupivacaine +meloxicam compsition | 3.4. mL (4.0 g) | 4 injections on each side of the wound (8 total) |
| | | Aprepitant composition | 6.0 mg/kg | IV Once daily on Days 3, 4, and 5 |
| 9 | 4 | Bupivacaine composition | 3.4 mL (4.0 g) | 4 injections on each side of the wound (8 total) |
| | | Aprepitant composition | 6.0 mg/kg | IV Once daily on Days 1, 2, 3, 4 ,5 and 6 |
| | | Oral meloxicam (Loxicom oral suspension) | 0.5 mg/kg | Oral Once daily on Days 1, 2, 3, 4 ,5 and 6 |

The apprepitant composition in Table 7 is the same as the composition comprising aprepitant of Table 2. The bupivacaine+meloxicam composition in Table 7 is the same composition comprising bupivacaine and meloxicam of Table 3. The bupivacaine composition in Table 7 is the same as that of Table 5.

The mean von Frey response for the animals in each test group is shown in Figure 3, where withdrawal force, in gram force is shown as a function of time, in hours, after administration *in vivo* to pigs. Group 6 (bars with black fill) animals showed good analgesia for 3 treatment days but diminished significantly on Days 4 and 5.

Comparing the results for Groups 7 (bars with diagonal lines) and 8 (bars with horizontal lines) and focusing on the Day 2 (48 hr timepoint) results, Group 7 animals that were dosed on days 1-3 show enhancement of analgesia followed by, a somewhat unexpected, decline in analgesia on Day 4. Group 8 animals, where dosing was initiated on Day 3, show no enhancement of analgesia. Therefore, it appears that the dosing of aprepitant is best when started prior to surgery and should be dosed daily thereafter.

Group 9 (bars with dotted fill) demonstrate poor analgesia on from 24 through 144 hours. These results indicate that the meloxicam is optimally effective when locally administered. While systemically administered meloxicam may provide analgesia, a high dose (higher than 0.5 mg/kg) may be required.

The results from this study and studies in Example 1 and 2 are presented FIG. 4 for comparison. These results demonstrated a dose response with respect to apprepitant with the dose of 6 mg/kg (Group 2, bars with dotted fill) providing enhanced analgesia for the duration of apprepitant dosing, 3mg/kg (Group 5, bars with diagonal line fill), and the 1.5 mg/kg (Group 6, bars with horizontal lines) providing enhancement of analgesia during the 3-day period that the extended release formulation of bupivacaine and meloxacam is normally providing analgesia. Historical data for extended release combination of bupivacaine and meloxicam (2.5% w/w and 0.075% w/w, respectively) shown in solid filled bars.

### Example 4

### Co-Formulated Extended Release Bupivacaine, Meloxicam and Aprepitant

A study was conducted to evaluate the effect of bupivacaine, meloxicam and aprepitant formulated into extended release formulations containing all three active agents for administration into the surgical incision. Group 10 animals were dosed with 3.4 mL of an extended release formuation containg bupivacaine, meloxicam and a high concentration of aprepitant. Group 11 animals were dosed with 3.4 mL of an extended release formuation containg bupivacaine, meloxicam and a low concentration of aprepitant.

Table 8 summaries the test groups and compositions. Analgesia was evaluated by response in the von Frey test, as described above. The baseline (pre-surgery) withdrawal score for the von Frey test was 60 g.

**Table 8**

| **Group No.** | **No. of Animals** | **Treatment** | **Dosing** | **Dosing Location** |
|---|---|---|---|---|
| 10 | 4 | Bupivacaine + meloxicam + high concentration aprepitant composition | 3.4 mL (4.0 g) | 4 injections on each side of the wound (8 total) |
| 11 | 4 | Bupivacaine + meloxicam + low concentration aprepitant composition | 3.4 mL (4.0 g) | 4 injections on each side of the wound (8 total) |

The compositions are presented in Table 9

**Table 9, Group 10 Bupivacaine, meloxicam, and high concentration aprepitant extended release composition**

| Component | Bupivacaine | Meloxicam | Aprepitant | Maleic acid | POE | DMSO | Triacetin |
|---|---|---|---|---|---|---|---|
| Concentration (% w/w) | 2.5 | 0.075 | 1.6 | 0.05 | 60.775 | 10 | 25 |

**Table 10, Group 11 Bupivacaine, meloxicam, and low concentration aprepitant extended release composition**

| Component | Bupivacaine | Meloxicam | Aprepitant | Maleic acid | POE | DMSO | Triacetin |
|---|---|---|---|---|---|---|---|
| Concentration (% w/w) | 2.5 | 0.075 | 0.5 | 0.05 | 61.875 | 10 | 25 |

The mean von Frey response for the animals in each test group is shown in Figure 5, where withdrawal force, in gram force is shown as a function of time, in hours, after administration *in vivo* to pigs. In both Group 10 and 11 animals, the combination formulations of bupivacaine, meloxicam, and high or low concentration aprepitant administered as a single wound site admistration during surgery provided nearly complete analgesia for the duration of the study. Historical data for extended release combination of bupivacaine and meloxicam (2.5% w/w and 0.075% w/w, respectively) shown in solid filled bars.

### Example 5

### Evaluating the Contribution of Combinations of Bupvacaine, Meloxicam, and Aprepitant

Table 11 summaries the test groups and compositions. Analgesia was evaluated by response in the von Frey test, as described above. The baseline (pre-surgery) withdrawal score for the von Frey test was 60 g.

**Table 11**

| **Group No.** | **No. of Animals** | **Treatment** | **Dosing** | **Dosing Location** |
|---|---|---|---|---|
| 12 | 4 | Bupivacaine + meloxicam + aprepitant composition | 3.4 mL (4.0 g) | 4 injections on each side of the wound (8 total) |
| 13 | 4 | Bupivacaine + aprepitant composition | 3.4 mL (4.0 g) | 4 injections on each side of the wound (8 total) |
| 14 | 4 | Bupivacaine + low concentration meloxicam + aprepitant composition | 3.4 mL (4.0 g) | 4 injections on each side of the wound (8 total) |
| 15 | 4 | Aprepitant composition | 3.4 mL (4.0 g) | 4 injections on each side of the wound (8 total) |
| 16 | 4 | Meloxicam + aprepitant composition | 3.4 mL (4.0 g) | 4 injections on each side of the wound (8 total) |

The compostions are presented in Tables 12 through 16

**Table 12, Group 12 Bupivacaine, meloxicam, and aprepitant extended release composition**

| Component | Bupivacaine | Meloxicam | Aprepitant | Maleic acid | POE | DMSO | Triacetin |
|---|---|---|---|---|---|---|---|
| Concentration (% w/w) | 2.5 | 0.075 | 0.1 | 0.05 | 62.275 | 10 | 25 |

**Table 13, Group 13 Bupivacaine and aprepitant extended release composition**

| Component | Bupivacaine | Meloxicam | Aprepitant | Maleic acid | POE | DMSO | Triacetin |
|---|---|---|---|---|---|---|---|
| **Concentration** (% w/w) | 2.5 | 0 | 1.6 | 0.05 | 60.85 | 10 | 25 |

**Table 14, Group 14 Bupivacaine, low concentration meloxicam, and aprepitant extended release composition**

| Component | Bupivacaine | Meloxicam | Aprepitant | Maleic acid | POE | DMSO | Triacetin |
|---|---|---|---|---|---|---|---|
| Concentration (% w/w) | 2.5 | 0.038 | 1.6 | 0.05 | 60.812 | 10 | 25 |

**Table 15, Group 15 Aprepitant extended release composition**

| Component | Bupivacaine | Meloxicam | Aprepitant | Maleic acid | POE | DMSO | Triacetin |
|---|---|---|---|---|---|---|---|
| **Concentration** (% w/w) | 0 | 0 | 0.5 | 0 | 64.5 | 10 | 25 |

**Table 16, Group 16 Meloxicam and aprepitant extended release composition**

| Component | Bupivacaine | Meloxicam | Aprepitant | Maleic acid | POE | DMSO | Triacetin |
|---|---|---|---|---|---|---|---|
| Concentration (% w/w) | 0 | 0.075 | 0.5 | 0 | 64.425 | 10 | 25 |

The mean von Frey response for the animals in each test group is shown in Figure 6, where withdrawal force, in gram force is shown as a function of time, in hours, after administration *in vivo* to pigs. The combination formulation of bupivacaine, meloxicam, and aprepitant (Group 12, solid black bars) administered as a single wound site admistration during surgery provided excellent analgesia for the duration of the study. In contrast, the formulations of bupivacaine and aprepitant (Group 13, bars with horizontal lines), bupivacaine, a low concentration meloxicam, and aprepitant (group 14, bars with checkerboard), aprepitant alone (Group 15, bars with diagonal lines), and meloxicam and aprepitant (Group 16, bars with dotted fill) provided modest analgesia during the first 24 hours for bupivacaine containing formulations and almost no analgesia from 24 hours to 114 hours.

Additional embodiments of the present compositions and methods and the like, will be apparent from the description, drawings, examples. and claims. As can be appreciated from the foregoing description, each and every feature described herein, and each and every combination of two or more of such features, is included within the scope of the present disclosure provided that the features included in such a combination are not mutually inconsistent. In addition, any feature or combination of features may be specifically excluded from any embodiment of the present invention. While compositions and methods are described in terms of "comprising," "containing," or "including" various components or steps, the compositions and methods can also "consist essentially of" or "consist of" the various components and steps. The term "consisting essentially of" excludes active agents not recited after the term.

## Claims

1. A composition for use in treating post-surgical pain in a patient in need thereof, wherein the composition comprises aprepitant, bupivacaine, and meloxicam.

2. The composition for use of claim 1, wherein the post-surgical pain is not neuropathic.

3. The composition for use of claim 1 or claim 2, which comprises administering the composition during surgery or after surgery.

4. The composition for use of any of claims 1 to 3, wherein the composition is administered in the form of an injectable emulsion comprising:
aprepitant, bupivacaine and meloxicam;
11 wt/wt% to 15 wt/wt% of an emulsion;
an oil;
a co-surfactant which comprises an alcohol;
a tonicity agent;
a pH modifier; and
water;
wherein the pH of the emulsion ranges from about 7.5 to 9.0.

5. The composition for use of any of claims 1 to 4, further comprising a delivery system, optionally wherein bupivacaine and meloxicam are present in the composition at a ratio ranging from about 15:1 to 50:1.

6. A pharmaceutical composition comprising:
(i) aprepitant, bupivacaine and meloxicam; and
(ii) a delivery vehicle, optionally wherein the delivery vehicle comprises a polyorthoester of formula (I): 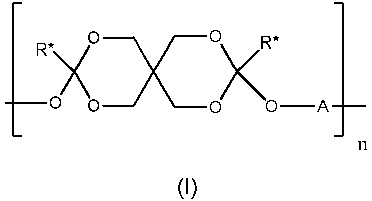
where: R* is a methyl, ethyl, propyl or butyl, n is the number of repeating units and is an integer ranging from 5 to 400, and A in each subunit is R¹ or R³;
R¹ is 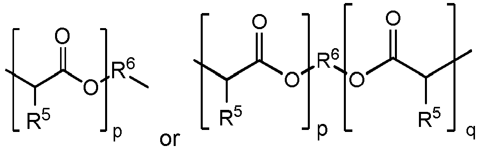
where p and q are each independently integers ranging from about 1 to 20, each R⁵ is independently hydrogen or C₁₋₄ alkyl; and R⁶ is: 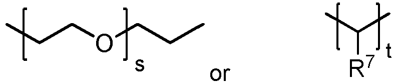
where s is an integer from 0 to 10; t is an integer from 2 to 30; and R⁷ is hydrogen or C₁₋₄ alkyl; R³ is: 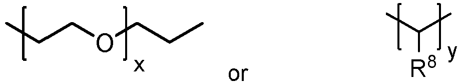
where x is an integer ranging from 1 to 100, y is an integer in a range from 2 to 30 and R⁸ is hydrogen or C₁₋₄ alkyl.

7. The pharmaceutical composition of claim 6, wherein the weight ratio of bupivacaine:aprepitant ranges from 1:1 to 60:1, such as from 1:1 to 5:1.

8. The pharmaceutical composition of claim 6 or claim 7, comprising the polyorthoester of formula (I) as defined in claim 6.

9. The pharmaceutical composition of any of claims 6 to 8, wherein the composition further comprises an aprotic solvent, such as an aprotic solvent selected from DMSO, NMP, and DMAC.

10. The pharmaceutical composition of any of claims 6 to 9 for use in treating post-surgical pain in a patient in need thereof, such as wherein the pharmaceutical composition is administered to the surgical site.

## Patentansprüche

1. Zusammensetzung zur Verwendung bei der Behandlung von postoperativem Schmerz bei einem Patienten, der dessen bedarf, wobei die Zusammensetzung Aprepitant, Bupivacain und Meloxicam umfasst.

2. Zusammensetzung zur Verwendung nach Anspruch 1, wobei der postoperative Schmerz nicht neuropathisch ist.

3. Zusammensetzung zur Verwendung nach Anspruch 1 oder Anspruch 2, die das Verabreichen der Zusammensetzung während der Operation oder nach der Operation umfasst.

4. Zusammensetzung zur Verwendung nach einem der Ansprüche 1 bis 3, wobei die Zusammensetzung in Form einer injizierbaren Emulsion verabreicht wird, die Folgendes umfasst:
Aprepitant, Bupivacain und Meloxicam;
11 Gew./Gew.-% bis 15 Gew./Gew.-% einer Emulsion;
ein Öl;
ein Co-Tensid, das einen Alkohol umfasst;
ein Tonizitätsmittel;
ein pH-Modifikator; und
Wasser;
wobei der pH-Wert der Emulsion im Bereich von etwa 7,5 bis 9,0 liegt.

5. Zusammensetzung zur Verwendung nach einem der Ansprüche 1 bis 4, ferner umfassend ein Verabreichungssystem, optional wobei Bupivacain und Meloxicam in der Zusammensetzung in einem Verhältnis im Bereich von etwa 15:1 bis 50:1 vorhanden sind.

6. Pharmazeutische Zusammensetzung, umfassend:
(i) Aprepitant, Bupivacain und Meloxicam; und
(ii) ein Verabreichungsvehikel, optional wobei das Verabreichungsvehikel einen Polyorthoester der Formel (I) umfasst: 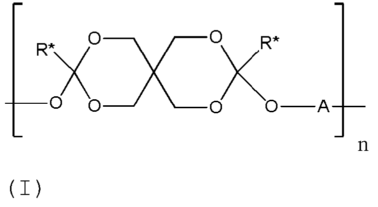
wobei: R* ein Methyl, Ethyl, Propyl oder Butyl ist, n die Anzahl der Wiederholungseinheiten ist und eine ganze Zahl im Bereich von 5 bis 400 ist, und A in jeder Untereinheit R¹ oder R³ ist;
R¹ ist: 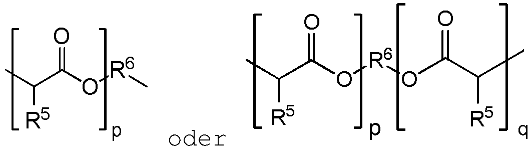
wobei p und q jeweils unabhängig ganze Zahlen im Bereich von etwa 1 bis 20 sind, jedes R⁵ unabhängig Wasserstoff oder C₁₋₄-Alkyl ist; und R⁶ ist: 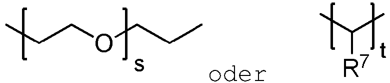
wobei s eine ganze Zahl von 0 bis 10 ist; t eine ganze Zahl von 2 bis 30 ist; und R⁷ Wasserstoff oder C₁₋₄₋ Alkyl ist; R³ ist: 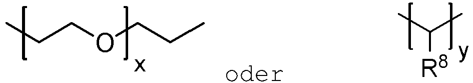
wobei x eine ganze Zahl im Bereich von 1 bis 100 ist, y eine ganze Zahl in einem Bereich von 2 bis 30 ist und R⁸ Wasserstoff oder C₁₋₄-Alkyl ist.

7. Pharmazeutische Zusammensetzung nach Anspruch 6, wobei das Gewichtsverhältnis von Bupivacain:Aprepitant im Bereich von 1:1 bis 60:1, wie zum Beispiel von 1:1 bis 5:1, liegt.

8. Pharmazeutische Zusammensetzung nach Anspruch 6 oder Anspruch 7, umfassend den Polyorthoester der Formel (I), wie in Anspruch 6 definiert.

9. Pharmazeutische Zusammensetzung nach einem der Ansprüche 6 bis 8, wobei die Zusammensetzung ferner ein aprotisches Lösungsmittel umfasst, wie zum Beispiel ein aprotisches Lösungsmittel, ausgewählt aus DMSO, NMP und DMAC.

10. Pharmazeutische Zusammensetzung nach einem der Ansprüche 6 bis 9 zur Verwendung bei der Behandlung von postoperativem Schmerz bei einem Patienten, der dessen bedarf, wie zum Beispiel wobei die pharmazeutische Zusammensetzung an der Operationsstelle verabreicht wird.

## Revendications

1. Composition pour l'utilisation dans le traitement de la douleur post-chirurgicale chez un patient ayant besoin de celle-ci, dans laquelle la composition comprend de l'aprepitant, de la bupivacaine et du meloxicam.

2. Composition pour l'utilisation selon la revendication 1, dans laquelle la douleur post-chirurgicale n'est pas neuropathique.

3. Composition pour l'utilisation selon la revendication 1 ou la revendication 2, qui comprend l'administration de la composition pendant une intervention chirurgicale ou après une intervention chirurgicale.

4. Composition pour l'utilisation selon l'une quelconque des revendications 1 à 3, dans laquelle la composition est administrée sous la forme d'une émulsion injectable comprenant :
de l'aprepitant, de la bupivacaine et du meloxicam ;
de 11 % p/p à 15 % p/p d'une émulsion ;
une huile ;
un co-tensioactif qui comprend un alcool ;
un agent de tonicité ;
un modificateur de pH ; et
de l'eau ;
dans laquelle le pH de l'émulsion varie d'environ 7,5 à 9,0.

5. Composition pour l'utilisation selon l'une quelconque des revendications 1 à 4, comprenant également un système d'administration, facultativement dans laquelle la bupivacaine et le meloxicam sont présents dans la composition dans un rapport variant d'environ 15:1 à 50:1.

6. Composition pharmaceutique comprenant :
(i) de l'aprepitant, de la bupivacaine et du meloxicam ; et
(ii) un vecteur d'administration, facultativement dans laquelle le vecteur d'administration comprend un polyorthoester de formule (I) : 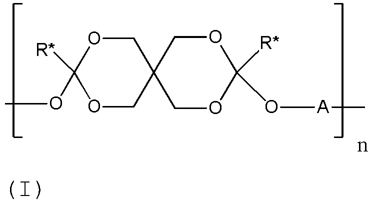
où : R* est un méthyle, un éthyle, un propyle ou un butyle, n est le nombre d'unités de répétition et est un entier compris entre 5 et 400, et A dans chaque sous-unité est R¹ ou R³ ;
R¹ est 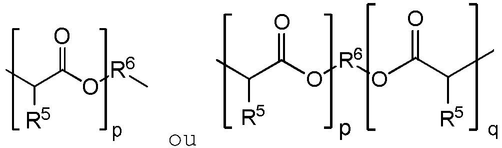
où p et q sont chacun indépendamment des entiers variant d'environ 1 à 20, chaque R⁵ est indépendamment un hydrogène ou un alkyle C₁₋₄ ; et R⁶ est : 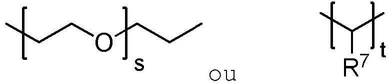
où s est un entier compris entre 0 et 10 ; t est un entier compris entre 2 et 30 ; et R⁷ est un hydrogène ou un alkyle C₁₋₄ ; R³ est : 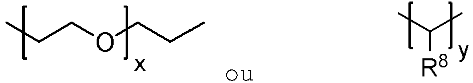
où x est un entier compris entre 1 et 100, y est un entier compris entre 2 et 30 et R⁸ est un hydrogène ou un alkyle C₁₋₄.

7. Composition pharmaceutique selon la revendication 6, dans laquelle le rapport pondéral bupivacaïne:aprépitant varie de 1:1 à 60:1, tel que de 1:1 à 5:1.

8. Composition pharmaceutique selon la revendication 6 ou la revendication 7, comprenant le polyorthoester de formule (I) tel que défini dans la revendication 6.

9. Composition pharmaceutique selon l'une quelconque des revendications 6 à 8, dans laquelle la composition comprend également un solvant aprotique, tel qu'un solvant aprotique choisi parmi le DMSO, le NMP et le DMAC.

10. Composition pharmaceutique selon l'une quelconque des revendications 6 à 9 pour l'utilisation dans le traitement de la douleur post-chirurgicale chez un patient ayant besoin de celle-ci, tel que dans laquelle la composition pharmaceutique est administrée sur le site chirurgical.
